# EUROPEAN PATENT APPLICATION

(11) **EP 4 759 833 A1**
(43) Date of publication of application: **17.06.2026**
(21) Application number: 24853641.9
(22) Date of filing: 08.08.2024
(51) Int. Cl.: C07K 19/00, C07K 14/165, C12N 15/50, C12N 15/62, C12N 15/861, A61K 39/215, A61P 31/14

(54) **PROTEIN, ADENOVIRUS AND VACCINE AGAINST INFECTION OF SUBTYPE OF SARS-COV-2 OMICRON MUTANT STRAIN XBB**

(30) Priority: 11.08.2023 CN 202311009822
(71) Applicant: WestVac Biopharma Co., Ltd., Chengdu, Sichuan 610000 (CN)
(72) Inventor: WEI, Xiawei, Chengdu, Sichuan 610065 (CN); LU, Guangwen, Chengdu, Sichuan 610065 (CN); CHENG, Ping, Chengdu, Sichuan 610065 (CN); YANG, Li, Chengdu, Sichuan 610065 (CN); LI, Jiong, Chengdu, Sichuan 610065 (CN); WANG, Wei, Chengdu, Sichuan 610065 (CN); YANG, Jingyun, Chengdu, Sichuan 610065 (CN); WEI, Yuquan, Chengdu, Sichuan 610065 (CN); WANG, Zhenling, Chengdu, Sichuan 610065 (CN); SHEN, Guobo, Chengdu, Sichuan 610065 (CN); YANG, Jinliang, Chengdu, Sichuan 610065 (CN); ZHAO, Zhiwei, Chengdu, Sichuan 610065 (CN)
(74) Representative: Gulde & Partner
(86) International application number: PCT/CN2024/110575
(87) International publication number: WO 2025/036230

(57) **Abstract**

The present invention relates to proteins, adenoviruses and vaccines against infection by SARS-CoV-2 Omicron XBB subvariants, which belongs to the medicine field. To address the lack of effective prophylactic and therapeutic drugs for preventing and/or treating infections by SARS-CoV-2 Omicron XBB variants and subvariants thereof, the present invention provides proteins, adenoviruses and vaccines for preventing and/or treating infection by SARS-CoV-2 Omicron XBB subvariants, wherein these vaccines are optimized and designed based on a full-length S protein, and the receptor-binding domain (RBD) and receptor-binding domain and heptad repeat (RBD-HR) sequences in the S protein of the SARS-CoV-2 Omicron XBB subvariants, specifically, XBB.1.16, XBB.1.5, XBB.1.16.6, BA.2.86, EG.5, JN.1, XBB.2.3 and XBB.2, and are capable of aiding the host in combating coronavirus infections, and particularly have a relatively good preventive and therapeutic effect against cross-infections caused by SARS-CoV-2 Omicron XBB subvariants.

## Description

### FIELD OF TECHNOLOGY

The present invention relates to proteins, adenoviruses and vaccines for preventing and/or treating infection by SARS-CoV-2 Omicron XBB subvariants, which belongs to the medicine field.

### BACKGROUND

Novel coronavirus (SARS-Cov-2) is a new β coronavirus named by the World Health Organization. This virus is enveloped, and its particles are round or oval, often polymorphic, with a diameter of 60nm-140nm. Its genetic characteristics are significantly different from those of SARS-CoV and MERS-CoV, so the virus is a branch of novel coronavirus that has not been discovered in humans before. Currently, major variants of SARS-CoV-2 include primarily five types: Alpha, Beta, Gamma, Delta, and Omicron, wherein the Omicron variants are divided into subvariants such as BA.1, BA.2, BA.2.12.1, BA.4/5, BQ.1.1, XBB.1.16, XBB.2, XBB.1.5, XBB.2.3, XBB.1.6, XBB.1.9.1, EG.5, XBB.1.16.6, JN.1, and BA.2.86.

Main structural proteins of SARS-CoV-2 include spike (Spike, S) protein, envelop (Envelop, E) protein, membrane (Membrane, M) protein and nucleocapsid (Nucleocapsid, N) protein, among which the S protein plays a key role in virus infection and virulence and is often used as an antigen for vaccines. Because the SARS-CoV-2 XBB variant contains multiple mutation sites, and the virus's S protein further contains multiple mutation sites, this causes the XBB variant and its subvariants to escape the antibodies stimulated by the vaccine of the mutant strains of the new coronavirus (Alpha, Beta, Gamma, Delta, and Omicron), resulting in the failure of the new coronavirus vaccine or reduced protection, which brings great pressure to the prevention and control of novel coronavirus. Therefore, the development of vaccines against the SARS-CoV-2 XBB variant, especially vaccines against various subvariants such as XBB.1.16, XBB.2, XBB.1.5, XBB.2.3, XBB.1.6, XBB.1.9.1, EG5, XBB.1.16.6, JN.1, and BA.2.86, and particularly broad-spectrum vaccines against various SARS-CoV-2 variants, is of great significance for the prevention and control of SARS-COV-2.

### SUMMARY

The present invention aims at solving at least one of the technical problems existing in the prior art. Therefore, the objective of the present invention is to provide proteins and adenoviruses for preventing and/or treating infection by SARS-CoV-2 Omicron XBB subvariants. The present invention also provides a vaccine for preventing and/or treating infection by SARS-CoV-2 Omicron XBB subvariants, including a recombinant protein vaccine and an adenoviral vector vaccine containing the protein. The present invention further provides a polyvaccine combination of different proteins/vaccine combinations, protein vaccines and adenoviral vector vaccines as well.

In a first aspect, the invention provides a protein for preventing and/or treating infection by SARS-CoV-2 Omicron XBB subvariants, which contains at least one amino acid sequence as shown from SEQ ID NO.1 to SEQ ID NO.18, SEQ ID NO.55. Preferably, the protein contains at least one amino acid sequence as shown from SEQ ID NO.1 to SEQ ID NO.11, and SEQ ID NO.55. More preferably, the protein contains at least one amino acid sequence as shown from SEQ ID NO.1, SEQ ID NO.5, SEQ ID NO.8 to SEQ ID NO.11, and SEQ ID NO.55.

The present invention further provides a precursor of the protein, which is connected to a signal peptide and/or protein tag on the protein.

Preferably, the protein tag is selected from at least one of a histidine tag, a thioredoxin tag (Trx tag), a glutathione transferase tag, a ubiquitin-like modifier protein tag, a maltose-binding protein tag, a c-Myc protein tag, an Avi tag protein tag, and a nitrogen source utilization substance A protein tag. Further, the protease recognition region for excising the protein tag is further linked to the protein for preventing and/or treating infection by SARS-CoV-2 Omicron XBB subvariants.

Preferably, the protease is selected from at least one of an enterokinase (EK enzyme), a TEV protease, a thrombin, a coagulation factor Xa, a carboxypeptidase A, and a rhinovirus 3c protease. Further, the amino acid sequence of the precursor is selected from at least one of SEQ ID NO.19 to SEQ ID NO.36, and SEQ ID NO.56. Preferably, the amino acid sequence of the precursor is selected from at least one of SEQ ID NO.19 to SEQ ID NO.29, and SEQ ID NO.56.

More preferably, the amino acid sequence of the precursor is selected from at least one of SEQ ID NO.19, SEQ ID NO.23, SEQ ID NO.26 to SEQ ID NO.29, and SEQ ID NO.56.

The present invention further provides a polynucleotide which encodes the protein or the precursor as described.

Further, the polynucleotide sequence is selected from at least one of SEQ ID NO.37 to SEQ ID NO.54, and SEQ ID NO.57. Preferably, the polynucleotide sequence is selected from at least one of SEQ ID NO.37 to SEQ ID NO.47, and SEQ ID NO.57. More preferably, the polynucleotide sequence is selected from at least one of SEQ ID NO.37, SEQ ID NO.41, SEQ ID NO.44 to SEQ ID NO.47, and SEQ ID NO.57.

The present invention further provides a recombinant vector which contains the polynucleotide. Further, the recombinant vector is at least one of an insect baculovirus expression vector, a mammalian cell expression vector, an Escherichia coli expression vector, and a yeast expression vector.

Preferably, the insect baculovirus expression vector is pFastBac1.

Preferably, the Escherichia coli expression vector is pET32a.

Preferably, the yeast expression vector is pPICZaA.

Preferably, the mammalian cell expression vector is a CHO cell expression vector.

More preferably, the CHO cell expression vector is pTT5 or FTP-002.

The present invention further provides a host cell which contains the recombinant vector.

Further, the host cell is at least one of an insect cell, a mammalian cell, Escherichia coli, and yeast. Preferably, the insect cell is selected from at least one of a sf9 cell, a sf21 cell, and a Hi5 cell. Preferably, the mammalian cell is a CHO cell.

The present invention further provides a preparation method for the protein or the precursor, wherein the method includes the following steps: culturing the host cell to express the protein or precursor, and then recovering the protein.

The present invention further provides a recombinant protein vaccine for preventing and/or treating infection by SARS-CoV-2 Omicron subvariants, which contains the protein and/or the precursor, as well as a pharmaceutically acceptable excipient or an adjuvant component.

Further, the adjuvant component is an immunologic adjuvant.

Preferably, the immunologic adjuvant is selected from at least one of the following: squalene-based oil-in-water emulsion, aluminum salt, calcium salt, phytosaponin, phytopolysaccharide, monophosphate lipid A, muramyl dipeptide, muramyl tripeptide, bacterial toxin, GM-CSF cytokine, lipid, and cationic liposomal material.

Further, at least one of the following is met:
the squalene-based oil-in-water emulsion is MF59;
the aluminum salt is selected from at least one of aluminum hydroxide and alum;
the calcium salt is tricalcium phosphate;
the phytosaponin is either QS-21 or ISCOM;
the plant polysaccharide is astragalus polysaccharide;
the bacterial toxin is selected from at least one of recombinant cholera toxin and diphtheria toxin; the lipid is selected from at least one of the following: phosphatidylethanolamine, phosphatidylcholine, cholesterol, and dioleoylphosphatidylethanolamine;
the cationic liposome material is selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl trifluoroacetate-2,3-dioleoxypropyl-2-(2-spermamido) ethylammonium, trimethyl dodecyl ammonium bromide, trimethyl tetradecyl ammonium bromide, trimethyl cetyl ammonium bromide, dimethyl dioctadecyl ammonium bromide, and CpG ODN. The present invention further provides a protein composition for preventing and/or treating infection by SARS-CoV-2 Omicron subvariants, which contains any two or more proteins or protein precursors for preventing and/or treating infection by SARS-CoV-2 Omicron XBB subvariants; the protein is constructed with any one amino acid sequence selected from SEQ ID NO.1 to SEQ ID NO.18, and SEQ ID NO.55; and the protein precursor is constructed with any amino acid sequence selected from SEQ ID NO.19 to SEQ ID NO.36, and SEQ ID NO.56.

The present invention further provides a recombinant protein vaccine composition for preventing and/or treating infection by SARS-CoV-2 Omicron subvariants, which contains any two or more different recombinant protein vaccines.

The difference of the recombinant protein vaccine is the amino acid sequence constructing the protein. The recombinant protein vaccine composition refers to the combination of two or more different recombinant protein vaccines prepared with different amino acid sequences. The amino acid sequence is selected from at least one of SEQ ID NO.1 to SEQ ID NO.36, SEQ ID NO.55, and SEQ ID NO.56.

The present invention further provides an adenoviral vector vaccine for preventing and/or treating infection by SARS-CoV-2 Omicron subvariants, which contains the polynucleotide encoding SARS-CoV-2 or a variant spike protein thereof. The polynucleotide sequence is obtained through codon optimization or cell optimization.

Preferably, the nucleotide sequence of the polynucleotide is selected from at least one of SEQ ID NO.37 to SEQ ID NO.54. More preferably, the polynucleotide sequence is selected from at least one of SEQ ID NO.48 to SEQ ID NO.54. More preferably, the polynucleotide sequence is selected from at least one of SEQ ID NO.48 to SEQ ID NO.51.

Further, the adenovirus vector of the adenoviral vector vaccine is selected from at least one of the following: adenovirus, Ankara vaccinia virus, and adeno-associated virus.

Preferably, the adenovirus vector is selected from replication-defective human adenovirus 5, 35, or 26 or/and replication-deficient chimpanzee adenovirus AdC68 or AdC7.

More preferably, the adenovirus vector is selected from replication-defective human adenovirus 5 with combined deletion of E1 and E3.

The present invention provides a method for preparing adenovirus of adenoviral vector vaccine, which comprises the following steps: constructing a shuttle plasmid vector of the polynucleotide encoding SARS-CoV-2 or a variant spike protein thereof; transfecting the constructed shuttle plasmid vector and a backbone plasmid into the host cell to culture the host cell; obtaining a replication-deficient recombinant adenovirus; and performing large-scale cultivation and purification.

Further, the polynucleotide sequence encoding SARS-CoV-2 or a variant spike protein thereof is selected from at least one of SEQ ID NO.37 to SEQ ID NO.54. Preferably, the polynucleotide sequence is selected from at least one of SEQ ID NO.48 to SEQ ID NO.54. More preferably, the polynucleotide sequence is selected from at least one of SEQ ID NO.48 to SEQ ID NO.51.

Further, the shuttle plasmid vector is at least one of pDC316-S, pDC315-S, pDC516-S, and pDC515-S.

Further, the backbone plasmid is at least one of pBHGlox_E1, 3Cre and pBHGlox_E1, and 3FLP. Further, the host cell is HEK293.

The present invention further provides an adenoviral vector vaccine composition for preventing and/or treating infection by SARS-CoV-2 Omicron subvariants, which contains any two or more different adenoviral vector vaccines. Different adenoviral vector vaccines refer to that the adenoviral vector vaccines are prepared with different nucleotide sequences. The adenoviral vector vaccine composition refers to the combination of two or more different prepared adenoviral vector vaccines. The nucleotide sequence is selected from at least one of SEQ ID NO.37 to SEQ ID NO.54.

Further, the protein, protein precursor, protein composition, recombinant protein vaccine, recombinant protein vaccine composition, adenoviral vector vaccine, and adenoviral vector vaccine composition are formulated as an intradermal injection preparation or a subcutaneous injection preparation, an intramuscular injection preparation, an intravenous injection preparation, an oral preparation or a nasal spray preparation.

Preferably, the vaccine is an intramuscular injection preparation and a nasal spray preparation.

The present invention further provides a pharmaceutical composition for treating and/or preventing infection by SARS-CoV-2 Omicron subvariants, which is the combination of at least one of recombinant protein, protein composition, recombinant protein vaccine, and recombinant protein vaccine composition, as well as at least one of adenoviral vector vaccine and adenoviral vector vaccine composition.

Preferably, the pharmaceutical composition is a polyvalent vaccine combination of at least one of the recombinant protein vaccine and the recombinant protein vaccine composition, as well as at least one of the adenoviral vector vaccine and the adenoviral vector vaccine composition.

Preferably, the pharmaceutical composition is a polyvalent vaccine combination of at least one of the recombinant protein and the protein composition, as well as at least one of the adenoviral vector vaccine and the adenoviral vector vaccine composition.

The present invitation provides a combined drug for treating and/or preventing infection by SARS-CoV-2 Omicron subvariants, which is the combination of at least one of the recombinant protein, the protein composition, the recombinant protein vaccine, the recombinant protein vaccine composition and at least one of the adenoviral vector vaccine and the adenoviral vector vaccine composition, which are separately or simultaneously administered,.

Preferably, the combined drug is the combination of at least one of the recombinant protein vaccine and the recombinant protein vaccine composition, as well as at least one of the adenoviral vector vaccine and the adenoviral vector vaccine composition.

Preferably, the combined drug is the combination of at least one of the recombinant protein and the protein composition, as well as at least one of the adenoviral vector vaccine and the adenoviral vector vaccine composition.

More preferably, the recombinant protein vaccine contains the protein or precursor for preventing and/or treating infection by SARS-CoV-2 Omicron XBB subvariants. The amino acid sequence of the protein or the precursor is selected from at least one of SEQ ID NO.1 to SEQ ID NO.11, SEQ ID NO.55, SEQ ID NO.19 to SEQ ID NO.29, and SEQ ID NO.56. Preferably, the amino acid sequence of the protein or the precursor is selected from at least one of SEQ ID NO.1, SEQ ID NO.5, SEQ ID NO.8 to SEQ ID NO.11, and SEQ ID NO.55 or SEQ ID NO.19, SEQ ID NO.23, SEQ ID NO.26 to SEQ ID NO.29, and SEQ ID NO.56. The recombinant protein vaccine composition is the combination of any two or more recombinant protein vaccines with different proteins.

The adenoviral vector vaccine contains at least one polynucleotide sequence as shown from SEQ ID NO.48 to SEQ ID NO.54. Preferably, the adenoviral vector vaccine contains at least one polynucleotide sequence as shown from SEQ ID NO.48 to SEQ ID NO.51.

The adenoviral vector vaccine composition refers to the combination of two or more adenoviral vector vaccines prepared with different polynucleotide sequences.

Most preferably, the recombinant protein vaccine contains at least one amino acid sequence as shown from SEQ ID NO.1, SEQ ID NO.5, SEQ ID NO.8 to SEQ ID NO.11, and SEQ ID NO.55. The adenoviral vector vaccine contains at least one polynucleotide sequence as shown from SEQ ID NO.48 to SEQ ID NO.51.

Further, the pharmaceutical composition or the combined drug is formulated as an intradermal injection preparation or a subcutaneous injection preparation, an intramuscular injection preparation, an intravenous injection preparation, an oral preparation or a nasal spray preparation; and preferably, the pharmaceutical composition or the combined drug is formulated as the intramuscular injection preparation and the nasal spray preparation.

The present invention further provides the use of the protein, the precursor, the protein composition, the recombinant protein vaccine or the composition thereof, the adenoviral vector vaccine or the composition thereof, and the pharmaceutical composition or the combined drug in the preparation of the drug for treating and/or preventing infection or pathogenicity caused by SARS-CoV-2 or variants thereof.

Preferably, the SARS-CoV-2 variant contains SARS-CoV-2 variants Alpha, Beta, Gamma, Delta, Omicron, and Omicron subvariants BA.1, BA.2, BA.2.12.1, BA.4/5, BQ.1.1, XBB.1.16, XBB.2, XBB.1.5, XBB.2.3, XBB.1.6, XBB.1.9.1, XBB.1.16.6, FL.1.5.1, HV.1, EG.5, EG.5.1, BA.2.86, JN.1, JN.1.13, KP.2, and KP.3.

The following sequences are constructed by the applicant on the basis of the RBD and RBD-HR sequences in the S protein as well as optimized full-length S protein of SARS-CoV-2 Omicron subvariants XBB.1.16, XBB.2, XBB.1.5, XBB.2.3, XBB.1.16.6, BA.2.86, EG5 and JN.1.
**SEQ ID NO.1,** RBD-HR sequence of optimized S protein of XBB.1.16 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNRPCNGVAGPNCYSPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.2,** RBD-HR sequence of optimized S protein of XBB.2 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGSNCYSPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.3,** RBD sequence of optimized S protein of XBB.1.5-1 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGPNCYSPLQSYGFRPTYGVG
HQPYRVVVLSFELLHAPATVCGPKKSTNLVKNKCVNFNFNG
**SEQ ID NO.4,** RBD sequence of optimized S protein of XBB.1.5-2 
VQPTESIVRFPNITNLCPFHEVFNATTFASVYAWNRKRISNCVADYSVIYNSASFSAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGPNCYSPLQSYGFRPTYGVG
HQPYRVVVLSFELLHAPATVCGPKKSTNLVKNKCVNFNFNG
**SEQ ID NO.5**, RBD-HR sequence of optimized S protein of XBB.1.5-3 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGPNCYSPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.6,** RBD-HR sequence of optimized S protein of XBB.1.5-4 
VQPTESIVRFPNITNLCPFHEVFNATTFASVYAWNRKRISNCVADYSVIYNSASFSAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGPNCYSPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.7**, RBD sequence of optimized S protein of XBB.2.3-1 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGPNCYSPLQSYGFRPTYGVG
HQPYRVVVLSFELLHASATVCGPKKSTNLVKNKCVNFNFNG
**SEQ ID NO.8**, RBD-HR sequence of optimized S protein of XBB.2.3-2 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLFRKSKLKPFERDISTEIYQAGNKPCNGVAGPNCYSPLQSYGFRPTYGVG
HQPYRVVVLSFELLHASATVCGPKKSTNLVKNKSVNFNFNGLYENQKLIANQFNSAIGKIQ
DSLSSTASALGKLQDVVNQNAQALNTLVKQLKNHTSPDVDLGDISGINASVVNIQKEIDRL
NEVAKNLNESLIDLQEL
**SEQ ID NO.9,** RBD-HR sequence of optimized S protein of XBB.1.16.6 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLLRKSKLKPFERDISTEIYQAGNRPCNGVAGPNCYSPLQSYGFRPTYGVG
HQPYRVVVLSFELLHAPATVCGPKKSTNLVKNKSVNFNFNGLYENQKLIANQFNSAIGKIQD
 
SLSSTASALGKLQDVVNQNAQALNTLVKQLKNHTSPDVDLGDISGINASVVNIQKEIDRLN
EVAKNLNESLIDLQEL
**SEQ ID NO.10**, RBD-HR sequence of optimized S protein of BA.2.86 
VQPTESIVRFPNVTNLCPFHEVFNATRFASVYAWNRTRISNCVADYSVLYNFAPFFAFKCYG
VSPTKLNDLCFTNVYADSFVIKGNEVSQIAPGQTGNIADYNYKLPDDFTGCVIAWNSNKLD
SKHSGNYDYWYRLFRKSKLKPFERDISTEIYQAGNKPCKGKGPNCYFPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.11**, RBD-HR sequence of optimized S protein of EG.5 
VQPTESIVRFPNITNLCPFHEVFNATTFASVYAWNRKRISNCVADYSVIYNFAPFFAFKCYGV
SPTKLNDLCFTNVYADSFVIRGNEVSQIAPGQTGNIADYNYKLPDDFTGCVIAWNSNKLDS
KPSGNYNYLYRLLRKSKLKPFERDISTEIYQAGNKPCNGVAGPNCYSPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.12**, Sequence of optimized full-length S protein of XBB.1.16, excluding signal peptide 
SQCVNLITRTQSYTNSFTRGVYYPDKVFRSSVLHSTQDLFLPFFSNVTWFHAIHVSGTNGTK
RFDNPALPFNDGVYFASTEKSNIIRGWIFGTTLDSKTQSLLIVNNATNVVIKVCEFQFCNDPF
LDVYQKNNKSWMESEFRVYSSANNCTFEYVSQPFLMDLVGKEGNFKNLREFVFKNIDGYF
KIYSKHTPINLERDLPQGFSALEPLVDLPIGINITRFQTLLALHRSYLTPVDSSSGWTAGAAAY
YVGYLQPRTFLLKYNENGTITDAVDCALDPLSETKCTLKSFTVEKGIYQTSNFRVQPTESIVR
FPNITNLCPFHEVFNATTFASVYAWNRKRISNCVADYSVIYNFAPFFAFKCYGVSPTKLNDLC
FTNVYADSFVIRGNEVSQIAPGQTGNIADYNYKLPDDFTGCVIAWNSNKLDSKPSGNYNYL
YRLFRKSKLKPFERDISTEIYQAGNRPCNGVAGPNCYSPLQSYGFRPTYGVGHQPYRVVVLS
FELLHAPATVCGPKKSTNLVKNKCVNFNFNGLTGTGVLTESNKKFLPFQQFGRDIADTTDAV
RDPQTLEILDITPCSFGGVSVITPGTNTSNQVAVLYQGVNCTEVPVAIHADQLTPTWRVYSTG
SNVFQTRAGCLIGAEYVNNSYECDIPIGAGICASYQTQTKSHGSASSVASQSIIAYTMSLGAE
NSVAYSNNSIAIPTNFTISVTTEILPVSMTKTSVDCTMYICGDSTECSNLLLQYGSFCTQLKR
ALTGIAVEQDKNTQEVFAQVKQIYKTPPIKYFGGFNFSQILPDPSKPSKRSPIEDLLFNKVTLA
DAGFIKQYGDCLGDIAARDLICAQKFNGLTVLPPLLTDEMIAQYTSALLAGTITSGWTFGAG
PALQIPFPMQMAYRFNGIGVTQNVLYENQKLIANQFNSAIGKIQDSLSSTPSALGKLQDVVN
 
HNAQALNTLVKQLSSKFGAISSVLNDILSRLDPPEAEVQIDRLITGRLQSLQTYVTQQLIRAA
EIRASANLAATKMSECVLGQSKRVDFCGKGYHLMSFPQSAPHGVVFLHVTYVPAQEKNFT
TAPAICHDGKAHFPREGVFVSNGTHWFVTQRNFYEPQIITTDNTFVSGNCDVVIGIVNNTVY
DPLQPELDSFKEELDKYFKNHTSPDVDLGDISGINASVVNIQKEIDRLNEVAKNLNESLIDLQ
ELGKYEQYIKWPWYIWLGFIAGLIAIVMVTIMLCCMTSCCSCLKGCCSCGSCCKFDEDDSE
PVLKGVKLHYT
**SEQ ID NO.13**, Sequence of optimized full-length S protein of XBB.2, excluding signal peptide 
SQCVNLITRTQSYTNSFTRGVYYPDKVFRSSVLHSTQDLFLPFFSNVTWFRAIHVSGTNGTK
RFDNPALPFNDGVYFASTEKSNIIRGWIFGTTLDSKTQSLLIVNNATNVVIKVCEFQFCNDPF
LDVYQKNNKSWMESEFRVYSSANNCTFEYVSQPFLMDLEGKEGNFKNLREFVFKNIDGYF
KIYSKHTPINLERDLPQGFSALEPLVDLPIGINITRFQTLLALHRSYLTPGGSSSGWTAGAAAY
YVGYLQPRTFLLKYNENGTITDAVDCALDPLSETKCTLKSFTVEKGIYQTSNFRVQPTESIVR
FPNITNLCPFHEVFNATTFASVYAWNRKRISNCVADYSVIYNFAPFFAFKCYGVSPTKLNDLC
FTNVYADSFVIRGNEVSQIAPGQTGNIADYNYKLPDDFTGCVIAWNSNKLDSKPSGNYNYL
YRLFRKSKLKPFERDISTEIYQAGNKPCNGVAGSNCYSPLQSYGFRPTYGVGHQPYRVVVL
SFELLHAPATVCGPKKSTNLVKNKCVNFNFNGLTGTGVLTESNKKFLPFQQFGRDIADTTDA
VRDPQTLEILDITPCSFGGVSVITPGTNTSNQVAVLYQGVNCTEVPVAIHADQLTPTWRVYST
GSNVFQTRAGCLIGAEYVNNSYECDIPIGAGICASYQTQTKSHGSASSVASQSIIAYTMSLGA
ENSVAYSNNSIAIPTNFTISVTTEILPVSMTKTSVDCTMYICGDSTECSNLLLQYGSFCTQLKR
ALTGIAVEQDKNTQEVFAQVKQIYKTPPIKYFGGFNFSQILPDPSKPSKRSPIEDLLFNKVTLA
DAGFIKQYGDCLGDIAARDLICAQKFNGLTVLPPLLTDEMIAQYTSALLAGTITSGWTFGAG
PALQIPFPMQMAYRFNGIGVTQNVLYENQKLIANQFNSAIGKIQDSLSSTPSALGKLQDVVN
HNAQALNTLVKQLSSKFGAISSVLNDILSRLDPPEAEVQIDRLITGRLQSLQTYVTQQLIRAA
EIRASANLAATKMSECVLGQSKRVDFCGKGYHLMSFPQSAPHGVVFLHVTYVPAQEKNFT
TAPAICHDGKAHFPREGVFVSNGTHWFVTQRNFYEPQIITTDNTFVSGNCDVVIGIVNNTVY
DPLQPELDSFKEELDKYFKNHTSPDVDLGDISGINASVVNIQKEIDRLNEVAKNLNESLIDLQ
ELGKYEQYIKWPWYIWLGFIAGLIAIVMVTIMLCCMTSCCSCLKGCCSCGSCCKFDEDDSE
PVLKGVKLHYT
**SEQ ID NO.14,** Sequence of optimized full-length S protein of XBB.1.5, excluding signal peptide 
SQCVNLITRTQSYTNSFTRGVYYPDKVFRSSVLHSTQDLFLPFFSNVTWFHAIHVSGTNGTK
RFDNPALPFNDGVYFASTEKSNIIRGWIFGTTLDSKTQSLLIVNNATNVVIKVCEFQFCNDPF
LDVYQKNNKSWMESEFRVYSSANNCTFEYVSQPFLMDLEGKEGNFKNLREFVFKNIDGYF
KIYSKHTPINLERDLPQGFSALEPLVDLPIGINITRFQTLLALHRSYLTPVDSSSGWTAGAAAY
 
YVGYLQPRTFLLKYNENGTITDAVDCALDPLSETKCTLKSFTVEKGIYQTSNFRVQPTESIVR
FPNITNLCPFHEVFNATTFASVYAWNRKRISNCVADYSVIYNFAPFFAFKCYGVSPTKLNDLC
FTNVYADSFVIRGNEVSQIAPGQTGNIADYNYKLPDDFTGCVIAWNSNKLDSKPSGNYNYL
YRLFRKSKLKPFERDISTEIYQAGNKPCNGVAGPNCYSPLQSYGFRPTYGVGHQPYRVVVL
SFELLHAPATVCGPKKSTNLVKNKCVNFNFNGLTGTGVLTESNKKFLPFQQFGRDIADTTDA
VRDPQTLEILDITPCSFGGVSVITPGTNTSNQVAVLYQGVNCTEVPVAIHADQLTPTWRVYST
GSNVFQTRAGCLIGAEYVNNSYECDIPIGAGICASYQTQTKSHRRARSVASQSIIAYTMSLG
AENSVAYSNNSIAIPTNFTISVTTEILPVSMTKTSVDCTMYICGDSTECSNLLLQYGSFCTQLK
RALTGIAVEQDKNTQEVFAQVKQIYKTPPIKYFGGFNFSQILPDPSKPSKRSFIEDLLFNKVTL
ADAGFIKQYGDCLGDIAARDLICAQKFNGLTVLPPLLTDEMIAQYTSALLAGTITSGWTFGA
GAALQIPFAMQMAYRFNGIGVTQNVLYENQKLIANQFNSAIGKIQDSLSSTASALGKLQDV
VNHNAQALNTLVKQLSSKFGAISSVLNDILSRLDKVEAEVQIDRLITGRLQSLQTYVTQQLI
RAAEIRASANLAATKMSECVLGQSKRVDFCGKGYHLMSFPQSAPHGVVFLHVTYVPAQEK
NFTTAPAICHDGKAHFPREGVFVSNGTHWFVTQRNFYEPQIITTDNTFVSGNCDVVIGIVNN
TVYDPLQPELDSFKEELDKYFKNHTSPDVDLGDISGINASVVNIQKEIDRLNEVAKNLNESLI
DLQELGKYEQYIKWPWYIWLGFIAGLIAIVMVTIMLCCMTSCCSCLKGCCSCGSCCKFDED
DSEPVLKGVKLHYT
**SEQ ID NO.15,** Sequence of optimized full-length S protein of XBB.2.3, excluding signal peptide

SQCVNLITRTQSYTNSFTRGVYYPDKVFRSSVLHSTQDLFLPFFSNVTWFHAIHVSGTNGTK
RFDNPALPFNDGVYFASTEKSNIIRGWIFGTTLDSKTQSLLIVNNATNVVIKVCEFQFCNDPF
LDVYQKNNKSWMESEFRVYSSANNCTFEYVSQPFLMDLEGKEGNFKNLREFVFKNIDGYF
KIYSKHTPINLERDLPQGFSALEPLVDLPIGINITRFQTLLALHRSYLTPGGSSSGWTAGAAAY
YVGYLQPRTFLLKYNENGTITDAVDCALDPLSETKCTLKSFTVEKGIYQTSNFRVQPTESIVR
FPNITNLCPFHEVFNATTFASVYAWNRKRISNCVADYSVIYNFAPFFAFKCYGVSPTKLNDLC
FTNVYADSFVIRGNEVSQIAPGQTGNIADYNYKLPDDFTGCVIAWNSNKLDSKPSGNYNYL
YRLFRKSKLKPFERDISTEIYQAGNKPCNGVAGPNCYSPLQSYGFRPTYGVGHQPYRVVVL
SFELLHASATVCGPKKSTNLVKNKCVNFNFNGLTGTGVLTESNKKFLPFQQFGRDIADTTDA
VRDPQTLEILDITPCSFGGVSVITPGTNTSNQVAVLYQGVNCTEVPVAIHADQLTPTWRVYST
GSNVFQTRAGCLIGAEYVNNSYECDIPIGAGICASYQTQTKSHGSASSVASQSIIAYTMSLGA
ENSVAYSNNSIAIPTNFTISVTTEILPVSMTKTSVDCTMYICGDSTECSNLLLQYGSFCTQLKR
ALTGIAVEQDKNTQEVFAQVKQIYKTPPIKYFGGFNFSQILPDPSKPSKRSPIEDLLFNKVTLA
DAGFIKQYGDCLGDIAARDLICAQKFNGLTVLPPLLTDEMIAQYTSALLAGTITSGWTFGAG
PALQIPFPMQMAYRFNGIGVTQNVLYENQKLIANQFNSAIGKIQDSLSSTPSALGKLQDVVN
HNAQALNTLVKQLSSKFGAISSVLNDILSRLDPPEAEVQIDRLITGRLQSLQTYVTQQLIRAA

EIRASANLAATKMSECVLGQSKRVDFCGKGYHLMSFPQSAPHGVVFLHVTYVPAQEKNFT
TAPAICHDGKAHFPREGVFVSNGTHWFVTQRNFYEPQIITTDNTFVSGNCDVVIGIVNNTVY
DPLQPELDSFKEELDKYFKNHTSPDVDLGDISGINASVVNIQKEIDRLNEVAKNLNESLIDLQ
ELGKYEQYIKWPWYIWLGFIAGLIAIVMVTIMLCCMTSCCSCLKGCCSCGSCCKFDEDDSE
PVLKGVKLHYT

**SEQ ID NO.16,** Sequence of optimized full-length S protein of XBB.1.6, excluding signal peptide SEQ ID NO.16 is a variant obtained by sequence substitution and/or deletion and/or insertion of at least one amino acid in an amino acid sequence of full-length S protein of XBB.1.6.

**SEQ ID NO.17,** Sequence of optimized full-length S protein of XBB.1.9.1, excluding signal peptide
SEQ ID NO.17 is a variant obtained by sequence substitution and/or deletion and/or insertion of at least one amino acid in an amino acid sequence of full-length S protein of XBB.1.9.1.

**SEQ ID NO.18,** Sequence of optimized full-length S protein of EG.5, excluding signal peptide SEQ ID NO.18 is a variant obtained by sequence substitution and/or deletion and/or insertion of at least one amino acid in an amino acid sequence of full-length S protein of EG5.

In order to assist the secretory expression of the protein, a signal peptide has been added to its amino acids when the protein is constructed. Moreover, in order to facilitate purification, a His tag can further be added to the amino acid sequence of protein. The present invention is the expression of optimized protein constructed on the basis of the S proteins of XBB.1.16, XBB.2, XBB.1.5, XBB.2.3, XBB.1.16.6, BA.2.86, and EG5, and its construction and design sequence is as shown from SEQ ID NO.19 to SEQ ID NO.29.

Further, the corresponding nucleotide sequences encoding the amino acid sequences are shown as SEQ ID NO.37 to SEQ ID NO.47.
**SEQ ID NO.19,** Sequence based on optimized and designed S protein-RBD-HR of Omicron_XBB.1.16, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNRPCNGVAGPNCYSPLQSYGFRPTYGVGHQPYRVVVLSFELLH
APATVCGPKKSTNLVKNKSVNFNFNGLYENQKLIANQFNSAIGKIQDSLSSTASALGKLQDV
 VNQNAQALNTLVKQLKNHTSPDVDLGDISGINASVVNIQKEIDRLNEVAKNLNESLIDLQEL
**SEQ ID NO.20**, Sequence based on optimized and designed S protein-RBD-HR of Omicron_XBB.2, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGSNCYSPLQSYGFRPTYGVGHQPYRVVVLSFELLH
APATVCGPKKSTNLVKNKSVNFNFNGLYENQKLIANQFNSAIGKIQDSLSSTASALGKLQDV
VNQNAQALNTLVKQLKNHTSPDVDLGDISGINASVVNIQKEIDRLNEVAKNLNESLIDLQEL
**SEQ ID NO.21,** Sequence based on optimized and designed S protein-RBD of Omicron_XBB.1.5-1, including: signal peptide-Trx tag-6His tag-EK digestion site-RBD sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDCIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGPNCYSPLQSYGFRPTYGVGHQPYRVVVLSFELLH
APATVCGPKKSTNLVKNKCVNFNFNG
**SEQ ID NO.22,** Sequence based on optimized and designed S protein-RBD of Omicron_XBB.1.5-2, including: signal peptide-Trx tag-6His tag-EK digestion site-RBD sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDCIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNSASFSAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGPNCYSPLQSYGFRPTYGVGHQPYRVVVLSFELLH
APATVCGPKKSTNLVKNKCVNFNFNG
**SEQ ID NO.23,** Sequence based on optimized and designed S protein-RBD-HR of Omicron_XBB.1.5-3, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGPNCYSPLQSYGFRPTYGVGHQPYRVVVLSFELLH
APATVCGPKKSTNLVKNKSVNFNFNGLYENQKLIANQFNSAIGKIQDSLSSTASALGKLQDV
VNQNAQALNTLVKQLKNHTSPDVDLGDISGINASVVNIQKEIDRLNEVAKNLNESLIDLQEL
**SEQ ID NO.24,** Sequence based on optimized and designed S protein-RBD-HR of Omicron_XBB.1.5-4, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHSSGDDDDKVQPTESIVRFPNITN
LCPFHEVFNATTFASVYAWNRKRISNCVADYSVIYNSASFSAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGPNCYSPLQSYGFRPTYGVGHQPYRVVVLSFELLH
APATVCGPKKSTNLVKNKSVNFNFNGLYENQKLIANQFNSAIGKIQDSLSSTASALGKLQDV
VNQNAQALNTLVKQLKNHTSPDVDLGDISGINASVVNIQKEIDRLNEVAKNLNESLIDLQEL
**SEQ ID NO.25**, Sequence based on optimized and designed S protein-RBD of Omicron_XBB.2.3-1, including: signal peptide-Trx tag-6His tag-EK digestion site-RBD sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDCIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGPNCYSPLQSYGFRPTYGVGHQPYRVVVLSFELLII
ASATVCGPKKSTNLVKNKCVNFNFNG
**SEQ ID NO.26**, Sequence based on optimized and designed S protein-RBD-HR of Omicron_XBB.2.3-2, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLFR
KSKLKPFERDISTEIYQAGNKPCNGVAGPNCYSPLQSYGFRPTYGVGHQPYRVVVLSFELLH
ASATVCGPKKSTNLVKNKSVNFNFNGLYENQKLIANQFNSAIGKIQDSLSSTASALGKLQDV
VNQNAQALNTLVKQLKNHTSPDVDLGDISGINASVVNIQKEIDRLNEVAKNLNESLIDLQEL
**SEQ ID NO.27,** Sequence based on optimized and designed S protein-RBD-HR of Omicron_XBB.1.16.6, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLLR
KSKLKPFERDISTEIYQAGNRPCNGVAGPNCYSPLQSYGFRPTYGVGHQPYRVVVLSFELLH
APATVCGPKKSTNLVKNKSVNFNFNGLYENQKLIANQFNSAIGKIQDSLSSTASALGKLQDV
VNQNAQALNTLVKQLKNHTSPDVDLGDISGINASVVNIQKEIDRLNEVAKNLNESLIDLQEL
**SEQ ID NO.28,** Sequence based on optimized and designed S protein-RBD-HR of Omicron_BA.2.86, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNVT
NLCPFHEVFNATRFASVYAWNRTRISNCVADYSVLYNFAPFFAFKCYGVSPTKLNDLCFTNV
YADSFVIKGNEVSQIAPGQTGNIADYNYKLPDDFTGCVIAWNSNKLDSKHSGNYDYWYRL
FRKSKLKPFERDISTEIYQAGNKPCKGKGPNCYFPLQSYGFRPTYGVGHQPYRVVVLSFELL
HAPATVCGPKKSTNLVKNKSVNFNFNGLYENQKLIANQFNSAIGKIQDSLSSTASALGKLQD
VVNQNAQALNTLVKQLKNHTSPDVDLGDISGINASVVNIQKEIDRLNEVAKNLNESLIDLQ
EL
**SEQ ID NO.29,** Sequence based on optimized and designed S protein-RBD-HR of Omicron_EG.5, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFHEVFNATTFASVYAWNRKRISNCVADYSVIYNFAPFFAFKCYGVSPTKLNDLCFTNVY
ADSFVIRGNEVSQIAPGQTGNIADYNYKLPDDFTGCVIAWNSNKLDSKPSGNYNYLYRLLR
KSKLKPFERDISTEIYQAGNKPCNGVAGPNCYSPLQSYGFRPTYGVGHQPYRVVVLSFELLH
APATVCGPKKSTNLVKNKSVNFNFNGLYENQKLIANQFNSAIGKIQDSLSSTASALGKLQDV
VNQNAQALNTLVKQLKNHTSPDVDLGDISGINASVVNIQKEIDRLNEVAKNLNESLIDLQEL
**SEQ ID NO.30,** Sequence based on optimized and designed full-length S protein of Omicron_XBB.1.16 (including native signal peptide), serving as amino acid sequence of S antigen for Ad-S-XBB.1.16 vaccine 
MFVFLVLLPLVSSQCVNLITRTQSYTNSFTRGVYYPDKVFRSSVLHSTQDLFLPFFSNVTWF
HAIHVSGTNGTKRFDNPALPFNDGVYFASTEKSNIIRGWIFGTTLDSKTQSLLIVNNATNVVI
KVCEFQFCNDPFLDVYQKNNKSWMESEFRVYSSANNCTFEYVSQPFLMDLVGKEGNFKNL
REFVFKNIDGYFKIYSKHTPINLERDLPQGFSALEPLVDLPIGINITRFQTLLALHRSYLTPVDS
SSGWTAGAAAYYVGYLQPRTFLLKYNENGTITDAVDCALDPLSETKCTLKSFTVEKGIYQT
SNFRVQPTESIVRFPNITNLCPFHEVFNATTFASVYAWNRKRISNCVADYSVIYNFAPFFAFKC
YGVSPTKLNDLCFTNVYADSFVIRGNEVSQIAPGQTGNIADYNYKLPDDFTGCVIAWNSNK
LDSKPSGNYNYLYRLFRKSKLKPFERDISTEIYQAGNRPCNGVAGPNCYSPLQSYGFRPTYG
VGHQPYRVVVLSFELLHAPATVCGPKKSTNLVKNKCVNFNFNGLTGTGVLTESNKKFLPFQ
QFGRDIADTTDAVRDPQTLEILDITPCSFGGVSVITPGTNTSNQVAVLYQGVNCTEVPVAIHA
DQLTPTWRVYSTGSNVFQTRAGCLIGAEYVNNSYECDIPIGAGICASYQTQTKSHGSASSVA
SQSIIAYTMSLGAENSVAYSNNSIAIPTNFTISVTTEILPVSMTKTSVDCTMYICGDSTECSNLL
LQYGSFCTQLKRALTGIAVEQDKNTQEVFAQVKQIYKTPPIKYFGGFNFSQILPDPSKPSKRS
PIEDLLFNKVTLADAGFIKQYGDCLGDIAARDLICAQKFNGLTVLPPLLTDEMIAQYTSALL
AGTITSGWTFGAGPALQIPFPMQMAYRFNGIGVTQNVLYENQKLIANQFNSAIGKIQDSLSS
TPSALGKLQDVVNHNAQALNTLVKQLSSKFGAISSVLNDILSRLDPPEAEVQIDRLITGRLQ
SLQTYVTQQLIRAAEIRASANLAATKMSECVLGQSKRVDFCGKGYHLMSFPQSAPHGVVFL
HVTYVPAQEKNFTTAPAICHDGKAHFPREGVFVSNGTHWFVTQRNFYEPQIITTDNTFVSG
NCDVVIGIVNNTVYDPLQPELDSFKEELDKYFKNHTSPDVDLGDISGINASVVNIQKEIDRL
NEVAKNLNESLIDLQELGKYEQYIKWPWYIWLGFIAGLIAIVMVTIMLCCMTSCCSCLKGC
 CSCGSCCKFDEDDSEPVLKGVKLHYT
**SEQ ID NO.31**, Sequence based on optimized and designed full-length S protein of Omicron_XBB.2 (including native signal peptide), serving as amino acid sequence of S antigen for Ad-S-XBB.2 vaccine 
MFVFLVLLPLVSSQCVNLITRTQSYTNSFTRGVYYPDKVFRSSVLHSTQDLFLPFFSNVTWF
RAIHVSGTNGTKRFDNPALPFNDGVYFASTEKSNIIRGWIFGTTLDSKTQSLLIVNNATNVVI
KVCEFQFCNDPFLDVYQKNNKSWMESEFRVYSSANNCTFEYVSQPFLMDLEGKEGNFKNL
REFVFKNIDGYFKIYSKHTPINLERDLPQGFSALEPLVDLPIGINITRFQTLLALHRSYLTPGGS
SSGWTAGAAAYYVGYLQPRTFLLKYNENGTITDAVDCALDPLSETKCTLKSFTVEKGIYQT
SNFRVQPTESIVRFPNITNLCPFHEVFNATTFASVYAWNRKRISNCVADYSVIYNFAPFFAFKC
YGVSPTKLNDLCFTNVYADSFVIRGNEVSQIAPGQTGNIADYNYKLPDDFTGCVIAWNSNK
LDSKPSGNYNYLYRLFRKSKLKPFERDISTEIYQAGNKPCNGVAGSNCYSPLQSYGFRPTYG
VGHQPYRVVVLSFELLHAPATVCGPKKSTNLVKNKCVNFNFNGLTGTGVLTESNKKFLPFQ
QFGRDIADTTDAVRDPQTLEILDITPCSFGGVSVITPGTNTSNQVAVLYQGVNCTEVPVAIHA
DQLTPTWRVYSTGSNVFQTRAGCLIGAEYVNNSYECDIPIGAGICASYQTQTKSHGSASSVA
SQSIIAYTMSLGAENSVAYSNNSIAIPTNFTISVTTEILPVSMTKTSVDCTMYICGDSTECSNLL
LQYGSFCTQLKRALTGIAVEQDKNTQEVFAQVKQIYKTPPIKYFGGFNFSQILPDPSKPSKRS
PIEDLLFNKVTLADAGFIKQYGDCLGDIAARDLICAQKFNGLTVLPPLLTDEMIAQYTSALL
AGTITSGWTFGAGPALQIPFPMQMAYRFNGIGVTQNVLYENQKLIANQFNSAIGKIQDSLSS
TPSALGKLQDVVNHNAQALNTLVKQLSSKFGAISSVLNDILSRLDPPEAEVQIDRLITGRLQ
SLQTYVTQQLIRAAEIRASANLAATKMSECVLGQSKRVDFCGKGYHLMSFPQSAPHGVVFL
HVTYVPAQEKNFTTAPAICHDGKAHFPREGVFVSNGTHWFVTQRNFYEPQIITTDNTFVSG
NCDVVIGIVNNTVYDPLQPELDSFKEELDKYFKNHTSPDVDLGDISGINASVVNIQKEIDRL
NEVAKNLNESLIDLQELGKYEQYIKWPWYIWLGFIAGLIAIVMVTIMLCCMTSCCSCLKGC
CSCGSCCKFDEDDSEPVLKGVKLHYT
**SEQ ID NO.32,** Sequence based on optimized and designed full-length S protein of Omicron_XBB.1.5 (including native signal peptide), serving as amino acid sequence of S antigen for Ad-S-XBB.1.5 vaccine 
MFVFLVLLPLVSSQCVNLITRTQSYTNSFTRGVYYPDKVFRSSVLHSTQDLFLPFFSNVTWF
HAIHVSGTNGTKRFDNPALPFNDGVYFASTEKSNIIRGWIFGTTLDSKTQSLLIVNNATNVVI
KVCEFQFCNDPFLDVYQKNNKSWMESEFRVYSSANNCTFEYVSQPFLMDLEGKEGNFKNL
REFVFKNIDGYFKIYSKHTPINLERDLPQGFSALEPLVDLPIGINITRFQTLLALHRSYLTPVDS
SSGWTAGAAAYYVGYLQPRTFLLKYNENGTITDAVDCALDPLSETKCTLKSFTVEKGIYQT
 
SNFRVQPTESIVRFPNITNLCPFHEVFNATTFASVYAWNRKRISNCVADYSVIYNFAPFFAFKC
YGVSPTKLNDLCFTNVYADSFVIRGNEVSQIAPGQTGNIADYNYKLPDDFTGCVIAWNSNK
LDSKPSGNYNYLYRLFRKSKLKPFERDISTEIYQAGNKPCNGVAGPNCYSPLQSYGFRPTYG
VGHQPYRVVVLSFELLHAPATVCGPKKSTNLVKNKCVNFNFNGLTGTGVLTESNKKFLPFQ
QFGRDIADTTDAVRDPQTLEILDITPCSFGGVSVITPGTNTSNQVAVLYQGVNCTEVPVAIHA
DQLTPTWRVYSTGSNVFQTRAGCLIGAEYVNNSYECDIPIGAGICASYQTQTKSHRRARSVA
SQSIIAYTMSLGAENSVAYSNNSIAIPTNFTISVTTEILPVSMTKTSVDCTMYICGDSTECSNLL
LQYGSFCTQLKRALTGIAVEQDKNTQEVFAQVKQIYKTPPIKYFGGFNFSQILPDPSKPSKRS
FIEDLLFNKVTLADAGFIKQYGDCLGDIAARDLICAQKFNGLTVLPPLLTDEMIAQYTSALL
AGTITSGWTFGAGAALQIPFAMQMAYRFNGIGVTQNVLYENQKLIANQFNSAIGKIQDSLSS
TASALGKLQDVVNHNAQALNTLVKQLSSKFGAISSVLNDILSRLDKVEAEVQIDRLITGRLQ
SLQTYVTQQLIRAAEIRASANLAATKMSECVLGQSKRVDFCGKGYHLMSFPQSAPHGVVFL
HVTYVPAQEKNFTTAPAICHDGKAHFPREGVFVSNGTHWFVTQRNFYEPQIITTDNTFVSG
NCDVVIGIVNNTVYDPLQPELDSFKEELDKYFKNHTSPDVDLGDISGINASVVNIQKEIDRL
NEVAKNLNESLIDLQELGKYEQYIKWPWYIWLGFIAGLIAIVMVTIMLCCMTSCCSCLKGC
CSCGSCCKFDEDDSEPVLKGVKLHYT
**SEQ ID NO.33,** Sequence based on optimized and designed full-length S protein of Omicron_XBB.2.3 (including native signal peptide), serving as amino acid sequence of S antigen for Ad-S-XBB.2.3 vaccine 
MFVFLVLLPLVSSQCVNLITRTQSYTNSFTRGVYYPDKVFRSSVLHSTQDLFLPFFSNVTWF
HAIHVSGTNGTKRFDNPALPFNDGVYFASTEKSNIIRGWIFGTTLDSKTQSLLIVNNATNVVI
KVCEFQFCNDPFLDVYQKNNKSWMESEFRVYSSANNCTFEYVSQPFLMDLEGKEGNFKNL
REFVFKNIDGYFKIYSKHTPINLERDLPQGFSALEPLVDLPIGINITRFQTLLALHRSYLTPGGS
SSGWTAGAAAYYVGYLQPRTFLLKYNENGTITDAVDCALDPLSETKCTLKSFTVEKGIYQT
SNFRVQPTESIVRFPNITNLCPFHEVFNATTFASVYAWNRKRISNCVADYSVIYNFAPFFAFKC
YGVSPTKLNDLCFTNVYADSFVIRGNEVSQIAPGQTGNIADYNYKLPDDFTGCVIAWNSNK
LDSKPSGNYNYLYRLFRKSKLKPFERDISTEIYQAGNKPCNGVAGPNCYSPLQSYGFRPTYG
VGHQPYRVVVLSFELLHASATVCGPKKSTNLVKNKCVNFNFNGLTGTGVLTESNKKFLPFQ
QFGRDIADTTDAVRDPQTLEILDITPCSFGGVSVITPGTNTSNQVAVLYQGVNCTEVPVAIHA
DQLTPTWRVYSTGSNVFQTRAGCLIGAEYVNNSYECDIPIGAGICASYQTQTKSHGSASSVA
SQSIIAYTMSLGAENSVAYSNNSIAIPTNFTISVTTEILPVSMTKTSVDCTMYICGDSTECSNLL
LQYGSFCTQLKRALTGIAVEQDKNTQEVFAQVKQIYKTPPIKYFGGFNFSQILPDPSKPSKRS
PIEDLLFNKVTLADAGFIKQYGDCLGDIAARDLICAQKFNGLTVLPPLLTDEMIAQYTSALL
AGTITSGWTFGAGPALQIPFPMQMAYRFNGIGVTQNVLYENQKLIANQFNSAIGKIQDSLSS
 
TPSALGKLQDVVNHNAQALNTLVKQLSSKFGAISSVLNDILSRLDPPEAEVQIDRLITGRLQ
SLQTYVTQQLIRAAEIRASANLAATKMSECVLGQSKRVDFCGKGYHLMSFPQSAPHGVVFL
HVTYVPAQEKNFTTAPAICHDGKAHFPREGVFVSNGTHWFVTQRNFYEPQIITTDNTFVSG
NCDVVIGIVNNTVYDPLQPELDSFKEELDKYFKNHTSPDVDLGDISGINASVVNIQKEIDRL
NEVAKNLNESLIDLQELGKYEQYIKWPWYIWLGFIAGLIAIVMVTIMLCCMTSCCSCLKGC
CSCGSCCKFDEDDSEPVLKGVKLHYT
**SEQ ID NO.34,** Sequence based on optimized and designed full-length S protein of Omicron_XBB.1.6 (including native signal peptide), serving as amino acid sequence of S antigen for Ad-S-XBB.1.6 vaccine
**SEQ ID NO.35,** Sequence based on optimized and designed full-length S protein of Omicron_XBB.1.9.1 (including native signal peptide), serving as amino acid sequence of S antigen for Ad-S-XBB.1.9.1 vaccine
**SEQ ID NO.36,** Sequence based on optimized and designed full-length S protein of Omicron_EG5 (including native signal peptide), serving as amino acid sequence of S antigen for Ad-S-EG5 vaccine
**SEQ ID NO.37,** Nucleotide sequence encoding sequence based on optimized and designed S protein-RBD-HR of Omicron_XBB.1.16 and nucleotide sequence encoding SEQ ID NO.19 
ATGCTACTAGTAAATCAGTCACACCAAGGTTTCAACAAGGAACACACCAGCAAGATGGT
GAGCGCTATCGTGCTGTACGTCCTGCTGGCCGCTGCTGCTCACAGCGCTTTCGCTGCTG
ACTCCATCCACATCAAGGACAGCGACGACCTGAAGAACCGTCTGGCCGAGGCCGGTGA
CAAGCTGGTCGTCATCGACTTCATGGCCACTTGGTGCGGTCCTTGCAAGATGATCGGCC
CTAAGCTGGACGAGATGGCTAACGAGATGTCCGACAGCATCGTGGTCCTGAAGGTGGA
CGTCGACGAGTGCGAGGACATCGCCACCGAATACAACATCAACAGCATGCCCACCTTCG
TGTTCGTGAAGAACAGCAAGAAGATCGAGGAATTTTCCGGCGCTAACGTCGACAAGCT
GCGTAACACCATCATCAAGCTGAAGCTGGCCGGCTCCGGCTCCGGCCACATGCATCACC
ACCACCACCATTCCTCCGGTGACGACGACGACAAGGTGCAGCCAACCGAATCTATCGTC
AGATTCCCAAACATCACTAACCTGTGCCCTTTCCACGAGGTGTTCAACGCTACCACTTTC
GCCAGCGTCTACGCTTGGAACCGCAAGCGTATCAGCAACTGCGTCGCCGACTACTCTGT
GATCTACAACTTCGCTCCTTTCTTCGCTTTCAAGTGCTACGGCGTGTCACCTACCAAGCT
GAACGACCTGTGCTTCACTAACGTCTACGCCGACTCCTTCGTGATCCGCGGAAACGAAG
TCTCTCAGATCGCTCCTGGACAGACCGGAAATATCGCTGACTACAACTACAAGCTGCCA
GACGACTTCACTGGCTGCGTGATCGCTTGGAACTCAAACAAGCTGGACTCCAAGCCTTC
TGGCAACTACAACTACCTGTACAGGCTGTTCAGAAAGTCAAAGCTGAAGCCTTTCGAGC
 
GCGACATCTCAACCGAAATCTACCAGGCTGGTAATCGACCCTGCAACGGTGTGGCTGGC
CCTAACTGCTACTCTCCCCTGCAGTCCTACGGTTTCCGTCCAACCTACGGAGTCGGTCAT
CAGCCTTACCGTGTGGTCGTGCTGAGCTTCGAACTGCTCCACGCTCCTGCTACTGTGTG
CGGTCCCAAGAAGTCTACTAACCTGGTCAAAAACAAAAGCGTCAACTTCAACTTCAAC
GGTCTGTACGAGAACCAGAAGCTGATCGCTAACCAGTTCAACAGCGCCATCGGCAAGAT
CCAGGACTCACTGTCATCCACTGCCTCCGCTCTGGGAAAGCTGCAGGACGTCGTGAACC
AGAACGCCCAGGCTCTGAACACCCTGGTCAAGCAGCTGAAGAACCACACCTCTCCTGA
CGTCGACCTGGGCGACATCAGCGGAATCAACGCTTCTGTCGTGAACATCCAGAAGGAG
ATCGACCGCCTGAACGAAGTGGCCAAGAACCTGAACGAATCCCTGATTGACCTCCAAG
AACTCTAA
**SEQ ID NO.38,** Nucleotide sequence encoding sequence based on optimized and designed S protein-RBD-HR of Omicron_XBB.2 and nucleotide sequence encoding SEQ ID NO.20 
ATGCTACTAGTAAATCAGTCACACCAAGGTTTCAACAAGGAACACACCAGCAAGATGGT
GAGCGCTATCGTGCTGTACGTCCTGCTGGCCGCTGCTGCTCACAGCGCTTTCGCTGCTG
ACTCCATCCACATCAAGGACAGCGACGACCTGAAGAACCGTCTGGCCGAGGCCGGTGA
CAAGCTGGTCGTCATCGACTTCATGGCCACTTGGTGCGGTCCTTGCAAGATGATCGGCC
CTAAGCTGGACGAGATGGCTAACGAGATGTCCGACAGCATCGTGGTCCTGAAGGTGGA
CGTCGACGAGTGCGAGGACATCGCCACCGAATACAACATCAACAGCATGCCCACCTTCG
TGTTCGTGAAGAACAGCAAGAAGATCGAGGAATTTTCCGGCGCTAACGTCGACAAGCT
GCGTAACACCATCATCAAGCTGAAGCTGGCCGGCTCCGGCTCCGGCCACATGCATCACC
ACCACCACCATTCCTCCGGTGACGACGACGACAAGGTGCAGCCAACCGAATCTATCGTC
AGATTCCCAAACATCACTAACCTGTGCCCTTTCCACGAGGTGTTCAACGCTACCACTTTC
GCCAGCGTCTACGCTTGGAACCGCAAGCGTATCAGCAACTGCGTCGCCGACTACTCTGT
GATCTACAACTTCGCTCCTTTCTTCGCTTTCAAGTGCTACGGCGTGTCACCTACCAAGCT
GAACGACCTGTGCTTCACTAACGTCTACGCCGACTCCTTCGTGATCCGCGGAAACGAAG
TCTCTCAGATCGCTCCTGGACAGACCGGAAATATCGCTGACTACAACTACAAGCTGCCA
GACGACTTCACTGGCTGCGTGATCGCTTGGAACTCAAACAAGCTGGACTCCAAGCCTTC
TGGCAACTACAACTACCTGTACAGGCTGTTCAGAAAGTCAAAGCTGAAGCCTTTCGAGC
GCGACATCTCAACCGAAATCTACCAGGCTGGTAATAAGCCCTGCAACGGTGTGGCTGGC
TCTAACTGCTACTCTCCCCTGCAGTCCTACGGTTTCCGTCCAACCTACGGAGTCGGTCAT
CAGCCTTACCGTGTGGTCGTGCTGAGCTTCGAACTGCTCCACGCTCCTGCTACTGTGTG
CGGTCCCAAGAAGTCTACTAACCTGGTCAAAAACAAAAGCGTCAACTTCAACTTCAAC
GGTCTGTACGAGAACCAGAAGCTGATCGCTAACCAGTTCAACAGCGCCATCGGCAAGAT
CCAGGACTCACTGTCATCCACTGCCTCCGCTCTGGGAAAGCTGCAGGACGTCGTGAACC
 
AGAACGCCCAGGCTCTGAACACCCTGGTCAAGCAGCTGAAGAACCACACCTCTCCTGA
CGTCGACCTGGGCGACATCAGCGGAATCAACGCTTCTGTCGTGAACATCCAGAAGGAG
ATCGACCGCCTGAACGAAGTGGCCAAGAACCTGAACGAATCCCTGATTGACCTCCAAG
AACTCTAA
**SEQ ID NO.39,** Nucleotide sequence encoding sequence based on optimized and designed S protein-RBD-HR of Omicron_XBB.1.5-1 and nucleotide sequence encoding SEQ ID NO.21 
ATGCTACTAGTAAATCAGTCACACCAAGGTTTCAACAAGGAACACACCAGCAAGATGGT
GAGCGCTATCGTGCTGTACGTCCTGCTGGCCGCTGCTGCTCACAGCGCTTTCGCTGCTG
ACTCCATCCACATCAAGGACAGCGACGACCTGAAGAACCGTCTGGCCGAGGCCGGTGA
CAAGCTGGTCGTCATCGACTTCATGGCCACTTGGTGCGGTCCTTGCAAGATGATCGGCC
CTAAGCTGGACGAGATGGCTAACGAGATGTCCGACTGCATCGTGGTCCTGAAGGTGGAC
GTCGACGAGTGCGAGGACATCGCCACCGAATACAACATCAACAGCATGCCCACCTTCGT
GTTCGTGAAGAACAGCAAGAAGATCGAGGAATTTTCCGGCGCTAACGTCGACAAGCTG
CGTAACACCATCATCAAGCTGAAGCTGGCCGGCTCCGGCTCCGGCCACATGCATCACCA
CCACCACCATTCCTCCGGTGACGACGACGACAAGGTGCAGCCAACCGAATCTATCGTCA
GATTCCCAAACATCACTAACCTGTGCCCTTTCCACGAGGTGTTCAACGCTACCACTTTCG
CCAGCGTCTACGCTTGGAACCGCAAGCGTATCAGCAACTGCGTCGCCGACTACTCTGTG
ATCTACAACTTCGCTCCTTTCTTCGCTTTCAAGTGCTACGGCGTGTCACCTACCAAGCTG
AACGACCTGTGCTTCACTAACGTCTACGCCGACTCCTTCGTGATCCGCGGAAACGAAGT
CTCTCAGATCGCTCCTGGACAGACCGGAAATATCGCTGACTACAACTACAAGCTGCCAG
ACGACTTCACTGGCTGCGTGATCGCTTGGAACTCAAACAAGCTGGACTCCAAGCCTTCT
GGCAACTACAACTACCTGTACAGGCTGTTCAGAAAGTCAAAGCTGAAGCCTTTCGAGC
GCGACATCTCAACCGAAATCTACCAGGCTGGTAATAAGCCCTGCAACGGTGTGGCTGGC
CCTAACTGCTACTCTCCCCTGCAGTCCTACGGTTTCCGTCCAACCTACGGAGTCGGTCAT
CAGCCTTACCGTGTGGTCGTGCTGAGCTTCGAACTGCTCCACGCTCCTGCTACTGTGTG
CGGTCCCAAGAAGTCTACTAACCTGGTCAAAAACAAATGTGTCAACTTCAACTTCAACG
GTTAA
**SEQ ID NO.40,** Nucleotide sequence encoding sequence based on optimized and designed S protein-RBD-HR of Omicron_XBB.1.5-2 and nucleotide sequence encoding SEQ ID NO.22 
ATGCTACTAGTAAATCAGTCACACCAAGGTTTCAACAAGGAACACACCAGCAAGATGGT
GAGCGCTATCGTGCTGTACGTCCTGCTGGCCGCTGCTGCTCACAGCGCTTTCGCTGCTG
ACTCCATCCACATCAAGGACAGCGACGACCTGAAGAACCGTCTGGCCGAGGCCGGTGA
CAAGCTGGTCGTCATCGACTTCATGGCCACTTGGTGCGGTCCTTGCAAGATGATCGGCC
 
CTAAGCTGGACGAGATGGCTAACGAGATGTCCGACTGCATCGTGGTCCTGAAGGTGGAC
GTCGACGAGTGCGAGGACATCGCCACCGAATACAACATCAACAGCATGCCCACCTTCGT
GTTCGTGAAGAACAGCAAGAAGATCGAGGAATTTTCCGGCGCTAACGTCGACAAGCTG
CGTAACACCATCATCAAGCTGAAGCTGGCCGGCTCCGGCTCCGGCCACATGCATCACCA
CCACCACCATTCCTCCGGTGACGACGACGACAAGGTGCAGCCAACCGAATCTATCGTCA
GATTCCCAAACATCACTAACCTGTGCCCTTTCCACGAGGTGTTCAACGCTACCACTTTCG
CCAGCGTCTACGCTTGGAACCGCAAGCGTATCAGCAACTGCGTCGCCGACTACTCTGTG
ATCTACAACTCCGCTAGCTTCTCTGCTTTCAAGTGCTACGGCGTGTCACCTACCAAGCTG
AACGACCTGTGCTTCACTAACGTCTACGCCGACTCCTTCGTGATCCGCGGAAACGAAGT
CTCTCAGATCGCTCCTGGACAGACCGGAAATATCGCTGACTACAACTACAAGCTGCCAG
ACGACTTCACTGGCTGCGTGATCGCTTGGAACTCAAACAAGCTGGACTCCAAGCCTTCT
GGCAACTACAACTACCTGTACAGGCTGTTCAGAAAGTCAAAGCTGAAGCCTTTCGAGC
GCGACATCTCAACCGAAATCTACCAGGCTGGTAATAAGCCCTGCAACGGTGTGGCTGGC
CCTAACTGCTACTCTCCCCTGCAGTCCTACGGTTTCCGTCCAACCTACGGAGTCGGTCAT
CAGCCTTACCGTGTGGTCGTGCTGAGCTTCGAACTGCTCCACGCTCCTGCTACTGTGTG
CGGTCCCAAGAAGTCTACTAACCTGGTCAAAAACAAATGTGTCAACTTCAACTTCAACG
GTTAA
**SEQ ID NO.41,** Nucleotide sequence encoding sequence based on optimized and designed S protein-RBD-HR of Omicron_XBB.1.5-3 and nucleotide sequence encoding SEQ ID NO.23 
ATGCTGCTGGTGAACCAGAGCCACCAGGGTTTCAACAAGGAACACACTAGCAAGATGG
TGTCTGCTATCGTCCTCTACGTGCTGCTCGCCGCTGCTCACTCAGCTTTCGCCGCTG
ACTCTATCCACATCAAGGACTCCGACGACCTCAAGAACCGTTTGGCCGAAGCCGGCGAC
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCAC
CACCACCATTCAAGCGGCGACGACGACGACAAGGTGCAGCCAACCGAATCAATCGTCC
GCTTCCCAAACATCACTAACCTGTGCCCATTCCACGAGGTGTTCAACGCCACCACTTTC
GCCTCTGTCTACGCTTGGAACCGCAAGCGCATCTCCAACTGTGTCGCCGACTACAGCGT
GATATACAACTTCGCTCCTTTCTTCGCATTCAAGTGTTACGGAGTGTCCCCTACCAAGCT
CAACGATTTGTGCTTCACAAACGTGTACGCTGACAGCTTCGTGATCCGTGGTAACGAGG
TTTCTCAAATCGCCCCTGGTCAGACAGGTAACATCGCTGACTACAACTACAAGCTCCCA
GACGACTTCACTGGCTGTGTTATCGCTTGGAACTCAAACAAGCTGGACTCTAAGCCGTC
 
TGGTAACTACAACTACCTCTACCGTCTGTTCCGCAAGAGCAAGCTCAAGCCATTCGAAC
GTGACATCAGCACAGAGATCTACCAAGCCGGTAACAAGCCCTGCAACGGCGTTGCCGG
ACCTAACTGCTACTCTCCCCTCCAGTCCTACGGATTCCGCCCAACCTACGGCGTGGGTCA
CCAGCCATACCGCGTGGTTGTGCTGAGCTTCGAACTGCTGCACGCCCCCGCTACCGTGT
GTGGCCCTAAGAAGAGCACTAACCTCGTTAAGAACAAGTCCGTGAACTTCAACTTCAAC
GGTCTCTACGAAAACCAGAAGCTGATCGCTAACCAGTTCAACTCCGCCATCGGCAAGAT
CCAGGACTCCCTGTCCTCCACAGCTTCCGCTCTGGGCAAGCTGCAGGACGTTGTGAACC
AAAACGCTCAGGCCCTGAACACACTGGTGAAGCAACTGAAGAACCACACCAGCCCCG
ACGTTGACCTGGGTGACATCAGCGGTATCAACGCTAGCGTGGTGAACATCCAGAAGGA
GATCGACAGGCTGAACGAAGTGGCTAAGAACTTGAACGAGAGCCTCATCGACCTGCAG
GAACTGTAA
**SEQ ID NO.42,** Nucleotide sequence encoding sequence based on optimized and designed S protein-RBD-HR of Omicron_XBB.1.5-4 and nucleotide sequence encoding SEQ ID NO.24 
ATGCTGCTGGTGAACCAGAGCCACCAGGGTTTCAACAAGGAACACACTAGCAAGATGG
TGTCTGCTATCGTCCTCTACGTGCTGCTCGCCGCCGCTGCTCACTCAGCTTTCGCCGCTG
ACTCTATCCACATCAAGGACTCCGACGACCTCAAGAACCGTTTGGCCGAAGCCGGCGAC
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCAC
CACCACCATTCAAGCGGCGACGACGACGACAAGGTGCAGCCAACCGAATCAATCGTCC
GCTTCCCAAACATCACTAACCTGTGCCCATTCCACGAGGTGTTCAACGCCACCACTTTC
GCCTCTGTCTACGCTTGGAACCGCAAGCGCATCTCCAACTGTGTCGCCGACTACAGCGT
GATATACAACTCCGCTTCTTTCTCTGCATTCAAGTGTTACGGAGTGTCCCCTACCAAGCT
CAACGATTTGTGCTTCACAAACGTGTACGCTGACAGCTTCGTGATCCGTGGTAACGAGG
TTTCTCAAATCGCCCCTGGTCAGACAGGTAACATCGCTGACTACAACTACAAGCTCCCA
GACGACTTCACTGGCTGTGTTATCGCTTGGAACTCAAACAAGCTGGACTCTAAGCCGTC
TGGTAACTACAACTACCTCTACCGTCTGTTCCGCAAGAGCAAGCTCAAGCCATTCGAAC
GTGACATCAGCACAGAGATCTACCAAGCCGGTAACAAGCCCTGCAACGGCGTTGCCGG
ACCTAACTGCTACTCTCCCCTCCAGTCCTACGGATTCCGCCCAACCTACGGCGTGGGTCA
CCAGCCATACCGCGTGGTTGTGCTGAGCTTCGAACTGCTGCACGCCCCCGCTACCGTGT
GTGGCCCTAAGAAGAGCACTAACCTCGTTAAGAACAAGTCCGTGAACTTCAACTTCAAC
GGTCTCTACGAAAACCAGAAGCTGATCGCTAACCAGTTCAACTCCGCCATCGGCAAGAT
 
CCAGGACTCCCTGTCCTCCACAGCTTCCGCTCTGGGCAAGCTGCAGGACGTTGTGAACC
AAAACGCTCAGGCCCTGAACACACTGGTGAAGCAACTGAAGAACCACACCAGCCCCG
ACGTTGACCTGGGTGACATCAGCGGTATCAACGCTAGCGTGGTGAACATCCAGAAGGA
GATCGACAGGCTGAACGAAGTGGCTAAGAACTTGAACGAGAGCCTCATCGACCTGCAG
GAACTGTAA
**SEQ ID NO.43,** Nucleotide sequence encoding sequence based on optimized and designed S protein-RBD of Omicron_XBB.2.3-1 and nucleotide sequence encoding SEQ ID NO.25 
ATGCTACTAGTAAATCAGTCACACCAAGGTTTCAACAAGGAACACACCAGCAAGATGGT
GAGCGCTATCGTGCTGTACGTCCTGCTGGCCGCTGCTGCTCACAGCGCTTTCGCTGCTG
ACTCCATCCACATCAAGGACAGCGACGACCTGAAGAACCGTCTGGCCGAGGCCGGTGA
CAAGCTGGTCGTCATCGACTTCATGGCCACTTGGTGCGGTCCTTGCAAGATGATCGGCC
CTAAGCTGGACGAGATGGCTAACGAGATGTCCGACTGCATCGTGGTCCTGAAGGTGGAC
GTCGACGAGTGCGAGGACATCGCCACCGAATACAACATCAACAGCATGCCCACCTTCGT
GTTCGTGAAGAACAGCAAGAAGATCGAGGAATTTTCCGGCGCTAACGTCGACAAGCTG
CGTAACACCATCATCAAGCTGAAGCTGGCCGGCTCCGGCTCCGGCCACATGCATCACCA
CCACCACCATTCCTCCGGTGACGACGACGACAAGGTGCAGCCAACCGAATCTATCGTCA
GATTCCCAAACATCACTAACCTGTGCCCTTTCCACGAGGTGTTCAACGCTACCACTTTCG
CCAGCGTCTACGCTTGGAACCGCAAGCGTATCAGCAACTGCGTCGCCGACTACTCTGTG
ATCTACAACTTCGCTCCTTTCTTCGCTTTCAAGTGCTACGGCGTGTCACCTACCAAGCTG
AACGACCTGTGCTTCACTAACGTCTACGCCGACTCCTTCGTGATCCGCGGAAACGAAGT
CTCTCAGATCGCTCCTGGACAGACCGGAAATATCGCTGACTACAACTACAAGCTGCCAG
ACGACTTCACTGGCTGCGTGATCGCTTGGAACTCAAACAAGCTGGACTCCAAGCCTTCT
GGCAACTACAACTACCTGTACAGGCTGTTCAGAAAGTCAAAGCTGAAGCCTTTCGAGC
GCGACATCTCAACCGAAATCTACCAGGCTGGTAATAAGCCCTGCAACGGTGTGGCTGGC
CCTAACTGCTACTCTCCCCTGCAGTCCTACGGTTTCCGTCCAACCTACGGAGTCGGTCAT
CAGCCTTACCGTGTGGTCGTGCTGAGCTTCGAACTGCTCCACGCTTCTGCTACTGTGTGC
GGTCCCAAGAAGTCTACTAACCTGGTCAAAAACAAATGTGTCAACTTCAACTTCAACGG
TTAA
**SEQ ID NO.44,** Nucleotide sequence encoding sequence based on optimized and designed S protein-RBD-HR of Omicron_XBB.2.3-2 and nucleotide sequence encoding SEQ ID NO.26 
ATGCTGCTGGTGAACCAGAGCCACCAGGGTTTCAACAAGGAACACACTAGCAAGATGG
TGTCTGCTATCGTCCTCTACGTGCTGCTCGCCGCCGCTGCTCACTCAGCTTTCGCCGCTG
ACTCTATCCACATCAAGGACTCCGACGACCTCAAGAACCGTTTGGCCGAAGCCGGCGAC
 
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCAC
CACCACCATTCAAGCGGCGACGACGACGACAAGGTGCAGCCAACCGAATCAATCGTCC
GCTTCCCAAACATCACTAACCTGTGCCCATTCCACGAGGTGTTCAACGCCACCACTTTC
GCCTCTGTCTACGCTTGGAACCGCAAGCGCATCTCCAACTGTGTCGCCGACTACAGCGT
GATATACAACTTCGCTCCTTTCTTCGCATTCAAGTGTTACGGAGTGTCCCCTACCAAGCT
CAACGATTTGTGCTTCACAAACGTGTACGCTGACAGCTTCGTGATCCGTGGTAACGAGG
TTTCTCAAATCGCCCCTGGTCAGACAGGTAACATCGCTGACTACAACTACAAGCTCCCA
GACGACTTCACTGGCTGTGTTATCGCTTGGAACTCAAACAAGCTGGACTCTAAGCCGTC
TGGTAACTACAACTACCTCTACCGTCTGTTCCGCAAGAGCAAGCTCAAGCCATTCGAAC
GTGACATCAGCACAGAGATCTACCAAGCCGGTAACAAGCCCTGCAACGGCGTTGCCGG
ACCTAACTGCTACTCTCCCCTCCAGTCCTACGGATTCCGCCCAACCTACGGCGTGGGTCA
CCAGCCATACCGCGTGGTTGTGCTGAGCTTCGAACTGCTGCACGCCTCCGCTACCGTGT
GTGGCCCTAAGAAGAGCACTAACCTCGTTAAGAACAAGTCCGTGAACTTCAACTTCAAC
GGTCTCTACGAAAACCAGAAGCTGATCGCTAACCAGTTCAACTCCGCCATCGGCAAGAT
CCAGGACTCCCTGTCCTCCACAGCTTCCGCTCTGGGCAAGCTGCAGGACGTTGTGAACC
AAAACGCTCAGGCCCTGAACACACTGGTGAAGCAACTGAAGAACCACACCAGCCCCG
ACGTTGACCTGGGTGACATCAGCGGTATCAACGCTAGCGTGGTGAACATCCAGAAGGA
GATCGACAGGCTGAACGAAGTGGCTAAGAACTTGAACGAGAGCCTCATCGACCTGCAG
GAACTGTAA
**SEQ ID NO.45,** Nucleotide sequence encoding sequence based on optimized and designed S protein-RBD-HR of Omicron_XBB.1.16.6 and nucleotide sequence encoding SEQ ID NO.27 
ATGCTGCTGGTGAACCAGAGCCACCAGGGTTTCAACAAGGAACACACTAGCAAGATGG
TGTCTGCTATCGTCCTCTACGTGCTGCTCGCCGCCGCTGCTCACTCAGCTTTCGCCGCTG
ACTCTATCCACATCAAGGACTCCGACGACCTCAAGAACCGTTTGGCCGAAGCCGGCGAC
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCACC
ACCACCATTCAAGCGGCGACGACGACGACAAGGTGCAGCCAACCGAATCAATCGTCCG
 
CTTCCCAAACATCACTAACCTGTGCCCATTCCACGAGGTGTTCAACGCCACCACTTTCGC
CTCTGTCTACGCTTGGAACCGCAAGCGCATCTCCAACTGTGTCGCCGACTACAGCGTGA
TATACAACTTCGCTCCTTTCTTCGCATTCAAGTGTTACGGAGTGTCCCCTACCAAGCTCA
ACGATTTGTGCTTCACAAACGTGTACGCTGACAGCTTCGTGATCCGTGGTAACGAGGTT
TCTCAAATCGCCCCTGGTCAGACAGGTAACATCGCTGACTACAACTACAAGCTCCCAGA
CGACTTCACTGGCTGTGTTATCGCTTGGAACTCAAACAAGCTGGACTCTAAGCCGTCTG
GTAACTACAACTACCTCTACCGTCTGCTCCGCAAGAGCAAGCTCAAGCCATTCGAACGT
GACATCAGCACAGAGATCTACCAAGCCGGTAACCGCCCCTGCAACGGCGTTGCCGGAC
CTAACTGCTACTCTCCCCTCCAGTCCTACGGATTCCGCCCAACCTACGGCGTGGGTCACC
AGCCATACCGCGTGGTTGTGCTGAGCTTCGAACTGCTGCACGCCCCCGCTACCGTGTGT
GGCCCTAAGAAGAGCACTAACCTCGTTAAGAACAAGTCCGTGAACTTCAACTTCAACG
GTCTCTACGAAAACCAGAAGCTGATCGCTAACCAGTTCAACTCCGCCATCGGCAAGATC
CAGGACTCCCTGTCCTCCACAGCTTCCGCTCTGGGCAAGCTGCAGGACGTTGTGAACCA
AAACGCTCAGGCCCTGAACACACTGGTGAAGCAACTGAAGAACCACACCAGCCCCGA
CGTTGACCTGGGTGACATCAGCGGTATCAACGCTAGCGTGGTGAACATCCAGAAGGAGA
TCGACAGGCTGAACGAAGTGGCTAAGAACTTGAACGAGAGCCTCATCGACCTGCAGGA
ACTGTAA
**SEQ ID NO.46,** Nucleotide sequence encoding sequence based on optimized and designed S protein-RBD-HR of Omicron _BA.2.86 and nucleotide sequence encoding SEQ ID NO.28 
ATGCTGCTGGTGAACCAGAGCCACCAGGGTTTCAACAAGGAACACACTAGCAAGATGG
TGTCTGCTATCGTCCTCTACGTGCTGCTCGCCGCTGCTCACTCAGCTTTCGCCGCTG
ACTCTATCCACATCAAGGACTCCGACGACCTCAAGAACCGTTTGGCCGAAGCCGGCGAC
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCACC
ACCACCATTCAAGCGGCGACGACGACGACAAGGTGCAGCCAACCGAATCAATCGTCCG
CTTCCCAAACGTCACTAACCTGTGCCCATTCCACGAGGTGTTCAACGCCACCCGTTTCG
CCTCTGTCTACGCTTGGAACCGCACGCGCATCTCCAACTGTGTCGCCGACTACAGCGTG
CTGTACAACTTCGCTCCTTTCTTCGCATTCAAGTGTTACGGAGTGTCCCCTACCAAGCTC
AACGATTTGTGCTTCACAAACGTGTACGCTGACAGCTTCGTGATCAAGGGTAACGAGGT
TTCTCAAATCGCCCCTGGTCAGACAGGTAACATCGCTGACTACAACTACAAGCTCCCAG
ACGACTTCACTGGCTGTGTTATCGCTTGGAACTCAAACAAGCTGGACTCTAAGCATTCT
 
GGTAACTACGACTACTGGTACCGTCTGTTCCGCAAGAGCAAGCTCAAGCCATTCGAACG
TGACATCAGCACAGAGATCTACCAAGCCGGTAACAAGCCCTGCAAGGGCAAGGGACCT
AACTGCTACTTCCCCCTCCAGTCCTACGGATTCCGCCCAACCTACGGCGTGGGTCACCA
GCCATACCGCGTGGTTGTGCTGAGCTTCGAACTGCTGCACGCCCCCGCTACCGTGTGTG
GCCCTAAGAAGAGCACTAACCTCGTTAAGAACAAGTCCGTGAACTTCAACTTCAACGGT
CTCTACGAAAACCAGAAGCTGATCGCTAACCAGTTCAACTCCGCCATCGGCAAGATCCA
GGACTCCCTGTCCTCCACAGCTTCCGCTCTGGGCAAGCTGCAGGACGTTGTGAACCAA
AACGCTCAGGCCCTGAACACACTGGTGAAGCAACTGAAGAACCACACCAGCCCCGAC
GTTGACCTGGGTGACATCAGCGGTATCAACGCTAGCGTGGTGAACATCCAGAAGGAGAT
CGACAGGCTGAACGAAGTGGCTAAGAACTTGAACGAGAGCCTCATCGACCTGCAGGAA
CTGTAA
**SEQ ID NO.47,** Nucleotide sequence encoding sequence based on optimized and designed S protein-RBD-HR of Omicron_EG.5 and nucleotide sequence encoding SEQ ID NO.29 
ATGCTGCTGGTGAACCAGAGCCACCAGGGTTTCAACAAGGAACACACTAGCAAGATGG
TGTCTGCTATCGTCCTCTACGTGCTGCTCGCCGCTGCTCACTCAGCTTTCGCCGCTG
ACTCTATCCACATCAAGGACTCCGACGACCTCAAGAACCGTTTGGCCGAAGCCGGCGAC
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCACC
ACCACCATTCAAGCGGCGACGACGACGACAAGGTGCAGCCAACCGAATCAATCGTCCG
CTTCCCAAACATCACTAACCTGTGCCCATTCCACGAGGTGTTCAACGCCACCACTTTCGC
CTCTGTCTACGCTTGGAACCGCAAGCGCATCTCCAACTGTGTCGCCGACTACAGCGTGA
TATACAACTTCGCTCCTTTCTTCGCATTCAAGTGTTACGGAGTGTCCCCTACCAAGCTCA
ACGATTTGTGCTTCACAAACGTGTACGCTGACAGCTTCGTGATCCGTGGTAACGAGGTT
TCTCAAATCGCCCCTGGTCAGACAGGTAACATCGCTGACTACAACTACAAGCTCCCAGA
CGACTTCACTGGCTGTGTTATCGCTTGGAACTCAAACAAGCTGGACTCTAAGCCGTCTG
GTAACTACAACTACCTCTACCGTCTGCTCCGCAAGAGCAAGCTCAAGCCATTCGAACGT
GACATCAGCACAGAGATCTACCAAGCCGGTAACAAGCCCTGCAACGGCGTTGCCGGAC
CTAACTGCTACTCTCCCCTCCAGTCCTACGGATTCCGCCCAACCTACGGCGTGGGTCACC
AGCCATACCGCGTGGTTGTGCTGAGCTTCGAACTGCTGCACGCCCCCGCTACCGTGTGT
GGCCCTAAGAAGAGCACTAACCTCGTTAAGAACAAGTCCGTGAACTTCAACTTCAACG
GTCTCTACGAAAACCAGAAGCTGATCGCTAACCAGTTCAACTCCGCCATCGGCAAGATC
 
CAGGACTCCCTGTCCTCCACAGCTTCCGCTCTGGGCAAGCTGCAGGACGTTGTGAACCA
AAACGCTCAGGCCCTGAACACACTGGTGAAGCAACTGAAGAACCACACCAGCCCCGA
CGTTGACCTGGGTGACATCAGCGGTATCAACGCTAGCGTGGTGAACATCCAGAAGGAGA
TCGACAGGCTGAACGAAGTGGCTAAGAACTTGAACGAGAGCCTCATCGACCTGCAGGA
ACTGTAA
**SEQ ID NO.48,** Nucleotide sequence encoding sequence based on optimized and designed full-length S protein of Omicron_XBB.1.16 (including native signal peptide, serving as nucleotide sequence of S antigen for Ad-S-XBB.1.16 vaccine) and nucleotide sequence encoding SEQ ID NO.30 
ATGTTCGTGTTCCTGGTGCTGCTGCCCCTGGTGAGCAGCCAATGCGTGAACCTGATCAC
AAGAACACAGAGCTACACCAACAGCTTCACAAGAGGCGTGTACTACCCCGACAAGGTG
TTCAGAAGCAGCGTGCTGCATTCCACCCAAGACCTGTTCCTGCCCTTCTTCAGCAACGT
GACCTGGTTCCACGCCATCCACGTGAGCGGCACCAACGGCACCAAGAGATTCGACAAC
CCCGCCCTGCCCTTCAACGACGGCGTGTACTTCGCTAGCACCGAGAAGAGCAACATCAT
CAGAGGCTGGATCTTCGGCACCACCCTGGACAGCAAAACACAGAGCCTGCTGATCGTG
AACAATGCCACCAACGTGGTGATCAAGGTGTGCGAGTTTCAGTTCTGCAACGACCCCTT
CCTGGACGTGTATCAGAAGAACAACAAGAGCTGGATGGAGAGCGAGTTCAGAGTGTAC
AGCAGCGCCAACAACTGCACCTTCGAGTACGTGAGCCAACCCTTCCTGATGGACCTGGT
GGGCAAGGAGGGCAACTTTAAGAACCTCAGAGAGTTCGTGTTCAAGAACATCGACGGC
TACTTCAAGATCTACAGCAAGCACACCCCCATCAACCTGGAGAGAGACCTGCCCCAAG
GCTTCAGCGCCCTGGAGCCCCTGGTGGACCTGCCCATCGGCATCAACATCACAAGATTT
CAGACCCTGCTGGCCCTGCACAGAAGCTATCTCACACCCGTCGATAGCAGCAGCGGGTG
GACAGCCGGGGCCGCTGCCTACTACGTGGGCTACCTGCAGCCTAGAACCTTCCTGCTGA
AGTACAACGAGAACGGCACAATCACCGACGCCGTCGACTGCGCCCTGGACCCCCTGAG
CGAGACCAAGTGCACCCTGAAGAGCTTCACCGTGGAGAAGGGCATCTATCAGACAAGC
AACTTCAGAGTGCAGCCCACCGAGAGCATCGTGAGATTCCCCAACATCACCAACCTGTG
CCCCTTCCACGAGGTGTTCAACGCCACCACCTTCGCTAGCGTGTACGCTTGGAATAGAA
AGAGAATCAGCAACTGCGTGGCCGACTACAGCGTGATCTACAACTTCGCCCCCTTCTTC
GCCTTCAAGTGTTACGGCGTGTCCCCCACCAAGCTGAACGACCTGTGCTTCACCAACGT
GTACGCCGACAGCTTCGTGATCAGAGGCAACGAGGTGAGCCAAATCGCCCCTGGGCAG
ACCGGCAACATCGCCGACTACAACTACAAGCTGCCCGACGACTTCACCGGCTGCGTGAT
CGCCTGGAACAGCAACAAGCTGGACTCCAAGCCTAGCGGGAATTACAACTACCTGTAC
AGACTGTTCAGAAAGAGCAAGCTGAAGCCCTTCGAGAGAGACATCAGCACCGAGATCT
ACCAAGCCGGCAACAGACCCTGCAACGGCGTGGCCGGCCCCAACTGCTACAGCCCCCT
 
GCAGAGCTACGGCTTCAGACCCACCTACGGCGTGGGCCATCAGCCCTACAGAGTGGTCG
TGCTGAGCTTCGAGCTGCTGCACGCCCCCGCCACCGTGTGCGGCCCCAAGAAGAGCAC
CAACCTGGTGAAGAACAAGTGCGTGAACTTCAACTTCAACGGGCTGACCGGCACCGGC
GTGCTGACCGAGAGCAACAAGAAGTTCCTGCCCTTTCAACAGTTCGGCAGAGACATCG
CCGACACCACCGATGCCGTGAGAGACCCTCAGACCCTGGAGATCCTGGACATCACCCCC
TGCAGCTTCGGCGGCGTGAGCGTGATCACCCCCGGCACCAACACAAGCAACCAAGTGG
CCGTGCTGTACCAAGGCGTGAACTGCACCGAGGTGCCCGTGGCCATCCACGCCGATCAG
CTGACCCCCACCTGGAGAGTGTATAGCACCGGCAGCAACGTGTTTCAGACAAGAGCCG
GCTGCCTGATCGGCGCCGAGTACGTGAACAACAGCTACGAGTGCGACATCCCCATCGGC
GCCGGCATCTGCGCTAGCTATCAGACACAGACCAAGAGCCACGGCAGCGCTAGCAGCG
TGGCTAGCCAAAGCATCATCGCCTACACCATGAGCCTGGGCGCCGAGAACAGCGTGGCC
TACAGCAACAACAGCATCGCCATCCCCACCAACTTCACCATCAGCGTGACCACCGAAAT
TCTGCCCGTGAGCATGACCAAGACAAGCGTGGACTGCACCATGTACATCTGCGGCGACA
GCACCGAGTGCAGCAACCTGCTCCTGCAGTACGGCAGCTTCTGCACACAGCTGAAGAG
AGCCCTGACCGGCATCGCCGTGGAGCAAGACAAGAACACCCAAGAGGTGTTCGCCCAA
GTGAAGCAGATCTACAAGACCCCCCCCATCAAGTACTTCGGCGGCTTCAACTTCAGCCA
AATCCTGCCCGACCCTAGCAAGCCCTCCAAGAGAAGCCCCATCGAGGACCTGCTGTTCA
ACAAGGTGACCCTGGCCGACGCCGGCTTCATCAAGCAGTACGGCGACTGCCTCGGCGA
CATCGCTGCTAGAGACCTGATCTGCGCTCAGAAGTTCAACGGGCTCACCGTGCTGCCCC
CCCTGCTGACCGACGAGATGATTGCTCAGTACACCTCCGCCCTGCTGGCCGGCACCATC
ACAAGCGGCTGGACCTTTGGGGCTGGCCCCGCCCTGCAGATCCCCTTCCCCATGCAGAT
GGCCTACAGATTCAACGGCATCGGCGTGACACAGAACGTGCTGTACGAGAATCAGAAG
CTGATCGCCAATCAGTTCAACAGCGCCATCGGCAAGATCCAAGACAGCCTGAGCAGCA
CCCCTAGCGCCCTGGGCAAGCTGCAAGACGTGGTGAACCACAACGCCCAAGCCCTGAA
CACCCTGGTGAAGCAGCTGAGCAGCAAGTTCGGCGCCATCAGCAGCGTGCTGAACGAT
ATCCTGAGCAGACTGGACCCCCCCGAGGCCGAGGTGCAGATCGATAGACTGATCACCGG
CAGACTGCAGAGCCTGCAGACCTACGTGACACAGCAGCTGATCAGAGCCGCCGAGATC
AGAGCTAGCGCCAACCTGGCCGCCACCAAGATGAGCGAGTGCGTGCTGGGGCAGAGCA
AGAGAGTGGACTTCTGCGGCAAGGGCTACCACCTGATGAGCTTCCCTCAGAGCGCCCC
CCACGGCGTGGTGTTCCTGCACGTGACCTACGTGCCCGCCCAAGAGAAGAACTTCACA
ACCGCCCCCGCCATCTGCCACGACGGCAAGGCCCACTTCCCTAGAGAGGGCGTGTTCGT
GAGCAACGGCACCCACTGGTTCGTGACACAGAGAAACTTCTACGAGCCTCAGATCATC
ACCACCGACAACACCTTCGTGAGCGGCAACTGCGACGTGGTGATCGGGATTGTCAACA
ACACCGTGTACGACCCCCTGCAACCTGAGCTGGACAGCTTCAAGGAGGAGCTGGACAA
GTACTTCAAGAACCACACAAGCCCCGACGTGGACCTGGGCGACATTTCCGGCATCAAC
 
GCTAGCGTGGTGAACATTCAGAAGGAGATCGACAGACTGAATGAGGTGGCCAAGAACC
TGAACGAGAGCCTGATCGACCTGCAAGAGCTGGGCAAGTACGAGCAGTACATCAAGTG
GCCCTGGTACATCTGGCTGGGCTTCATCGCCGGCCTGATCGCCATCGTGATGGTGACCAT
CATGCTGTGCTGCATGACAAGCTGCTGTAGCTGCCTGAAGGGCTGCTGCAGCTGCGGCT
CCTGCTGCAAGTTCGACGAGGACGACAGCGAGCCCGTGCTGAAGGGCGTGAAGCTGCA
CTACACCTGA
**SEQ ID NO.49,** Nucleotide sequence encoding sequence based on optimized and designed full-length S protein of Omicron_XBB.2 (including native signal peptide, serving as nucleotide sequence of S antigen for Ad-S-XBB.2 vaccine) and nucleotide sequence encoding SEQ ID NO.31 
ATGTTCGTGTTCCTGGTGCTGCTGCCCCTGGTGAGCAGCCAATGCGTGAACCTGATCAC
AAGAACACAGAGCTACACCAACAGCTTCACAAGAGGCGTGTACTACCCCGACAAGGTG
TTCAGAAGCAGCGTGCTGCATTCCACCCAAGACCTGTTCCTGCCCTTTTTCAGCAACGT
GACCTGGTTCAGAGCCATCCACGTGAGCGGCACCAACGGCACCAAGAGATTCGACAAC
CCCGCCCTGCCCTTCAACGACGGCGTGTACTTCGCTAGCACCGAGAAGAGCAACATCAT
CAGAGGCTGGATCTTCGGCACCACCCTGGACAGCAAGACACAGAGCCTGCTGATCGTG
AACAACGCCACCAACGTGGTGATCAAGGTGTGCGAGTTTCAGTTCTGCAACGACCCCTT
CCTGGACGTGTATCAGAAGAACAACAAGAGCTGGATGGAGAGCGAGTTCAGAGTGTAC
AGCAGCGCCAACAACTGCACCTTCGAGTACGTGAGCCAACCCTTCCTGATGGACCTGG
AGGGCAAGGAGGGCAACTTCAAGAACCTCAGAGAGTTCGTGTTCAAGAACATCGACGG
CTACTTCAAGATCTACAGCAAGCACACCCCCATCAACCTGGAGAGAGACCTGCCCCAAG
GCTTCAGCGCCCTGGAGCCCCTGGTGGACCTGCCCATCGGCATCAACATCACAAGATTT
CAGACCCTGCTGGCTCTGCACCGGAGCTACCTGACCCCTGGCGGCAGCAGCAGCGGGT
GGACCGCCGGCGCCGCTGCCTACTACGTGGGCTACCTGCAGCCTAGAACCTTCCTGCTG
AAGTACAACGAGAACGGCACCATTACCGATGCCGTGGACTGTGCCCTGGACCCCCTGA
GCGAGACCAAGTGCACCCTGAAGAGCTTCACCGTGGAGAAGGGCATCTATCAGACAAG
CAACTTCAGAGTGCAGCCCACCGAGAGCATCGTGAGATTCCCCAACATCACCAACCTGT
GCCCCTTCCACGAGGTGTTCAACGCCACCACCTTCGCTAGCGTGTACGCCTGGAACCGG
AAGAGAATCAGCAACTGCGTGGCCGACTACAGCGTGATCTACAACTTCGCCCCCTTCTT
CGCCTTCAAGTGTTACGGGGTGAGCCCCACCAAGCTGAACGACCTGTGCTTCACCAAC
GTGTACGCCGACAGCTTCGTGATCAGAGGCAACGAGGTGAGCCAAATCGCCCCTGGGC
AGACCGGCAACATCGCCGACTACAACTACAAGCTGCCCGACGACTTCACCGGCTGCGT
GATCGCCTGGAACTCCAACAAGCTCGACAGCAAGCCTAGCGGCAACTACAACTACCTGT
ACAGACTGTTCAGAAAGAGCAAGCTGAAGCCCTTCGAGAGAGACATCAGCACCGAGAT
CTACCAAGCCGGCAACAAGCCCTGCAACGGCGTGGCCGGCAGCAACTGCTACAGCCCC
 
CTGCAGAGCTACGGCTTCAGACCCACCTACGGCGTGGGCCATCAGCCCTACAGAGTGGT
CGTGCTGAGCTTCGAGCTGCTGCACGCCCCCGCCACCGTGTGCGGCCCCAAGAAGAGC
ACCAACCTGGTGAAGAACAAGTGCGTGAACTTCAACTTTAACGGCCTGACCGGCACCG
GCGTGCTGACCGAGAGCAACAAGAAGTTCCTGCCTTTCCAACAGTTCGGCAGAGACAT
CGCCGACACCACCGACGCCGTGAGAGACCCTCAGACCCTGGAGATCCTGGACATCACC
CCTTGCAGCTTCGGCGGCGTGAGCGTGATCACCCCCGGCACCAACACAAGCAACCAAG
TGGCCGTGCTGTACCAAGGCGTGAACTGCACCGAGGTGCCCGTGGCCATCCACGCCGAT
CAGCTGACCCCCACCTGGCGGGTGTACAGCACCGGGAGCAACGTGTTTCAGACAAGAG
CCGGCTGCCTGATCGGCGCCGAGTACGTGAACAACAGCTACGAGTGCGACATCCCCATC
GGCGCCGGCATCTGCGCTAGCTATCAGACACAGACCAAGAGCCACGGCAGCGCTAGCA
GCGTGGCTAGCCAAAGCATCATCGCCTACACCATGAGCCTGGGCGCCGAGAACAGCGT
GGCCTACAGCAACAACAGCATCGCCATCCCCACCAACTTCACCATCAGCGTGACCACCG
AGATCCTGCCCGTGAGCATGACCAAGACAAGCGTGGACTGCACCATGTACATCTGCGGC
GACAGCACCGAGTGCAGCAACCTGCTCCTGCAGTACGGCAGCTTCTGCACACAGCTGA
AGAGAGCCCTGACCGGCATCGCCGTGGAGCAAGACAAGAACACCCAAGAGGTGTTCG
CCCAAGTGAAGCAGATCTACAAGACCCCCCCCATCAAGTACTTCGGCGGCTTCAACTTC
AGCCAAATCCTCCCCGACCCTAGCAAGCCCTCCAAGAGAAGCCCCATCGAGGACCTGCT
GTTCAACAAGGTGACCCTGGCCGACGCCGGCTTCATCAAGCAGTACGGCGACTGCCTG
GGCGATATCGCCGCTAGAGACCTGATCTGCGCTCAGAAGTTCAACGGCCTGACCGTCCT
GCCCCCCCTGCTGACCGACGAGATGATCGCCCAATATACATCCGCCCTCCTGGCCGGCAC
CATCACATCCGGGTGGACCTTTGGGGCCGGGCCTGCCCTGCAGATCCCCTTCCCCATGC
AGATGGCCTACAGATTCAACGGCATCGGCGTGACACAGAACGTGCTGTACGAGAATCAG
AAGCTGATCGCCAATCAGTTCAACAGCGCCATCGGCAAGATCCAAGACAGCCTGAGCA
GCACCCCTAGCGCCCTGGGCAAGCTGCAAGACGTGGTGAACCACAACGCCCAAGCCCT
GAACACCCTGGTGAAGCAGCTGAGCAGCAAGTTCGGCGCCATCAGCTCCGTGCTGAAC
GACATCCTGAGCAGACTGGACCCCCCCGAGGCCGAGGTGCAAATCGACAGACTGATCA
CCGGCAGACTGCAGAGCCTGCAGACCTACGTGACACAGCAGCTGATCAGAGCCGCCGA
GATCAGAGCTAGCGCCAACCTGGCCGCCACCAAGATGAGCGAGTGCGTGCTGGGGCAG
AGCAAGAGAGTGGACTTCTGCGGCAAGGGCTACCACCTGATGAGCTTCCCTCAGAGCG
CCCCCCACGGCGTGGTGTTCCTGCACGTGACCTACGTGCCCGCCCAAGAGAAGAACTTC
ACCACAGCCCCCGCCATCTGCCACGACGGCAAGGCCCACTTCCCTAGAGAGGGCGTGT
TCGTGAGCAACGGCACCCACTGGTTCGTGACACAGAGAAACTTCTACGAGCCTCAGAT
CATCACCACCGACAACACCTTCGTGAGCGGCAACTGCGACGTGGTGATCGGCATCGTGA
ACAATACAGTGTACGACCCCCTGCAGCCCGAGCTGGACAGCTTCAAGGAGGAGCTGGA
CAAGTACTTCAAAAACCACACAAGCCCCGACGTGGACCTGGGCGACATCTCCGGCATC
 
AACGCTAGCGTGGTGAACATTCAGAAGGAGATCGATAGACTGAACGAGGTGGCCAAGA
ACCTGAACGAGAGCCTGATCGACCTGCAAGAGCTGGGCAAGTACGAGCAGTACATCAA
GTGGCCCTGGTACATCTGGCTGGGCTTCATCGCCGGCCTGATCGCCATCGTGATGGTGAC
CATCATGCTGTGCTGCATGACAAGCTGCTGCAGCTGCCTGAAAGGCTGCTGTAGCTGCG
GCAGCTGCTGCAAGTTCGACGAGGACGACAGCGAGCCCGTGCTGAAGGGCGTGAAGC
TGCACTACACCTGA
**SEQ ID NO.50,** Nucleotide sequence encoding sequence based on optimized and designed full-length S protein of Omicron_XBB.1.5 (including native signal peptide, serving as nucleotide sequence of S antigen for Ad-S-XBB.1.5 vaccine) and nucleotide sequence encoding SEQ ID NO.32 
ATGTTCGTGTTCCTGGTGCTGCTGCCCCTGGTGAGCAGCCAATGCGTGAACCTGATCAC
AAGAACACAGAGCTACACCAACAGCTTCACAAGAGGCGTGTACTACCCCGACAAGGTG
TTCAGAAGCAGCGTGCTGCATTCCACCCAAGACCTGTTCCTCCCCTTCTTTTCCAACGTG
ACCTGGTTCCACGCCATCCACGTGAGCGGCACCAACGGCACCAAGAGATTCGACAACC
CCGCCCTGCCCTTCAACGACGGCGTGTACTTCGCTAGCACCGAGAAGAGCAACATCATC
AGAGGCTGGATCTTCGGCACCACCCTGGACAGCAAGACACAGAGCCTGCTGATCGTGA
ACAATGCCACCAACGTGGTGATCAAGGTGTGCGAGTTTCAGTTCTGCAACGACCCCTTC
CTGGACGTGTATCAGAAGAACAACAAGAGCTGGATGGAGAGCGAGTTCAGAGTGTACA
GCAGCGCTAACAACTGCACCTTCGAGTACGTGAGCCAACCCTTCCTGATGGACCTGGAG
GGCAAGGAGGGCAACTTCAAGAACCTCAGAGAGTTCGTGTTCAAGAACATCGACGGCT
ACTTCAAGATCTACAGCAAGCACACCCCCATCAACCTGGAGAGAGACCTGCCCCAAGG
CTTCAGCGCCCTGGAGCCCCTGGTGGACCTGCCCATCGGCATCAACATCACAAGATTTC
AGACCCTGCTGGCCCTGCACAGAAGCTATCTGACACCTGTGGACAGCAGCAGCGGCTG
GACAGCTGGGGCCGCTGCCTACTACGTGGGCTACCTGCAGCCTAGAACCTTCCTGCTGA
AGTACAACGAGAACGGCACAATCACCGATGCCGTGGATTGCGCCCTGGACCCCCTGAG
CGAGACCAAGTGCACCCTGAAGAGCTTCACCGTGGAGAAGGGCATCTATCAGACAAGC
AACTTCAGAGTGCAGCCCACCGAGAGCATCGTGAGATTCCCCAACATCACCAACCTGTG
CCCCTTCCACGAGGTGTTCAACGCCACCACCTTCGCTAGCGTGTACGCTTGGAACAGAA
AAAGAATCAGCAACTGCGTGGCCGACTACAGCGTGATCTACAACTTCGCCCCCTTCTTC
GCCTTCAAGTGCTACGGGGTGAGCCCCACCAAGCTGAACGACCTGTGCTTCACCAACG
TGTACGCCGACAGCTTCGTGATCAGAGGCAACGAGGTGAGCCAAATCGCCCCTGGGCA
GACCGGCAACATCGCCGACTACAACTACAAGCTGCCCGACGACTTCACCGGCTGCGTGA
TCGCCTGGAACAGCAACAAGCTGGACAGCAAACCTAGCGGCAACTACAACTACCTGTA
CAGACTGTTCAGAAAGAGCAAGCTGAAGCCCTTCGAGAGAGACATCAGCACCGAGATC
 
TACCAAGCCGGCAACAAGCCCTGCAACGGCGTGGCCGGCCCCAACTGCTACAGCCCCC
TGCAGAGCTACGGCTTCAGACCCACCTACGGCGTGGGCCATCAGCCCTACAGAGTGGTC
GTGCTGAGCTTCGAGCTGCTGCACGCCCCCGCCACCGTGTGCGGCCCCAAGAAGAGCA
CCAACCTGGTGAAGAACAAGTGCGTGAACTTCAACTTCAACGGCCTCACCGGCACCGG
CGTGCTGACCGAGAGCAACAAGAAGTTCCTGCCCTTTCAGCAGTTCGGCAGAGACATC
GCCGACACCACCGACGCCGTGAGAGACCCTCAGACCCTGGAGATCCTGGACATCACCC
CCTGTAGCTTCGGCGGCGTGAGCGTGATCACCCCCGGCACCAACACAAGCAACCAAGT
GGCCGTGCTGTACCAAGGCGTGAACTGCACCGAGGTGCCCGTGGCCATCCACGCCGATC
AGCTGACCCCCACCTGGAGAGTCTACTCCACCGGCAGCAACGTGTTTCAGACAAGAGC
CGGCTGCCTGATCGGCGCCGAGTACGTGAACAACAGCTACGAGTGCGACATCCCCATCG
GCGCCGGCATCTGCGCTAGCTATCAGACACAGACCAAGAGCCACGGCAGCGCTAGCAG
CGTGGCTAGCCAAAGCATCATCGCCTACACCATGAGCCTGGGCGCCGAGAACAGCGTG
GCCTACAGCAACAACAGCATCGCCATCCCCACCAACTTCACCATCAGCGTGACCACCGA
GATCCTGCCCGTCAGCATGACCAAGACAAGCGTGGACTGCACCATGTACATCTGCGGCG
ACAGCACCGAGTGCAGCAACCTGCTCCTGCAGTACGGCAGCTTCTGCACACAGCTGAA
GAGAGCCCTGACCGGCATCGCCGTGGAGCAAGACAAGAACACCCAAGAGGTGTTCGCC
CAAGTGAAGCAGATCTACAAGACCCCCCCCATCAAGTACTTCGGCGGCTTCAACTTCAG
CCAAATCCTCCCCGACCCCTCCAAACCTAGCAAGAGAAGCTTCATCGAGGACCTGCTGT
TCAACAAGGTGACCCTGGCCGACGCCGGCTTCATCAAGCAGTACGGCGACTGCCTGGG
CGATATCGCCGCTAGAGACCTGATCTGCGCTCAGAAGTTCAATGGCCTGACCGTGCTGC
CCCCCCTGCTGACCGACGAGATGATCGCTCAGTATACAAGCGCTCTGCTCGCTGGCACC
ATTACAAGCGGGTGGACCTTCGGCGCTGGCGCTGCCCTGCAGATCCCCTTCGCCATGCA
GATGGCCTACAGATTCAACGGCATCGGCGTGACACAGAACGTGCTGTACGAGAATCAGA
AGCTGATCGCCAATCAGTTCAACAGCGCCATCGGCAAGATCCAAGACAGCCTGAGCAG
CACCGCTAGCGCCCTGGGCAAGCTGCAAGACGTGGTGAACCACAACGCCCAAGCCCTG
AACACCCTGGTGAAGCAGCTGAGCAGCAAGTTCGGCGCCATCAGCAGCGTGCTCAACG
ACATCCTGAGCAGACTGGACCCCCCCGAGGCCGAGGTGCAGATCGATAGACTGATCACC
GGCAGACTGCAGAGCCTGCAGACCTACGTGACACAGCAGCTGATCAGAGCCGCCGAGA
TCAGAGCTAGCGCCAACCTGGCCGCCACCAAGATGAGCGAGTGCGTGCTGGGGCAGAG
CAAGAGAGTGGACTTCTGCGGCAAGGGCTACCACCTGATGAGCTTCCCTCAGAGCGCC
CCCCACGGCGTGGTGTTCCTGCACGTGACCTACGTGCCCGCCCAAGAGAAGAACTTCA
CCACAGCCCCCGCCATCTGCCACGACGGCAAGGCCCACTTCCCTAGAGAGGGCGTGTTC
GTGAGCAACGGCACCCACTGGTTCGTGACACAGAGAAACTTCTACGAGCCTCAGATCAT
CACCACCGACAACACCTTCGTGAGCGGCAACTGCGACGTGGTGATCGGCATCGTGAATA
ATACCGTGTACGACCCCCTGCAGCCCGAGCTGGACAGCTTCAAGGAGGAGCTGGACAA
 
GTATTTCAAGAACCATACAAGCCCCGACGTGGACCTGGGCGACATCAGCGGCATCAACG
CTAGCGTGGTGAACATTCAGAAGGAAATCGACAGACTCAACGAGGTGGCCAAGAACCT
GAACGAGAGCCTGATCGACCTGCAAGAGCTGGGCAAGTACGAGCAGTACATCAAGTGG
CCCTGGTACATCTGGCTGGGCTTCATCGCCGGCCTGATCGCCATCGTGATGGTGACCATC
ATGCTGTGCTGCATGACAAGCTGCTGCAGCTGCCTGAAGGGCTGTTGCAGCTGCGGCAG
CTGCTGCAAGTTCGACGAGGACGACAGCGAGCCCGTGCTGAAGGGCGTGAAGCTGCAC
TACACCTGA
**SEQ ID NO.51,** Nucleotide sequence encoding sequence based on optimized and designed full-length S protein of Omicron_XBB.2.3 (including native signal peptide, serving as nucleotide sequence of S antigen for Ad-S-XBB.2.3 vaccine) and nucleotide sequence encoding SEQ ID NO.33 
ATGTTCGTGTTCCTGGTGCTGCTGCCCCTGGTGAGCAGCCAATGCGTGAACCTGATCAC
AAGAACACAGAGCTACACCAACAGCTTCACAAGAGGCGTGTACTACCCCGACAAGGTG
TTCAGAAGCAGCGTGCTGCACTCCACCCAAGACCTGTTTCTGCCCTTTTTCAGCAACGT
GACCTGGTTCCACGCCATCCACGTGAGCGGCACCAACGGCACCAAGAGATTCGACAAC
CCCGCCCTGCCCTTCAACGACGGCGTGTACTTCGCTAGCACCGAGAAGAGCAACATCAT
CAGAGGCTGGATCTTCGGCACCACCCTGGACAGCAAAACACAGAGCCTGCTGATCGTG
AACAATGCCACCAACGTGGTGATCAAGGTGTGCGAGTTTCAGTTCTGCAACGACCCCTT
CCTGGACGTGTATCAGAAGAACAACAAGAGCTGGATGGAGAGCGAGTTCAGAGTGTAC
AGCTCCGCCAACAACTGCACCTTCGAGTACGTGAGCCAACCCTTCCTGATGGACCTGGA
GGGCAAGGAGGGCAACTTCAAGAACCTCCGGGAGTTCGTGTTCAAGAACATCGACGGC
TACTTCAAGATCTACAGCAAGCACACCCCCATCAACCTGGAGAGAGACCTGCCCCAAG
GCTTCAGCGCCCTGGAGCCCCTGGTGGACCTGCCCATCGGCATCAACATCACAAGATTT
CAGACCCTGCTGGCTCTGCACAGAAGCTATCTCACCCCCGGCGGGAGCAGCAGCGGCT
GGACCGCCGGGGCTGCTGCCTACTACGTGGGCTACCTGCAGCCTAGAACCTTCCTGCTG
AAGTACAACGAGAACGGCACCATTACCGATGCCGTGGACTGTGCCCTGGACCCCCTGA
GCGAGACCAAGTGCACCCTGAAGAGCTTCACCGTGGAGAAGGGCATCTATCAGACAAG
CAACTTCAGAGTGCAGCCCACCGAGAGCATCGTGAGATTCCCCAACATCACCAACCTGT
GCCCCTTCCACGAGGTGTTCAACGCCACCACCTTCGCTAGCGTGTACGCCTGGAACCGG
AAGAGAATCAGCAACTGCGTGGCCGACTACAGCGTGATCTACAACTTCGCCCCCTTCTT
CGCCTTCAAATGCTACGGCGTGAGCCCCACCAAGCTGAACGACCTGTGCTTCACCAACG
TGTACGCCGACAGCTTCGTGATCAGAGGCAACGAGGTGAGCCAAATCGCCCCTGGGCA
GACCGGCAACATCGCCGACTACAACTACAAGCTGCCCGACGACTTCACCGGCTGCGTGA
TCGCCTGGAACTCCAACAAGCTGGACAGCAAGCCTAGCGGCAACTACAACTACCTGTA
 
CAGACTGTTCAGAAAGAGCAAGCTGAAGCCCTTCGAGAGAGACATCAGCACCGAGATC
TACCAAGCCGGCAACAAGCCCTGCAACGGCGTGGCCGGCCCCAACTGCTACAGCCCCC
TGCAGAGCTACGGCTTCAGACCCACCTACGGCGTGGGCCATCAGCCCTACAGAGTGGTC
GTGCTGAGCTTCGAGCTGCTGCACGCTAGCGCCACCGTGTGCGGCCCCAAGAAGAGCA
CCAACCTGGTGAAGAACAAGTGCGTGAACTTCAACTTCAACGGCCTGACCGGCACCGG
CGTGCTGACCGAGAGCAACAAGAAGTTCCTGCCCTTTCAGCAGTTCGGCAGAGACATC
GCCGACACCACCGACGCCGTGAGAGACCCTCAGACCCTGGAGATCCTGGACATCACCC
CTTGCAGCTTCGGGGGCGTGAGCGTGATCACCCCCGGCACCAACACAAGCAACCAAGT
GGCCGTGCTGTACCAAGGCGTGAACTGCACCGAGGTGCCCGTGGCCATCCACGCCGATC
AGCTGACCCCCACCTGGCGGGTGTATAGCACCGGCAGCAACGTGTTTCAGACAAGAGC
CGGCTGCCTGATCGGCGCCGAGTACGTGAACAACAGCTACGAGTGCGACATCCCCATCG
GCGCCGGCATCTGCGCTAGCTATCAGACACAGACCAAGAGCCACGGCAGCGCTAGCAG
CGTGGCTAGCCAAAGCATCATCGCCTACACCATGAGCCTGGGCGCCGAGAACAGCGTG
GCCTACAGCAACAACAGCATCGCCATCCCCACCAACTTCACCATCAGCGTGACCACCGA
AATCCTGCCCGTGTCCATGACCAAGACAAGCGTGGACTGCACCATGTACATCTGCGGCG
ACAGCACCGAGTGCAGCAACCTGCTCCTGCAGTACGGCAGCTTCTGCACACAGCTGAA
GAGAGCCCTGACCGGCATCGCCGTGGAGCAAGACAAGAACACCCAAGAGGTGTTCGCC
CAAGTGAAGCAGATCTACAAGACCCCCCCCATCAAGTACTTCGGCGGCTTCAACTTCAG
CCAAATCCTGCCCGACCCTAGCAAGCCTAGCAAGAGAAGCCCCATCGAGGACCTGCTGT
TCAACAAGGTGACCCTGGCCGACGCCGGCTTCATCAAGCAGTACGGCGACTGCCTCGG
CGACATTGCTGCTAGAGACCTGATCTGCGCTCAGAAGTTCAACGGCCTCACAGTCCTCC
CCCCCCTGCTGACCGACGAGATGATTGCTCAGTACACAAGCGCCCTGCTGGCCGGCACC
ATTACAAGCGGGTGGACCTTTGGCGCTGGCCCTGCCCTGCAGATCCCCTTCCCCATGCA
GATGGCCTACAGATTCAACGGCATCGGCGTGACACAGAACGTGCTGTACGAGAATCAGA
AGCTGATCGCCAATCAGTTCAACAGCGCCATCGGCAAGATCCAAGACAGCCTGAGCAG
CACCCCTAGCGCCCTGGGCAAGCTGCAAGACGTGGTGAACCACAACGCCCAAGCCCTG
AACACCCTGGTGAAGCAGCTGAGCAGCAAGTTCGGCGCCATCTCCTCCGTGCTGAATG
ACATCCTGAGCAGACTGGACCCCCCCGAGGCCGAGGTGCAGATTGACAGACTGATCAC
CGGCAGACTGCAGAGCCTGCAGACCTACGTGACACAGCAGCTGATCAGAGCCGCCGAG
ATCAGAGCTAGCGCCAACCTGGCCGCCACCAAGATGAGCGAGTGCGTGCTGGGGCAGA
GCAAGAGAGTGGACTTCTGCGGCAAGGGCTACCACCTGATGAGCTTCCCTCAGAGCGC
CCCCCACGGCGTGGTGTTCCTGCACGTGACCTACGTGCCCGCCCAAGAGAAGAACTTC
ACCACCGCCCCCGCCATCTGCCACGACGGCAAGGCCCACTTCCCTAGAGAGGGCGTGTT
CGTGAGCAACGGCACCCACTGGTTCGTGACACAGAGAAACTTCTACGAGCCTCAGATC
ATCACCACCGACAACACCTTCGTGAGCGGCAACTGCGACGTGGTGATCGGCATCGTGA
 
ACAACACAGTGTACGACCCCCTGCAGCCCGAGCTGGACAGCTTCAAGGAGGAGCTGGA
CAAGTACTTTAAAAACCACACAAGCCCCGACGTGGACCTCGGCGACATCTCCGGCATCA
ACGCTAGCGTGGTGAACATTCAGAAGGAGATCGATAGACTGAACGAGGTGGCCAAGAA
CCTGAACGAGAGCCTGATCGACCTGCAAGAGCTGGGCAAGTACGAGCAGTACATCAAG
TGGCCCTGGTACATCTGGCTGGGCTTCATCGCCGGCCTGATCGCCATCGTGATGGTGACC
ATCATGCTGTGCTGCATGACAAGCTGCTGCTCCTGCCTGAAGGGGTGCTGCAGCTGCGG
CAGCTGCTGCAAGTTCGACGAGGACGACAGCGAGCCCGTGCTGAAGGGCGTGAAGCT
GCACTACACCTGA
**SEQ ID NO.52,** Nucleotide sequence encoding sequence based on optimized and designed full-length S protein of Omicron_XBB.1.6, including native signal peptide, serving as nucleotide sequence of S antigen for Ad-S-XBB.1.6 vaccine
**SEQ ID NO.53,** Nucleotide sequence encoding sequence based on optimized and designed full-length S protein of Omicron_XBB.1.9.1, including native signal peptide, serving as nucleotide sequence of S antigen for Ad-S-XBB.1.9.1 vaccine
**SEQ ID NO.54,** Nucleotide sequence encoding sequence based on optimized and designed full-length S protein of Omicron_EG. 5, including native signal peptide, serving as nucleotide sequence of S antigen for Ad-S-EG5 vaccine
**SEQ ID NO.55,** RBD-HR sequence of optimized S protein of JN.1 
VQPTESIVRFPNVTNLCPFHEVFNATRFASVYAWNRTRISNCVADYSVLYNFAPFFAFKCYG
VSPTKLNDLCFTNVYADSFVIKGNEVSQIAPGQTGNIADYNYKLPDDFTGCVIAWNSNKLD
SKHSGNYDYWYRSFRKSKLKPFERDISTEIYQAGNKPCKGKGPNCYFPLQSYGFRPTYGVG
HQPYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRLN
EVAKNLNESLIDLQEL
**SEQ ID NO.56,** Sequence based on optimized and designed S protein-RBD-HR of Omicron JN.1, including: signal peptide-Trx tag-6His tag-EK digestion site-RBD sequence-HR1 sequence-HR2 sequence 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNVT
NLCPFHEVFNATRFASVYAWNRTRISNCVADYSVLYNFAPFFAFKCYGVSPTKLNDLCFTNV
YADSFVIKGNEVSQIAPGQTGNIADYNYKLPDDFTGCVIAWNSNKLDSKHSGNYDYWYRS
FRKSKLKPFERDISTEIYQAGNKPCKGKGPNCYFPLQSYGFRPTYGVGHQPYRVVVLSFELL
 
HAPATVCGPKKSTNLVKNKSVNFNFNGLYENQKLIANQFNSAIGKIQDSLSSTASALGKLQD
VVNQNAQALNTLVKQLKNHTSPDVDLGDISGINASVVNIQKEIDRLNEVAKNLNESLIDLQ
EL
**SEQ ID NO.57**, Nucleotide sequence encoding sequence based on optimized and designed S protein-RBD-HR of Omicron_JN.1 and nucleotide sequence encoding SEQ ID NO.56

ATGCTGCTGGTGAACCAGAGCCACCAGGGTTTCAACAAGGAACACACTAGCAAGATGG
TGTCTGCTATCGTCCTCTACGTGCTGCTCGCCGCTGCTCACTCAGCTTTCGCCGCTG
ACTCTATCCACATCAAGGACTCCGACGACCTCAAGAACCGTTTGGCCGAAGCCGGCGAC
AAGCTGGTCGTTATCGACTTCATGGCTACCTGGTGTGGCCCTTGCAAGATGATCGGACCT
AAGCTGGACGAAATGGCTAACGAGATGAGCGACAGCATCGTCGTTCTGAAGGTGGACG
TCGACGAATGTGAGGACATCGCCACTGAGTACAACATCAACAGCATGCCTACCTTCGTT
TTCGTGAAGAACTCTAAGAAGATCGAGGAGTTCTCTGGTGCCAACGTGGACAAGCTCC
GTAACACCATCATCAAGCTCAAGCTGGCCGGCAGCGGTAGCGGTCACATGCACCACCAC
CACCACCATTCAAGCGGCGACGACGACGACAAGGTGCAGCCAACCGAATCAATCGTCC
GCTTCCCAAACGTCACTAACCTGTGCCCATTCCACGAGGTGTTCAACGCCACCCGTTTC
GCCTCTGTCTACGCTTGGAACCGCACGCGCATCTCCAACTGTGTCGCCGACTACAGCGT
GCTGTACAACTTCGCTCCTTTCTTCGCATTCAAGTGTTACGGAGTGTCCCCTACCAAGCT
CAACGATTTGTGCTTCACAAACGTGTACGCTGACAGCTTCGTGATCAAGGGTAACGAGG
TTTCTCAAATCGCCCCTGGTCAGACAGGTAACATCGCTGACTACAACTACAAGCTCCCA
GACGACTTCACTGGCTGTGTTATCGCTTGGAACTCAAACAAGCTGGACTCTAAGCATTC
TGGTAACTACGACTACTGGTACCGTTCGTTCCGCAAGAGCAAGCTCAAGCCATTCGAAC
GTGACATCAGCACAGAGATCTACCAAGCCGGTAACAAGCCCTGCAAGGGCAAGGGACC
TAACTGCTACTTCCCCCTCCAGTCCTACGGATTCCGCCCAACCTACGGCGTGGGTCACCA
GCCATACCGCGTGGTTGTGCTGAGCTTCGAACTGCTGCACGCCCCCGCTACCGTGTGTG
GCCCTAAGAAGAGCACTAACCTCGTTAAGAACAAGTCCGTGAACTTCAACTTCAACGGT
CTCTACGAAAACCAGAAGCTGATCGCTAACCAGTTCAACTCCGCCATCGGCAAGATCCA
GGACTCCCTGTCCTCCACAGCTTCCGCTCTGGGCAAGCTGCAGGACGTTGTGAACCAA
AACGCTCAGGCCCTGAACACACTGGTGAAGCAACTGAAGAACCACACCAGCCCCGAC
GTTGACCTGGGTGACATCAGCGGTATCAACGCTAGCGTGGTGAACATCCAGAAGGAGAT
CGACAGGCTGAACGAAGTGGCTAAGAACTTGAACGAGAGCCTCATCGACCTGCAGGAA
CTGTAA

Beneficial effects: In the present invention, the recombinant protein vaccine and the adenoviral vector vaccine are prepared, and the proteantigen of the recombinant protein vaccine is constructed and expressed based on the RBD sequence of S proteins (aa 320-545) or the HR1 sequence and HR2 sequence of the RBD-HR of SARS-CoV-2 Omicron variants XBB.1.16, XBB.2, XBB.1.5, XBB.2.3, XBB.1.16.6, BA.2.86, EG5, and JN.1. In addition, after adding the corresponding vaccine adjuvant to the prepared proteantigen, it can better help the host resist the cross infection caused by the Omicron XBB variant and subvariants thereof, which is of great significance for the development of recombinant protein vaccines against the SARS-CoV-2 Omicron XBB variant and subvariants thereof. Animal studies have demonstrated that a polyvaccine, formed by combining different recombinant protein antigens or vaccines, exhibits enhanced efficacy against the Omicron XBB variant and its subvariants.

Furthermore, a recombinant adenovirus vector is constructed through codon optimization based on nucleotide sequences encoding the full-length S proteins of XBB.1.16, XBB.2, XBB.1.5, and XBB.2.3 to obtain an adenoviral vector vaccine. The adenoviral vector vaccine is combined with the recombinant protein vaccine to develop a multivalent vaccine with enhanced prophylactic and therapeutic efficacy against SARS-CoV-2 and variants thereof. A nasal spray prepared demonstrates superior protection against infection by SARS-CoV-2 and Omicron subvariants including but not limited to XBB.1.5, XBB.1.6, XBB.1.16, XBB.1.16.6, XBB.2.3, FL.1.5.1, HV.1, EG.5, EG.5.1, BA.2.86, JN.1, JN.1.13, KP.2, and KP.3.

### BRIEF DESCRIPTION OF DRAWINGS

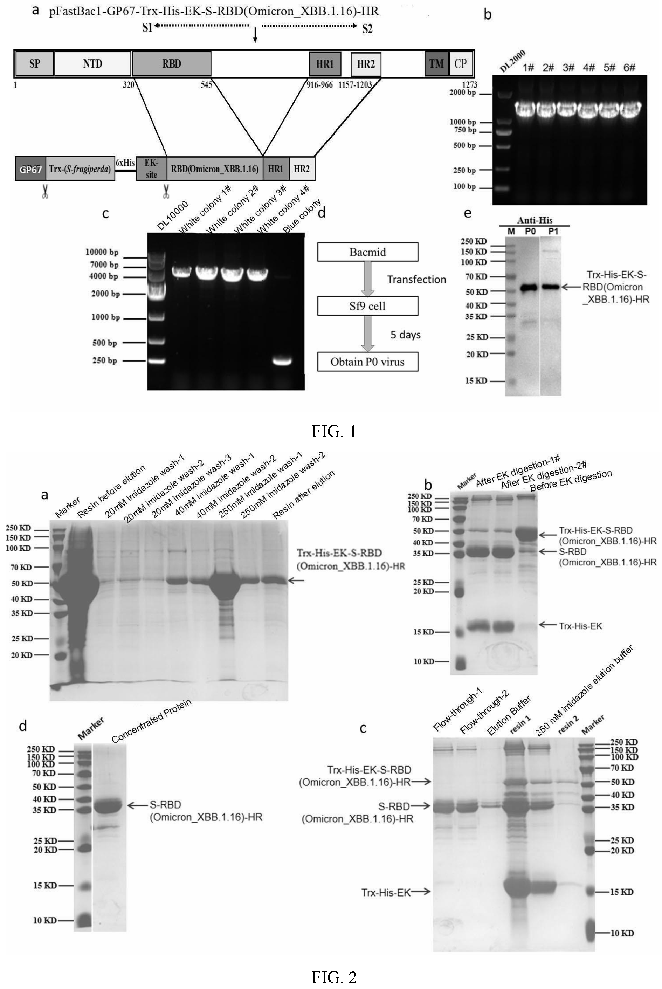

FIG. 1 illustrates the construction and expression of the recombinant protein antigen RBD_{XBB.1.16}-HR of Embodiment 1, in which, a. design sketch of pFastBac1-GP67-Trx-His-EK-S-RBD(Omicron_XBB.1.16)-HR; b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;
FIG. 2 illustrates the expression and purification results of the recombinant protein antigen RBD_{XBB.1.16}-HR in Embodiment 1, in which, a. Elution profile of Ni-affinity chromatography from baculovirus-infected supernatant; b. Verification of EK digestion efficiency on EK-digested sample; and c-d. Purification of the target protein with Ni-affinity chromatography resin after EK digestion(d), and SDS-PAGE analysis(c);

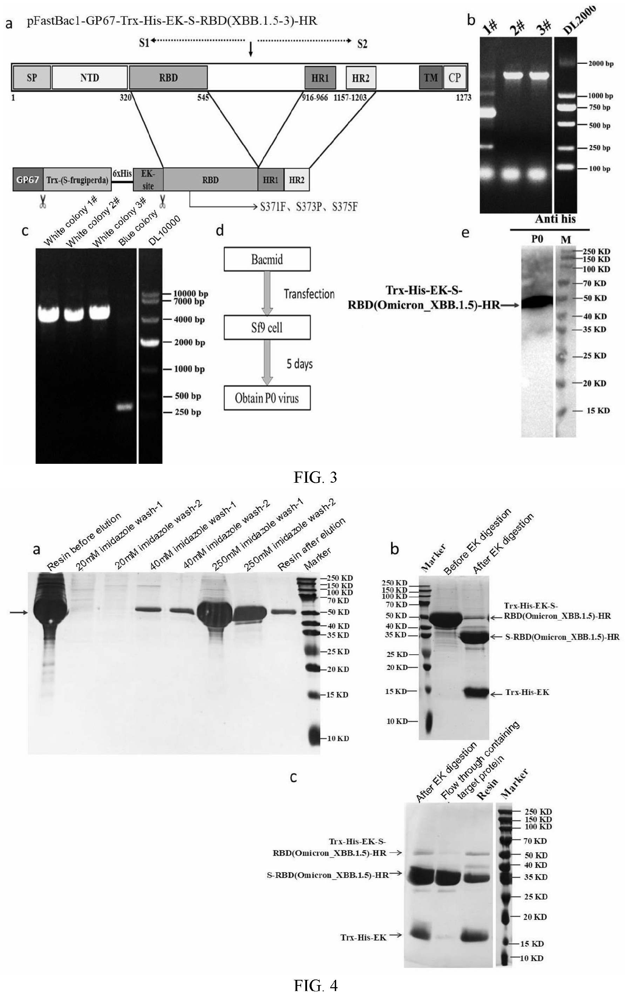

FIG. 3 illustrates the construction and expression of the recombinant protein antigen RBD_{XBB.1.5-3}-HR of Embodiment 1, in which, a. design sketch of pFastBac1-GP67-Trx-His-EK-S-RBD (Omicron_XBB.1.5-3)-HR; b. 1% agarose gel electrophoresis of cloned PCR products; c. 1% agarose gel electrophoresis of PCR verification for blue-white colony liquid; d. Packaging and amplification flowchart of recombinant baculovirus; and e. WB validation results during baculovirus amplification;
FIG. 4 illustrates the expression and purification results of the recombinant protein antigen RBD_{XBB.1.5-3}-HR in Embodiment 1, in which, a. Elution profile of Ni-affinity chromatography from baculovirus-infected supernatant; b. Verification of EK digestion efficiency on EK-digested sample; and c. Purification of the target protein with Ni-affinity chromatography resin after EK digestion;

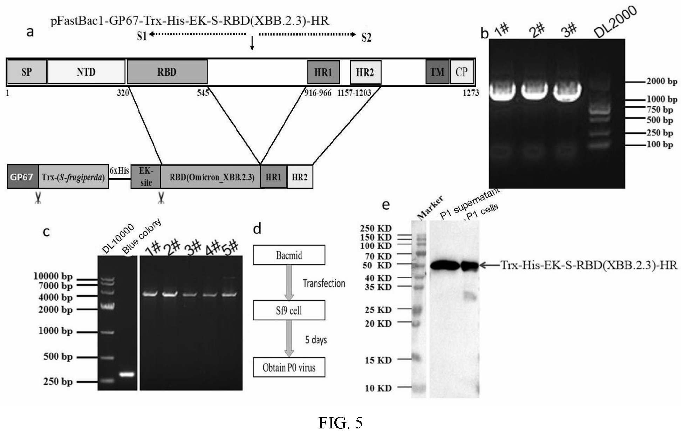

FIG. 5 illustrates the construction and expression of the recombinant protein antigen RBD_{XBB.2.3}-HR of Embodiment 1, in which, a. design sketch of pFastBac1-GP67-Trx-His-EK-S-RBD (Omicron_XBB.2.3)-HR; b. 1% agarose gel electrophoresis of cloned PCR products; c. 1% agarose gel electrophoresis of PCR verification for blue-white colony liquid; d. Packaging and amplification flowchart of recombinant baculovirus; and e. WB validation results during baculovirus amplification;

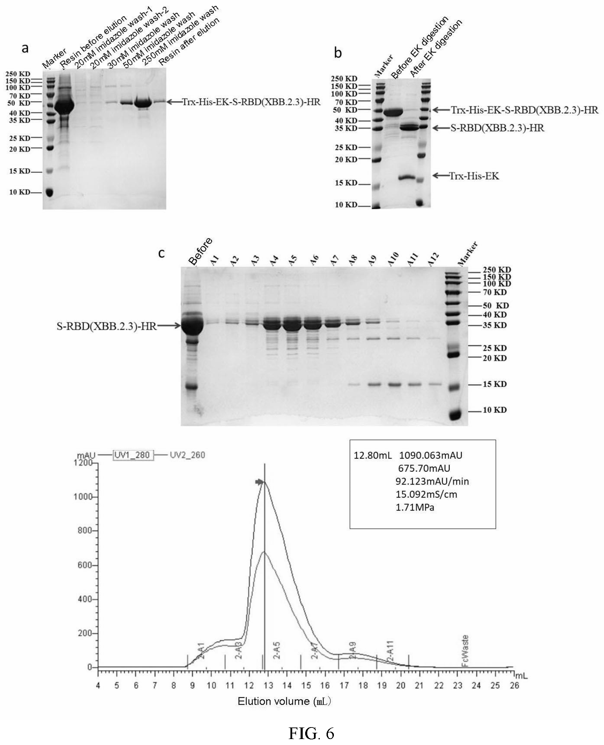

FIG. 6 illustrates the expression and purification results of the recombinant protein antigen RBD_{XBB.2.3}-HR in Embodiment 1, in which, a. Elution profile of Ni-affinity chromatography from baculovirus-infected supernatant; b. Verification of EK digestion efficiency on EK-digested sample; and c. Purification by size-exclusion chromatography after EK digestion and SDS-PAGE analysis;

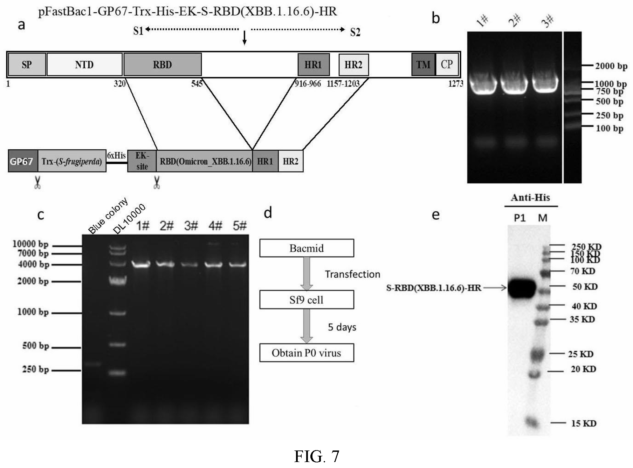

FIG. 7 illustrates the construction and expression of the recombinant protein antigen RBD_{XBB.1.16.6}-HR of Embodiment 1, in which, a. design sketch of pFastBac1-GP67-Trx-His-EK-S-RBD(Omicron_XBB.1.16.6)-HR; b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;

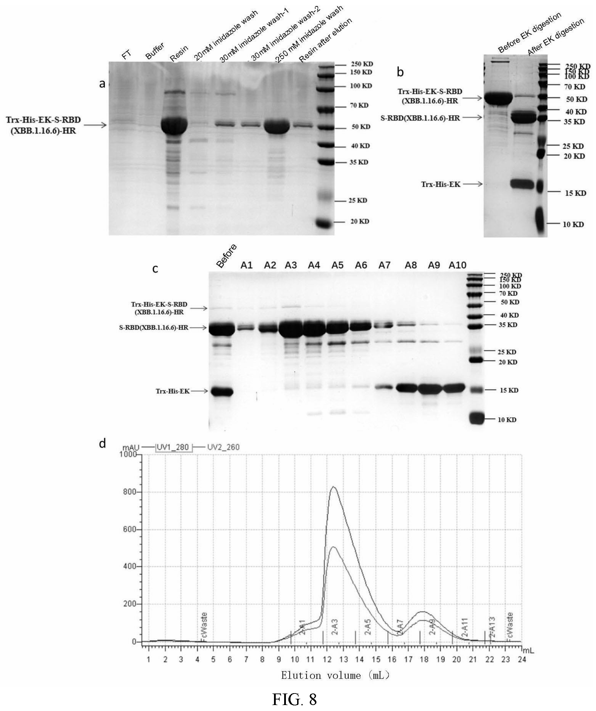

FIG. 8 illustrates the expression and purification results of the recombinant protein antigen RBD_{XBB.1.16.6}-HR in Embodiment 1, in which, a. Elution profile of Ni-affinity chromatography from baculovirus-infected supernatant; b. Verification of EK digestion efficiency on EK-digested sample; and c-d. Purification by size-exclusion chromatography after EK digestion and SDS-PAGE analysis;

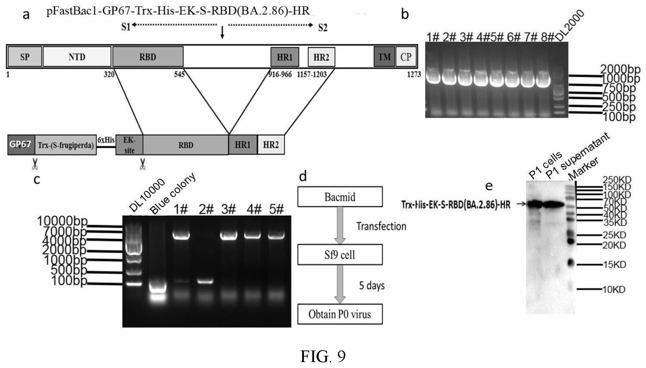

FIG. 9 illustrates the construction and expression of the recombinant protein antigen RBD_{BA.2.86}-HR of Embodiment 1, in which, a. design sketch of pFastBac1-GP67-Trx-His-EK-S-RBD(BA.2.86)-HR; b. 1% agarose gel electrophoresis of cloned PCR products; c. 1% agarose gel electrophoresis of PCR verification for blue-white colony liquid; d. Packaging and amplification flowchart of recombinant baculovirus; and e. WB validation results during baculovirus amplification;

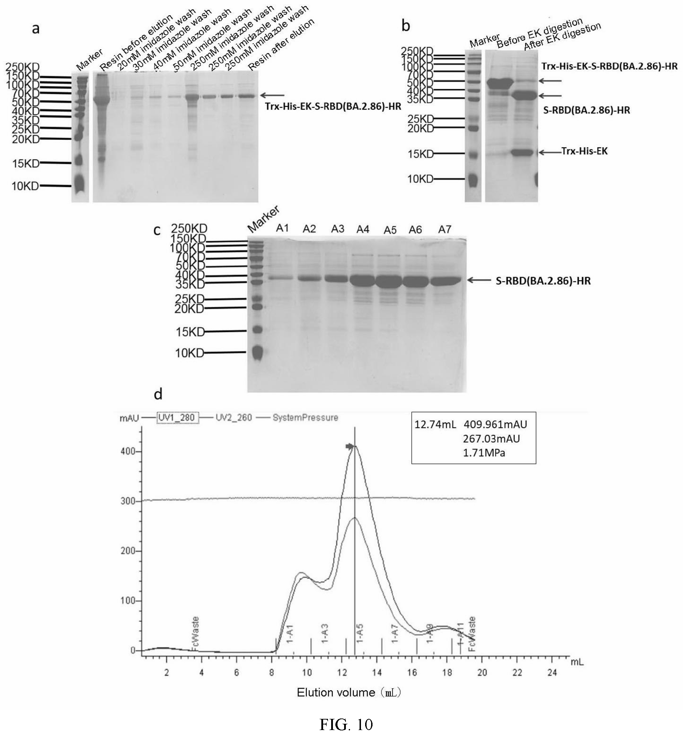

FIG. 10 illustrates the expression and purification results of the recombinant protein antigen RBD_{BA.2.86}-HR in Embodiment 1, in which, a. Elution profile of Ni-affinity chromatography from baculovirus-infected supernatant; b. Verification of EK digestion efficiency on EK-digested sample; and c-d. Purification by size-exclusion chromatography after EK digestion and SDS-PAGE analysis;

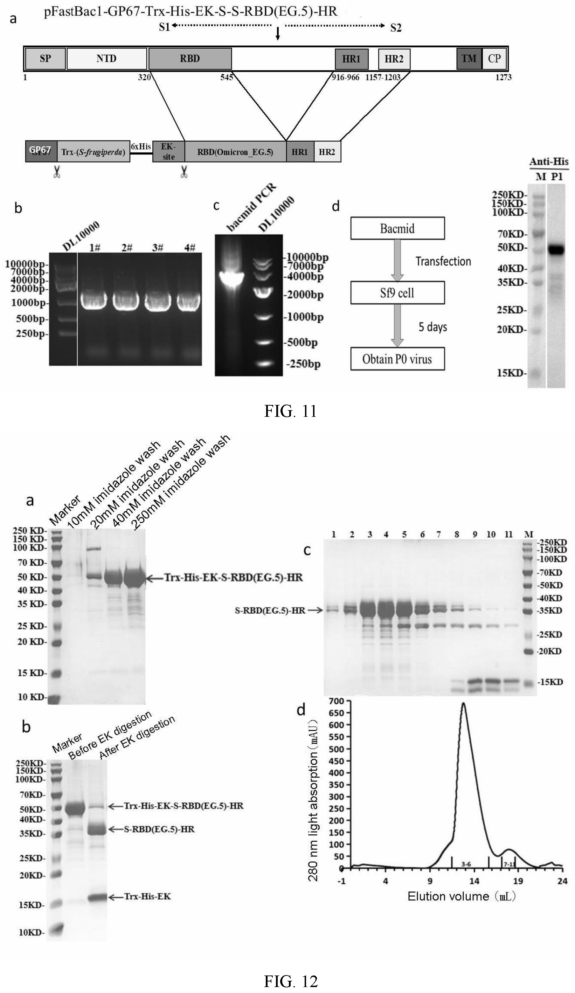

FIG. 11 illustrates the construction and expression of the recombinant protein antigen RBD_{EG.5}-HR of Embodiment 1, in which, a. design sketch of pFastBac1-GP67-Trx-His-EK-S-RBD(EG.5)-HR; b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;
FIG. 12 illustrates the expression and purification results of the recombinant protein antigen RBD_{EG.5}-HR in Embodiment 1, in which, a. Elution profile of Ni-affinity chromatography from baculovirus-infected supernatant; b. Verification of EK digestion efficiency on EK-digested sample; and c-d. Protein Purification by size-exclusion chromatography after EK digestion(d), and SDS-PAGE analysis(c);

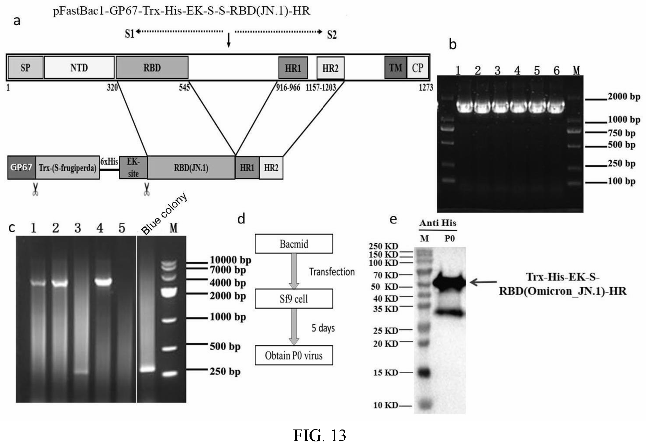

FIG. 13 illustrates the construction and expression of the recombinant protein antigen RBD_{JN.1}-HR of Embodiment 1, in which, a. design sketch of pFastBac1-GP67-Trx-His-EK-S-RBD(JN.1)-HR; b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;

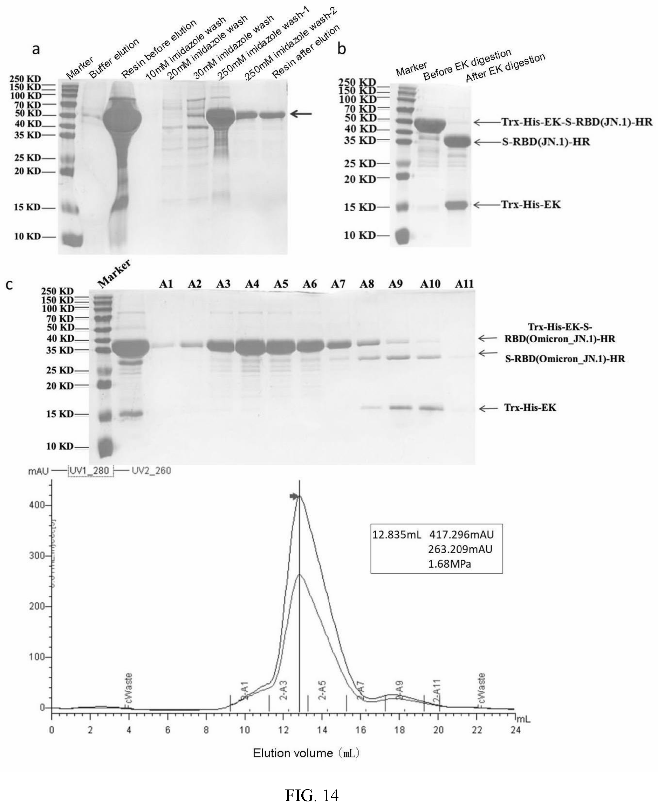

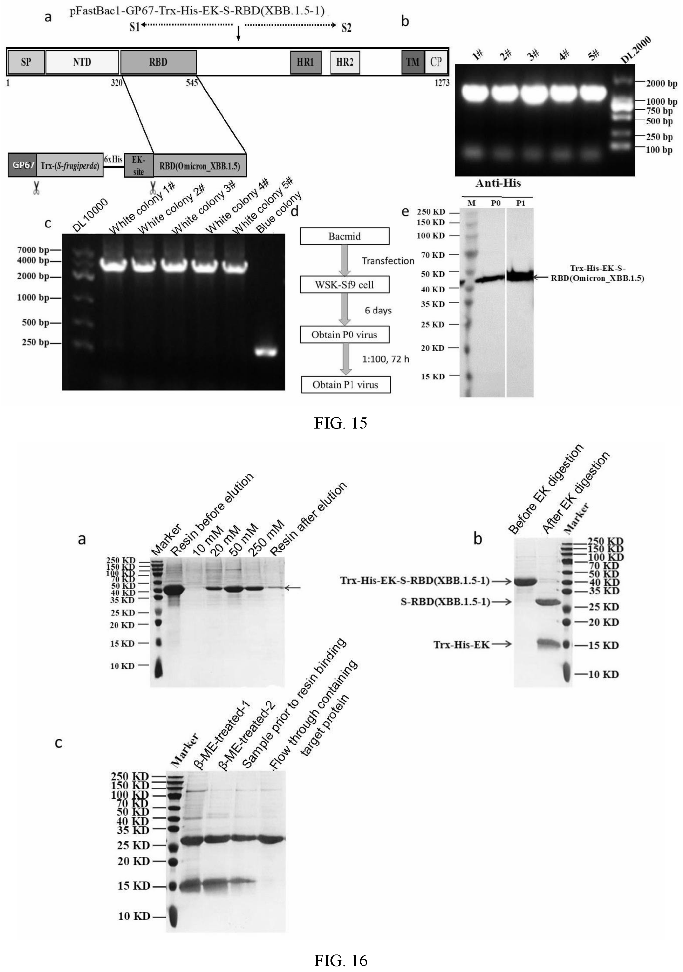

FIG. 14 illustrates the expression and purification results of the recombinant protein antigen RBD_{JN.1}-HR in Embodiment 1, in which, a. Elution profile of Ni-affinity chromatography from baculovirus-infected supernatant; b. Verification of EK digestion efficiency on EK-digested sample; and c. Purification by size-exclusion chromatography after EK digestion and SDS-PAGE analysis; FIG. 15 illustrates the construction and expression of the recombinant protein antigen RBD_{XBB.1.5-1} of Embodiment 1, in which, a. design sketch of pFastBac1-GP67-Trx-His-EK-S-RBD(XBB.1.5-1); b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;
FIG. 16 illustrates the expression and purification results of the recombinant protein antigen RBD_{XBB.1.5-1} in Embodiment 1, in which, a. Elution profile of Ni-affinity chromatography from baculovirus-infected supernatant; b. Verification of EK digestion efficiency on EK-digested sample; and c. Purification of the target protein with Ni-affinity chromatography resin after EK digestion, and SDS-PAGE analysis;

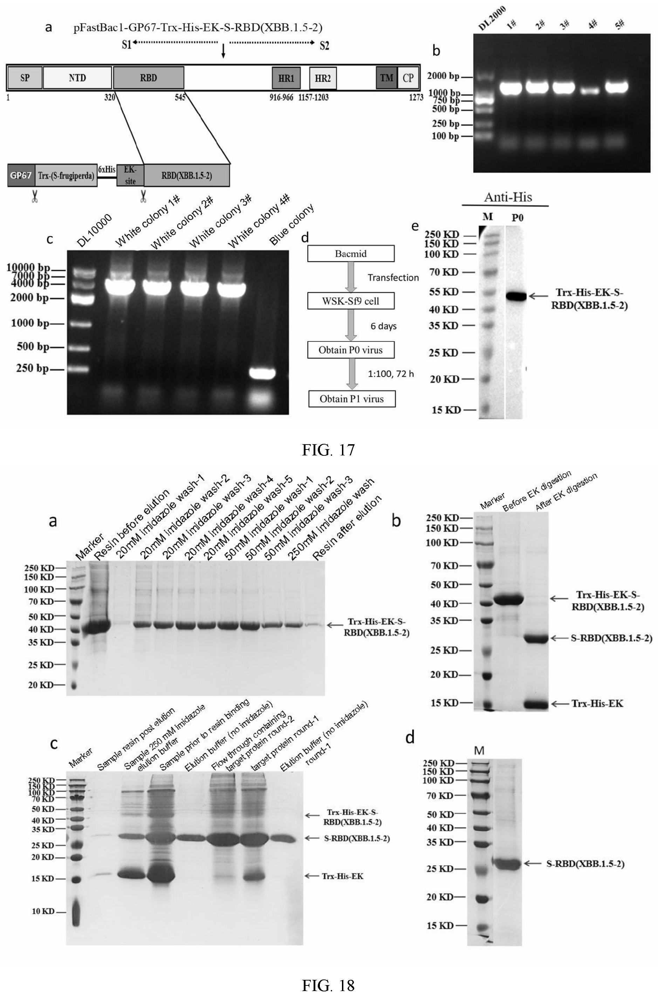

FIG. 17 illustrates the construction and expression of the recombinant protein antigen RBD_{XBB.1.5-2} of Embodiment 1, in which, a. design sketch of pFastBac1-GP67-Trx-His-EK-S-RBD(XBB.1.5-2); b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;
FIG. 18 illustrates the expression and purification results of the recombinant protein antigen RBD_{XBB.1.5-2} in Embodiment 1, in which, a. Elution profile of Ni-affinity chromatography from baculovirus-infected supernatant; b. Verification of EK digestion efficiency on EK-digested sample; and c-d. Purification of the target protein with Ni-affinity chromatography resin after EK digestion(c), and SDS-PAGE analysis(d);

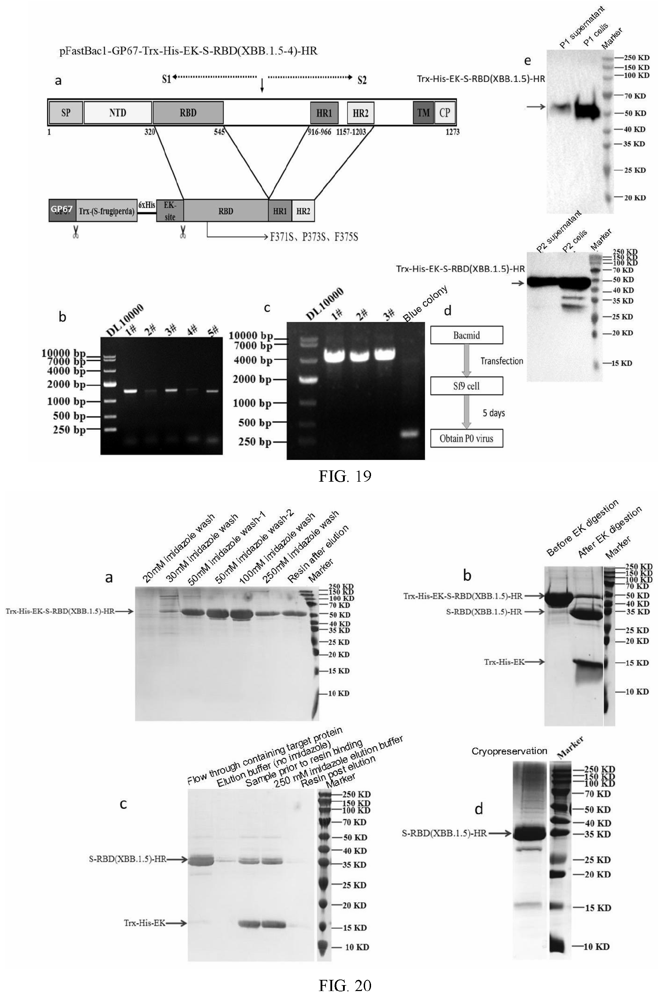

FIG. 19 illustrates the construction and expression of the recombinant protein antigen RBD_{XBB.1.5-4}-HR of Embodiment 1, in which, a. design sketch of pFastBac1-GP67-Trx-His-EK-S-RBD(XBB.1.5-4)-HR; b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;
FIG. 20 illustrates the expression and purification results of the recombinant protein antigen RBD_{XBB.1.5-4}-HR in Embodiment 1, in which, a. Elution profile of Ni-affinity chromatography from baculovirus-infected supernatant; b. Verification of EK digestion efficiency on EK-digested sample; and c. Purification of the target protein results of Ni-affinity chromatography resin after EK digestion;

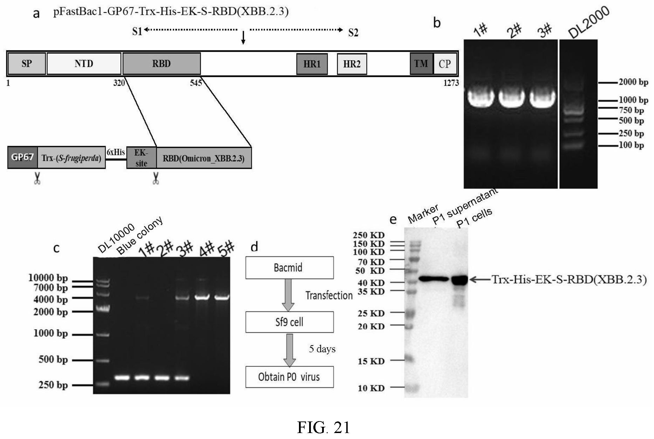

FIG. 21 illustrates the construction and expression of the recombinant protein antigen RBD_{XBB.2.3} of Embodiment 1, in which, a. design sketch of pFastBac1-GP67-Trx-His-EK-S-RBD(XBB.2.3); b. 1% agarose gel electrophoresis of cloned PCR products; c. 1% agarose gel electrophoresis of PCR verification for blue-white colony liquid; d. Packaging and amplification flowchart of recombinant baculovirus; and e. WB validation results during baculovirus amplification;

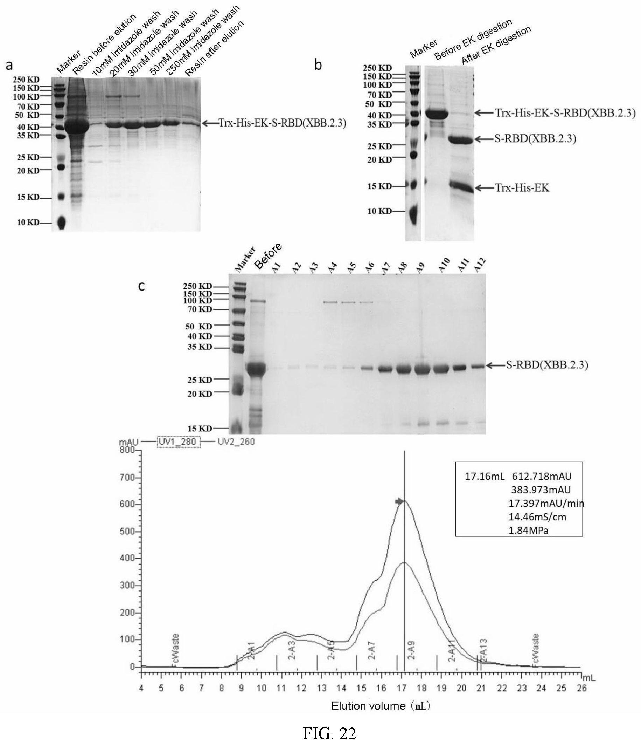

FIG. 22 illustrates the expression and purification results of the recombinant protein antigen RBD_{XBB.2.3} in Embodiment 1, in which, a. Elution profile of Ni-affinity chromatography from baculovirus-infected supernatant; b. Verification of EK digestion efficiency on EK-digested sample; and c. Purification of the target protein with Ni-affinity chromatography resin after EK digestion, and SDS-PAGE analysis;

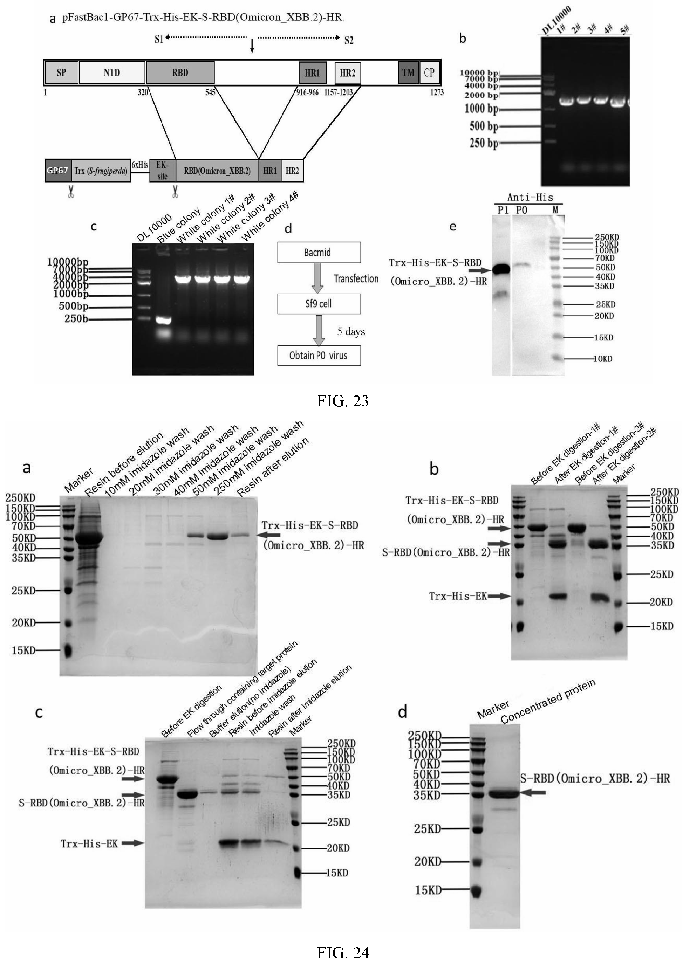

FIG. 23 illustrates the construction and expression of the recombinant protein antigen RBD_{XBB.2}-HR of Embodiment 1, in which, a. design sketch of pFastBac1-GP67-Trx-His-EK-S-RBD(Omicron_XBB.2)-HR; b. 1% agarose gel electrophoresis of cloned PCR products; c. 1% agarose gel electrophoresis of PCR-amplified recombinant bacmid; d. Packaging and amplification flowchart of recombinant baculovirus; and e. WB validation results during baculovirus amplification;
FIG. 24 illustrates the expression and purification results of the recombinant protein antigen RBD_{XBB.2}-HR in Embodiment 1, in which, a. Elution profile of Ni-affinity chromatography from baculovirus-infected supernatant; b. Verification of EK digestion efficiency on EK-digested sample; and c-d. Purification by size-exclusion chromatography after EK digestion(c), and SDS-PAGE analysis(d);

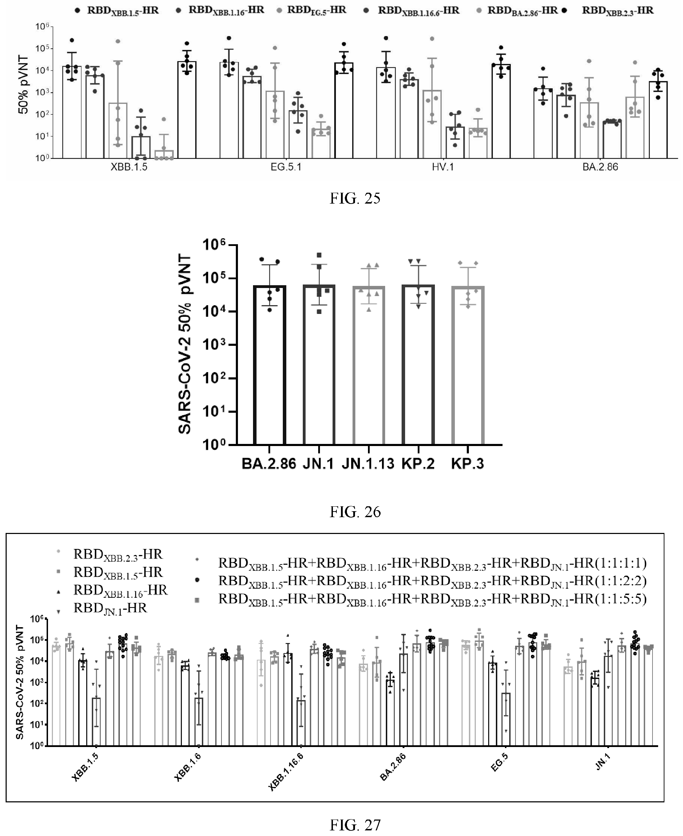

FIG. 25 illustrates GMT results of serums neutralizing antibodies against multiple pseudoviruses following intramuscular immunization with six protein vaccines in Test Example 1, including RBD_{XBB.1.16}-HR (SEQ ID No.1), RBD_{XBB.1.5-3}-HR (SEQ ID No.5), RBD_{XBB.2.3}-HR (SEQ ID No.8), RBD_{XBB.1.16.6}-HR (SEQ ID No.9), RBD_{BA.2.86}-HR (SEQ ID No.10), and RBD_{EG.5}-HR (SEQ ID No.11);
FIG. 26 illustrates GMT results of serums neutralizing antibodies against multiple pseudoviruses using the RBD_{JN.1}-HR protein (SEQ ID No. 55) as antigen in Test Example 1;
FIG. 27 illustrates GMT results of serums neutralizing antibodies through intramuscular immunization against multiple pseudoviruses in Test Example 2, comparing monovalent vaccines - RBD_{XBB.1.5-3}-HR (SEQ ID No. 5), RBD_{XBB.1.16}-HR (SEQ ID No. 1), RBD_{XBB.2.3}-HR (SEQ ID No. 8), and RBD_{JN.1}-HR (SEQ ID No. 55) - with tetravalent formulations (RBD_{XBB.1.5-3}-HR + RBD_{XBB.1.16}-HR + RBD_{XBB.2.3}-HR + RBD_{JN.1}-HR, mixing ratios of 1:1:1:1, 1:1:2:2, and 1:1:5:5);

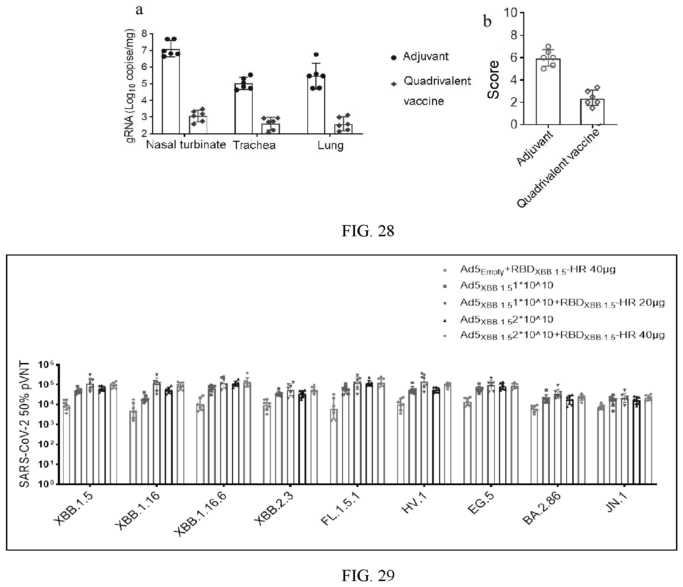

FIG. 28 illustrates results of the live virus challenge experiment after intramuscular immunization in mice with tetravalent vaccine RBD_{XBB.1.5-3}-HR + RBD_{XBB.1.16}-HR + RBD_{XBB.2.3}-HR + RBD_{JN.1}-HR (mixing ratio: 1:1:5:5) in Test Example 2; a illustrates viral gRNA levels in nasal turbinate, trachea, and lung tissues collected at day 5 post-infection, quantified by RT-qPCR; and b illustrates histopathological scoring of lung tissues following live XBB.1.16 virus challenge;
FIG. 29 illustrates results of neutralizing antibodies against multiple pseudoviruses via combined intranasal immunization in rats with Ad5_{XBB.1.5} adenoviral vector (SEQ ID No. 50) and recombinant protein vaccine RBD_{XBB.1.5-3}-HR (SEQ ID No. 5) in Test Example 3.

### DETAILED DESCRIPTION OF EMBODIMENTS

### Terms and abbreviations:

Monophosphate lipid A (MPL); squalene-based oil-in-water emulsion (MF59), recombinant cholera toxin (rCTB), astragalus polysaccharide (APS), phosphatidylethanolamine (PE), phosphatidylcholine (PC), cholesterol (Chol), dioleoyl phosphatidylethanolamine (DOPE), (2,3-dioleoxypropyl) trimethyl ammonium chloride (DOTAP), N-[1-(2,3-dioleyloxy)propyl]-N,N,N-trimethylammonium chloride (DOTMA), cationic cholesterol (DC-Chol), dimethyl trifluoroacetate-2,3-dioleoxypropyl-2-(2-spermamido) ethylammonium (DOSPA), trimethyl dodecyl ammonium bromide (DTAB), trimethyl tetradecyl ammonium bromide (TTAB), trimethyl cetyl ammonium bromide (CTAB), dimethyl didoctadecyl ammonium bromide (DDAB), CpG ODN (synthetic CpG with unmethylated cytosine and guanine dinucleotide as core sequence).

The solution of the present invention will be explained with reference to embodiments. It will be understood by those skilled in the art that the following embodiments are merely intended for illustrating the present invention, instead of limiting the scope of the present invention. If specific technologies or conditions are not specified in the embodiments, technologies or conditions described in literatures in the art or product specifications should prevail. If manufacturers are not indicated in reagents or instruments used, they are all conventional products that can be obtained through market purchase.

### Embodiment 1 Preparation of Recombinant Protein and Vaccine by Using Insect Baculovirus Expression Vector System

### Construction of recombinant protein antigen RBD_{XBB.1.16}-HR:

### 1. Construction and design of S-RBD(Omicron_XBB.1.16)-HR

Since the SARS-CoV-2 S protein is a membrane-anchored protein, to simulate its secretory pathway, we incorporated the GP67 signal peptide at the N-terminus of the S-RBD (Omicron_XBB.1.16)-HR for protein expression and construction, to facilitate secretory expression. This signal peptide would be autocatalytically cleaved by insect cells during protein secretion. Additionally, downstream of the GP67 signal peptide, we incorporated: a thioredoxin (Trx) tag from Spodoptera frugiperda (S. frugiperda) to facilitate proper folding of S-RBD(Omicron_XBB.1.16)-HR; a 6xHis tag to enable subsequent purification; and an EK digestion site to allow removal of both the Trx and 6xHis tags. The protein expression and construction would be able to remove all non-S-RBD (Omicron_XBB.1.16)-HR redundant amino acids through EK digestion. The expression & construction design pattern is shown in FIG. 1a. Its amino acid sequence is as shown in SEQ ID NO.19 and its nucleotide sequence is as shown in SEQ ID NO.37.

### 2. Identification for construction of recombinant plasmid

The designed encoding fragment was cloned into the pFastBac1 vector plasmid and verified by colony PCR. The colony PCR results demonstrated that all six selected clones successfully amplified the GP67-Trx-His-EK-S-RBD (Omicron_XBB.1.16)-HRfragment, as shown in FIG. 1b.

### 3. Identification of recombinant bacmid

The correctly identified pFastBac1-GP67-Trx-His-EK-S-RBD (Omicron_XBB.1.16)-HR recombinant clones were selected. After plasmid extraction, the recombinant plasmids were transformed into DH10b competent cells, followed by blue-white screening and colony PCR verification. The colony PCR product was detected by 1% agarose gel electrophoresis, and the identification result is as shown as FIG. 1c. The white colony showed the bacmid clone that had been recombined, and the blue colony showed the bacmid clone that had not been recombined.

### 4. Packaging of recombinant baculovirus

The recombinant bacmid was transfected into sf9 insect cells, and the P0 generation recombinant baculovirus was harvested after 5 days. The workflow for recombinant baculovirus packaging and baculovirus amplification is illustrated in FIG. 1d.

### 5. Expression and verification of target protein

The expression of the target protein was simultaneously taken place during the amplification of the above-mentioned baculovirus. Since the His tag was included before the tag of the target protein was removed, we used the WB experiment of Anti-His to verify the expression of the recombinant protein. The verification result showed that an obvious stripe was observed between 50 KD and 60 KD Marker stripes, and had a size matching with that of the Trx-His-EK-S-RBD (Omicron_XBB.1.16)-HR protein, indicating the successful amplification of baculovirus and successful expression of the target protein. The detection results are as shown in FIG. 1e.

### 6. Purification and identification of target protein

The recombinant baculovirus infected sf9 cells, a cell culture fluid was harvested after 3 days, and the verification for the protein purification was carried out through Ni-affinity chromatography resin. The results are as shown in FIG. 2a. The target protein was mainly eluted at 250 mM imidazole, and high-purity target protein could be obtained after elution.

### 7. Verification for removing tag of EK digestion

The target protein eluate was concentrated and adjusted to a concentration of 1 mg/mL, and EK enzyme was added. After enzyme digestion at 18 °C for 14 h, it was identified by SDS-PAGE gel electrophoresis. The results are as shown in FIG. 2b. The result showed that the EK enzyme could cut the Trx-His-EK (the amino acid sequence at the EK digestion site) tag from the target protein.

### 8. Removal verification for removed tag

After EK digestion, the sample was subjected to further purification using the Ni-affinity chromatography resin to separate the Trx-His-EK-S-RBD (Omicron_XBB.1.16)-HR protein (uncut full-length protein), the S-RBD (Omicron_XBB.1.16)-HR protein and the Trx-His-EK tag (cleaved tag fragment). The results are as shown in FIG. 2c-d. The results demonstrated that the Trx-His-EK tag was successfully removed and eliminated through subsequent purification steps, yielding the tag-free S-RBD (Omicron_XBB.1.16)-HRprotein, with the amino acid sequence shown in SEQ ID NO.1.

Similarly, following the construction method for the recombinant protein antigen RBD_{XBB.1.16}-HR (with variations in recombinant baculovirus packaging generations at Step 4 and using either the Ni-affinity chromatography resin or the size-exclusion chromatography for purification at Step 8), we constructed the following: RBD_{XBB.1.5-3}-HR (SEQ ID NO.5), RBD_{XBB.2.3}-HR (SEQ ID NO.8), RBD_{XBB.1.16.6}-HR (SEQ ID NO.9), RBD_{BA.2.86}-HR (SEQ ID NO.10), RBD_{EG.5}-HR (SEQ ID NO.11), RBD_{JN.1}-HR (SEQ ID NO.55), RBD_{XBB.1.S-1} (SEQ ID NO.3), RBD_{XBB.1.5-2} (SEQ ID NO.4), RBD_{XBB.1.5-4}-HR (SEQ ID NO.6), RBD_{XBB.2.3} (SEQ ID NO.7), and RBD_{XBB.2}-HR (SEQ ID NO.2). Among them, the construction design, process, and corresponding target protein expression data for RBD_{XBB.1.5-3}-HR are shown in FIGs. 3-4, the amino acid sequence of the designed encoding fragment pFastBac1-GP67-Trx-His-EK-S-RBD (XBB.1.5-3)-HR is as shown in SEQ ID NO.23, and the nucleotide sequence is as shown in SEQ ID NO.41.

Among them, the construction design, process, and corresponding target protein expression data for RBD_{XBB.2.3}-HR are shown in FIGs. 5-6, the amino acid sequence of the designed encoding fragment pFastBac1-GP67-Trx-His-EK-S-RBD(XBB.2.3)-HR is as shown in SEQ ID NO.26, and the nucleotide sequence is as shown in SEQ ID NO.44.

Among them, the construction design, process, and corresponding target protein expression data for RBD_{XBB.1.16.6}-HR are shown in FIGs. 7-8, the amino acid sequence of the designed encoding fragment pFastBac1-GP67-Trx-His-EK-S-RBD (XBB.1.16.6)-HR is as shown in SEQ ID NO.27, and the nucleotide sequence is as shown in SEQ ID NO.45.

Among them, the construction design, process, and corresponding target protein expression data for RBD_{BA.2.86}-HR are shown in FIGs. 9-10, the amino acid sequence of the designed encoding fragment pFastBac1-GP67-Trx-His-EK-S-RBD (BA.2.86)-HR is as shown in SEQ ID NO.28, and the nucleotide sequence is as shown in SEQ ID NO.46.

Among them, the construction design, process, and corresponding target protein expression data for RBD_{EG.5}-HR are shown in FIGs. 11-12, the amino acid sequence of the designed encoding fragment pFastBac1-GP67-Trx-His-EK-S-S-RBD (EG.5)-HR is as shown in SEQ ID NO.29, and the nucleotide sequence is as shown in SEQ ID NO.47.

Among them, the construction design, process, and corresponding target protein expression data for RBD_{JN.1}-HR are shown in FIGs. 13-14, the amino acid sequence of the designed encoding fragment pFastBac1-GP67-Trx-His-EK-S-RBD (JN.1)-HR is as shown in SEQ ID NO.56, and the nucleotide sequence is as shown in SEQ ID NO.57.

Among them, the construction design, process, and corresponding target protein expression data for RBD_{XBB.1.5-1} are shown in FIGs. 15-16, the amino acid sequence of the designed encoding fragment pFastBac1-GP67-Trx-His-EK-S-RBD (XBB.1.5-1) is as shown in SEQ ID NO.21, and the nucleotide sequence is as shown in SEQ ID NO.39.

Among them, the construction design, process, and corresponding target protein expression data for RBD_{XBB.1.5-2} are shown in FIGs. 17-18, the amino acid sequence of the designed encoding fragment pFastBac1-GP67-Trx-His-EK-S-RBD (XBB.1.5-2) is as shown in SEQ ID NO.22, and the nucleotide sequence is as shown in SEQ ID NO.40.

Among them, the construction design, process, and corresponding target protein expression data for RBD_{XBB.1.5-4}-HR are shown in FIGs. 19-20, the amino acid sequence of the designed encoding fragment pFastBac1-GP67-Trx-His-EK-S-RBD (XBB.1.5-4)-HR is as shown in SEQ ID NO.24, and the nucleotide sequence is as shown in SEQ ID NO.42.

Among them, the construction design, process, and corresponding target protein expression data for RBD_{XBB.2.3} are shown in FIGs. 21-22, the amino acid sequence of the designed encoding fragment pFastBac1-GP67-Trx-His-EK-S-RBD (XBB.2.3) is as shown in SEQ ID NO.25, and the nucleotide sequence is as shown in SEQ ID NO.43.

Among them, the construction design, process, and corresponding target protein expression data for RBD_{XBB.2}-HR are shown in FIGs. 23-24, the amino acid sequence of the designed encoding fragment pFastBac1-GP67-Trx-His-EK-S-RBD (XBB.2)-HR is as shown in SEQ ID NO.20, and the nucleotide sequence is as shown in SEQ ID NO.38.

### Embodiment 2 Preparation of Recombinant Adenovirus Vaccine

### 1. Optimization and synthesis of S protein gene sequence

The Omicron XBB.1.5 variant was selected as the template to obtain the gene sequence of its S protein, while retaining the native signal peptide of the S protein. Based on this, in order to ensure enhanced protein expression, codon optimization was performed, followed by gene synthesis of the optimized sequence (the nucleotide sequence is as shown in SEQ ID NO.50).

### 2. Packaging of recombinant adenovirus vaccine against SARS-COV-2

During the gene synthesis process, after the synthesized product S gene was cloned into the pDC316 vector using a recombinant cloning strategy, a shuttle plasmid (pDC316-S) was obtained. The constructed pDC316-S, comprising the S gene of Omicron XBB.1.5 variant, was co-transfected with the backbone plasmid pBHGlox_E1,3Cre from the AdMax adenovirus system into HEK293 cells to package the recombinant adenovirus. The process is as follows:
1) Inoculated 8×10⁵ HEK293A cells per well into a six-well plate with high-glucose DMEM + 10% FBS medium, cultured them overnight in a 37°C cell culture incubator containing 5% CO₂.
2) The next day, replaced the medium with high-glucose DMEM supplemented with 2% FBS, followed by co-transfection of HEK293A cells with the backbone plasmid (pBHGlox_E1,3Cre) and shuttle plasmid using lipofectamine 3000. The specific steps were as follows: took 4µg of backbone plasmid and 2µg of shuttle plasmid from each transfection well, diluted with 125µL Opti-MEM medium, and then added 12µL P3000 reagent; took another 1.5mL EP tube, diluted 7.5µL lipofectamine3000 with 125µL Opti-MEM medium; mixed the diluted plasmid and diluted lipofectamine3000 at a 1:1 ratio, incubated at room temperature for 10-15 minutes, and then added to the cells, continued to culture the cells, and subcultured them in 25cm² cell culture flasks after the cells grew full, observed the signs of viral cytopathic effect (CPE) every day, when the cells grew full of the bottom of the flask, transferred them to 75cm² cell culture flasks until the cells showed obvious plaques, and harvested the virus when most of the cells were diseased and fell off from the bottom.
3) Collected the virus-containing cell culture, centrifuged at 1200rpm for 3 minutes, aspirated the virus-containing supernatant, resuspended the cell pellet with 1/10 culture volume of virus-containing supernatant, alternately placed in -80°C refrigerator and 37°C water bath, froze and thawed three times repeatedly. Centrifuged at 3000rpm for 20 minutes, collected the virus-containing supernatant, and combined it with the above-mentioned virus-containing supernatant, which was the virus seed of adenovirus vaccine.
4) Took 50µL of the vaccine candidate strain virus liquid, added 2µL of protease K, digested at 50°C for 30min to release the viral genome, used this as a template to PCR amplify the S gene sequence, and sequenced and identified the PCR product after electrophoresis gel recovery. Requirements for PCT amplification is as follows:
   Denaturation: 95°C, 10min; Denaturation: 95°C, 10s; Annealing: 64°C, 30s; Extension: 72°C, 2min; Extension: 72°C, 5min; Cycle number: 40 times.

### 3. Amplification of recombinant adenovirus vaccine against SARS-COV-2

The identified correct recombinant adenovirus vaccine strains were amplified step by step in 293 cells. The specific process is as follows: added 4.0×10⁶ cells/mL of 293H cells with MOI = 3, and cultured for 48-72 hours. Collected the virus culture, and prepared the master virus seed bank and working virus seed bank by repeatedly freezing and thawing the sample 3 times. The recombinant adenovirus vaccine was amplified in a shake flask or a bioreactor, and the virus culture was harvested when extensive cytopathic effect was observed in the majority of cells. The process of amplifying cells and viruses in the bioreactor as follows: First, sterilized the assembled bioreactor, then added cell culture fluid to the bioreactor, once the operating conditions were stabilized at 37°C, pH 7.0, DO 50%, and 50rpm, collected cells in the shake flask, inoculated the cells into the bioreactor at a seeding density of 1.0×10⁶ cells/mL, supplemented the cell culture fluid to a total volume of 10L, maintained the bioreactor culture conditions at 37°C, 50-95rpm, pH7.15 - 7.25, and DO 30%-50%, took daily samples to monitor glucose concentration, cell density and cell morphology; inoculated the bioreactor with the recombinant adenovirus vaccine virus seed at an MOI of 3 when the cell density in the bioreactor reached 4.0×10⁶ cells/mL, took daily samples to monitor glucose concentration, cell viability, and virus titer after inoculation; and stopped the culture when most cells (50%-70%) had detached from the microcarriers, added virus lysis buffer to the bioreactor at a final concentration of 0.05%-1% Tween 80, performed lysis at 37°C for 2-4 hours, then collected the viral solution.

### 4. Purification of recombinant adenovirus vaccine against SARS-COV-2

The collected virus was purified by cesium chloride ultracentrifugation or ion exchange chromatography, and the specific process is as follows:

### (1) Purification of adenovirus vaccine by cesium chloride ultracentrifugation

Centrifuged the collected viral culture at 1200g for 10 minutes and aspirated the virus-containing supernatant, resuspended the cell pellet in virus-containing supernatant at 1/10 of the original culture volume, performed three freeze-thaw cycles using a -80°C freezer and 37°C water bath, centrifuged at 3000rpm for 10-20 minutes and collected the supernatant. Concentrated the virus-containing supernatant 10-fold using a 100K-300K ultrafiltration membrane; prepared a 1.4g/mL cesium chloride solution (by mixing 53g of cesium chloride with 87mL 10mM Tris-HCl, PH 7.9) and a 1.2g/mL cesium chloride solution (by mixing 26.8g of cesium chloride with 92 mL 10 mM Tris-HCl, pH 7.9); slowly added 8mL of 1.4g/mL cesium chloride solution into the ultracentrifuge tube, followed by gently adding 6mL of 1.2g/mL cesium chloride solution, finally, added 20mL of virus-containing supernatant on top of the discontinuous gradient, balanced the tube, then centrifuged at 100000×g and 4°C for 90 minutes; after centrifugation, used a syringe to extract the blue virus band; dialyzed to remove cesium chloride, and then stored the purified virus solution at -80°C.

### (2) Purification of adenovirus by ion exchange chromatography

Collected the viral culture and lysed it using 0.05%-1% Tween 20 at 37°C for 2-4 hours, clarified the lysed culture by filtering through 1.2µm and 0.45µm capsule filters, concentrated the sample 5-10 fold using a tangential flow filtration membrane with a molecular weight cutoff of 100-300 kD, washed the concentrate with 5-10 volumes of washing buffer (50 mM Tris-HCl, 2 mM MgCl₂, 0-500 mM NaCl, pH 8.0), and collected the washed sample; performed anion exchange chromatography using resins such as Capto Q Impress, Q Sepharose XL, Source 30Q, or Source 15Q, and the detailed procedure is as follows: balanced the column with equilibration buffer at a flow rate of 20mL/min for 5 column volumes, after equilibration, loaded the sample at a flow rate of 10mL/min, and once loading was complete, continued equilibrating with equilibration buffer until the conductivity stabilized; eluted the sample using a linear gradient from 100% low-salt buffer to 100% high-salt buffer over 10 column volumes at a flow rate of 10 mL/min, and collected the fractions corresponding to each elution peak; after elution, regenerated the column with 2M NaCl buffer for 5-10 column volumes at a flow rate of 20mL/min. Collected the virus peak, then performed buffer exchange on the eluted virus sample by dialysis or tangential flow filtration.

The amino acid sequence of the Ad5_{XBB.1.5} recombinant adenovirus vaccine antigen prepared by the above methods is as shown in SEQ ID NO. 32.

### The following experiments proved the effects of the recombinant protein vaccine and adenovirus vaccine prepared by the present invention.

### Animal Preparation, Animal Immunization and Sample Collection

1. Immunization procedure of monovalent vaccine: Female NIH mice (6 - 8 weeks old, SPF grade) were purchased from Beijing Vital River Laboratory Animal Technology Co., Ltd. (China) and housed under specific pathogen-free conditions at the State Key Laboratory of Biotherapy, Sichuan University. All mouse experiments were approved by the Institutional Animal Care and Use Committee (IACUC) of Sichuan University (Chengdu, Sichuan, China). The prepared recombinant protein antigens were separately mixed with an equal volume of an MF59 adjuvant to formulate monovalent vaccines. Mice were immunized intramuscularly three times on days 0, 21, and 42 (10 µg protein per mouse). Serum was collected 14 days after the third immunization to measure neutralizing antibodies and evaluate vaccine immunogenicity.
2. Basic immunization procedure of quadrivalent vaccine: Female NIH mice (6 - 8 weeks old, SPF grade) were purchased from Beijing Vital River Laboratory Animal Technology Co., Ltd. and housed under specific pathogen-free conditions at the State Key Laboratory of Biotherapy, Sichuan University. Four kinds of different recombinant protein antigens were mixed and combined with an equal volume of the MF59 adjuvant to formulate a quadrivalent vaccine. Mice were immunized intramuscularly three times on days 0, 21, and 42 (10 µg protein per mouse). Serum was collected 14 days after the third immunization to assess neutralizing antibody responses and evaluate vaccine immunogenicity.
3. Female BALB/c rats aged 6 - 8 weeks were purchased from Beijing Vital River Laboratory Animal Technology Co., Ltd. (China), and raised in an environment without special pathogens at the State Key Laboratory of Biotherapy, Sichuan University. To prepare the two-component intranasal vaccine, Ad5_{XBB.1.5} (1×10¹⁰ VP for low dose or 2×10¹⁰ VP for high dose) was mixed with RBD_{XBB.1.5}-HR (20 µg for low dose or 40 µg for high dose) in a total volume of 100 µL (low dose) or 200 µL (high dose). BALB/c rats were intranasally immunized three times (on days 0, 21, and 42) with the following formulations: (1) 2×10¹⁰VP Ad5_{Empty} and 40 µg RBD_{XBB.1.5}-HR; (2) 1×10¹⁰ VP Ad5_{XBB.1.5};(3) 1 × 10¹⁰ VP Ad5_{XBB.1.5} and 20 µg RBD_{XBB.1.5}-HR; and (4) 2×10¹⁰ VP Ad5_{XBB.1.5}; and (5) 2×10¹⁰ VP Ad5_{XBB.1.5} and 40 µg RBD_{XBB.1.5}-HR. Blood samples were collected 14 days after the third immunization to detect the neutralizing antibody, evaluating the immunogenicity of the vaccine.

### Test Example 1: Detection of Neutralizing Antibodies in Immune Serum of Recombinant Protein Vaccines Using Pseudovirus Neutralization Method

SARS-CoV-2 pseudoviruses, including XBB.1.5, XBB.1.6, XBB.1.16.6, EG.5.1, FL.1.5.1, HV.1, BA.2.86, EG.5, JN.1, JN.1.13, KP.2, KP.3, XBB.1.16, and XBB.2.3, were purchased from Genomeditech.

Briefly, the inactivated serum samples (56°C for 30min) were diluted by a factor of three, ranging from 30 to 65610, and then incubated with an equal volume of diluted pseudovirus at 37°C for 1 h. Then, 1.2×10⁴ HEK-293T cells expressing human ACE2 receptor (293T/ACE2) were added to each well and incubated in 0.5% CO₂ at 37°C for 48 h to express luciferase. Finally, the supernate was removed, then a lysis reagent (100µL/well) (Beyotime, RG005) with a luciferase substrate was added, and a multi-mode microwell plate reader (PerkinElmer, USA) was used to measure the luminous quantity in the 293T/ACE2 cell. 50% neutralizing rate of the pseudovirus was measured and calculated with GraphPad Prism 8.0.2. A positive control group only included cells and viruses, a negative control group only included cells, and a sample group included cells, samples and viruses. The neutralization percentage was calculated using the following formula: Neutralization (%) = [(Positive Control - Test Sample) / (Positive Control - Negative Control)] × 100%.

Monovalent recombinant protein vaccines were composed of seven proteins - RBD_{XBB.1.5-3}-HR (SEQ ID NO.5), RBD_{XBB.1.16}-HR (SEQ ID NO.1), RBD_{EG.5}-HR (SEQ ID NO.11), RBD_{XBB.1.16.6}-HR (SEQ ID NO.9), RBD_{BA.2.86}-HR (SEQ ID NO.10), RBD_{XBB.2.3}-HR (SEQ ID NO.8), and RBD_{JN.1}-HR (SEQ ID NO.55) - mixed with the adjuvant MF59. The quadrivalent vaccines were prepared by mixing the protein antigens RBD_{XBB.1.5-3}-HR, RBD_{XBB.1.16}-HR, RBD_{XBB.2.3}-HR, and RBD_{JN.1}-HR at ratios of 1:1:1:1, 1:1:2:2, and 1:1:5:1, followed by combining with an equal volume of the MF59 adjuvant. The mice were immunized with these vaccines, and serum was collected to measure neutralizing antibody levels against pseudoviruses including XBB.1.5, XBB.1.6, XBB.1.16.6, EG.5.1, FL.1.5.1, HV.1, BA.2.86, EG.5, JN.1, JN.1.13, KP.2, KP.3, XBB.1.16, and XBB.2.3.

As shown in FIG. 25, the six protein vaccines (RBD_{XBB.1.5-3}-HR, RBD_{XBB.1.16}-HR, RBD_{EG.5}-HR, RBD_{XBB.1.16.6}-HR, RBD_{BA.2.86}-HR, RBD_{XBB.2.3}-HR) elicited varying geometric mean titers (GMT) of 50% neutralization against four pseudoviruses (XBB.1.5, EG.5.1, HV.1, and BA.2.86). Among them, RBD_{XBB.1.5-3}-HR and RBD_{XBB.2.3}-HR protein vaccines demonstrated superior serum neutralizing antibody responses against diverse Omicron variants, with particularly high neutralizing antibody response against the EG.5.1 pseudovirus.

As shown in FIG. 26, sera collected 14 days after the third immunization with the RBD_{JN.1}-HR protein vaccine demonstrated potent neutralizing antibody responses against newly emerged Omicron variants, including BA.2.86, JN.1, JN.1.13, KP.2, and KP.3, indicating broad-spectrum neutralization coverage against diverse Omicron variants.

As shown in FIG. 27, the RBD_{XBB.1.5-3}-HR, RBD_{XBB.1.16}-HR, RBD_{XBB.2.3}-HR, and RBD_{JN.1}-HR protein antigens were mixed together (mixing ratios of 1:1:1:1, 1:1:2:2, 1:1:5:5) and combined with an equal volume of the MF59 adjuvant to form a quadrivalent vaccine. The quadrivalent vaccine exhibited effective neutralizing activity against the pseudoviruses of the variants representing current XBB subvariants (XBB.1.5, XBB.1.6, XBB.1.16.6, BA.2.86, EG.5, and JN.1) which were the representative mutations of the XBB spectrum currently spreading globally. The simple protein vaccines of RBD_{XBB.1.5-3}-HR, RBD_{XBB.1.16}-HR, RBD_{XBB.2.3}-HR, and RBD_{JN.1}-HR, especially the monovalent RBD_{JN.1}-HR protein vaccine, showed relatively weak protection against the pseudoviruses of XBB variants (XBB.1.5, XBB.1.6, XBB.1.16.6, and EG.5), with GMT values of 190, 186, 146, and 318 respectively. However, the combination of RBD_{XBB.1.5-3}-HR, RBD_{XBB.1.16}-HR, RBD_{XBB.2.3}-HR, and RBD_{JN.1}-HR, regardless of the mixing ratio, exhibited better protection against the pseudoviruses of all XBB variants, demonstrating the unique advantages of the quadrivalent vaccine.

### Test Example 2 Challenge Protection Study against SARS-CoV-2 XBB.1.16 Variant

Prevention of viral infection is one of the most critical indicators reflecting the protective efficacy of respiratory vaccines. Therefore, this test example aimed to evaluate whether the prepared quadrivalent recombinant protein vaccine could provide protective immune responses for preventing and/or treating epidemic variants. NIH mice (6 - 8 weeks old) were divided into two groups (n=6/group) and were intramuscularly immunized with 10 µg of quadrivalent vaccine (mixing ratio 1:1:5:5 of RBD_{XBB.1.5-3}-HR + RBD_{XBB.1.16}-HR + RBD_{XBB.2.3}-HR + RBD_{JN.1}-HR) mixed 1:1 with the MF59 adjuvant on days 0, 21, and 42. The adjuvant-only mice served as controls. Then, on day 63, the mice were intranasally challenged with 1×10⁶ PFU of live XBB.1.16 virus. Daily weight changes and throat swab viral loads were recorded. On day 5 post-infection, the mice were euthanized and their tissues were collected to detect the viral load and pathological changes. The viral genomic RNA (gRNA) in nasal turbinate, trachea and lung tissue samples was detected by reverse transcription quantitative polymerase chain reaction (RT-qPCR) assay. The primer and probe sequences, including 5'-GACCCCAAAATCAGCGAAAT-3' (SEQ ID NO. 58 forward), 5'-TCTGGTTACTGCCAGTTGAATCTG-3' (SEQ ID NO. 59 reverse), and 5'-FAM-ACNGCCGCATTACGTTGGTGGACC-BHQ1-3' (SEQ ID NO. 60 probe sequence), were used to detect the gRNA levels. Pathological changes in lung tissues were assessed by hematoxylin and eosin (H&E) staining.

All experiments involving live SARS-CoV-2 virus challenge in animals were rigorously reviewed and approved by the Institutional Animal Care and Use Committee (IACUC) of the Institute of Medical Biology, Chinese Academy of Medical Sciences. Furthermore, these experiments were conducted in ABSL-4 at the Kunming National High-Level Biosafety Primate Research Center(Established under the Institute of Medical Biotechnology (IMB)).

As shown in FIG. 28, the mice were euthanized on day 5 post-infection for tissue collection to evaluate viral load and histopathology. High gRNA levels were detected in the nasal turbinate, trachea, and lung tissues of adjuvant-only control mice. In contrast, the mice immunized with the quadrivalent vaccine exhibited significantly reduced viral loads in nasal turbinate, tracheal, and lung tissues. Additionally, mild pathological changes were observed in the lung tissues of the adjuvant-only control group, characterized by multifocal consolidation areas, thickened alveolar septa, alveolar congestion, and patchy inflammatory infiltrates. Just as expected, the lung tissues of the vaccinated mice showed normal histological structure, with complete alveoli and no obvious inflammation. Consequently, pathology scores in the lung tissues of the vaccinated mice were significantly lower compared to the adjuvant-only control group. These results demonstrated that the quadrivalent recombinant protein vaccine elicited protective immune responses in both the upper and lower respiratory tracts, effectively preventing infection by live virus.

### Test Example 3 Pseudovirus Neutralization Experiment for Recombinant Protein Vaccine in Combination with Adenovirus Vaccine

The pseudovirus source and the operation of the neutralization method were identical to those described in Test Example 1. As illustrated in FIG. 29, intranasal administration of either 1×10¹⁰ VP (low dose) or 2×10¹⁰ VP (high dose) Ad5_{XBB.1.5} adenovirus (SEQ ID No.50) combined with 20 µg or 40 µg RBD_{XBB.1.5-3}-HR recombinant protein vaccine (SEQ ID No.5) elicited significantly higher serum neutralizing antibody titers for against multiple Omicron variants (XBB.1.5, XBB.1.16, XBB.1.16.6, XBB.2.3, FL.1.5.1, HV.1, EG.5, BA.2.86, and JN.1). Obviously, the combination of adenovirus vaccine and recombinant protein vaccine elicited significantly stronger serum neutralizing antibodies compared to standalone Ad5 adenovirus or recombinant protein vaccines, demonstrating that the adenovirus-subunit protein formulation enhanced neutralizing protection capabilities in the blood and at the mucosal site, thereby effectively preventing SARS-CoV-2 infection.

## Claims

1. A protein for preventing and/or treating infection by SARS-CoV-2 Omicron XBB subvariants, wherein the protein has at least one amino acid sequence as shown from SEQ ID NO.1 to SEQ ID NO.18, and SEQ ID NO.55.

2. The protein for preventing and/or treating infection by SARS-CoV-2 Omicron XBB subvariants according to claim 1, wherein the protein contains at least one amino acid sequence as shown from SEQ ID NO.1 to SEQ ID NO.11 and SEQ ID NO.55; and preferably, the protein contains at least one amino acid sequence as shown from SEQ ID NO.1, SEQ ID NO.5, SEQ ID NO.8, SEQ ID NO.9, SEQ ID NO.10, SEQ ID NO.11, and SEQ ID NO.55.

3. A precursor of the protein according to claim 1 or 2, wherein the protein is linked to a signal peptide and/or a protein tag; and preferably, the protein tag is selected from at least one of the following: a histidine tag, a thioredoxin tag, a glutathione transferase tag, a ubiquitin-like modifier protein tag, a maltose-binding protein tag, a c-Myc protein tag, an Avi tag protein tag, and a nitrogen source utilization substance A protein tag.

4. The protein precursor according to claim 3, wherein a protease recognition region for excising the protein tag is further linked to the protein for preventing and/or treating infection by SARS-CoV-2 Omicron XBB subvariants; and preferably, the protease is selected from at least one of enterokinase, TEV protease, thrombin, coagulation factor Xa, carboxypeptidase A, and rhinovirus 3c protease.

5. The protein precursor according to claim 3 or 4, wherein an amino acid sequence of the precursor is selected from at least one of SEQ ID NO.19 to SEQ ID NO.36, and SEQ ID NO.56; preferably, the amino acid sequence of the precursor is at least one of SEQ ID NO.19 to SEQ ID NO.29, and SEQ ID NO.56; and more preferably, the amino acid sequence of the precursor is selected from at least one of SEQ ID NO.19, SEQ ID NO.23, SEQ ID NO.26, SEQ ID NO.27, SEQ ID NO.28, SEQ ID NO.29 and SEQ ID NO.56.

6. A polynucleotide, wherein the polynucleotide encodes the protein according to claim 1 or 2, or the precursor according to any one of claims 3-5.

7. The polynucleotide according to claim 6, wherein a sequence of the polynucleotide is selected from at least one of SEQ ID NO.37 to SEQ ID NO.54, and SEQ ID NO.57; preferably, the polynucleotide sequence is selected from at least one of SEQ ID NO.37 to SEQ ID NO.47, and SEQ ID NO.57; more preferably, the polynucleotide sequence is selected from at least one of SEQ ID NO.37, SEQ ID NO.41, SEQ ID NO.44, SEQ ID NO.45, SEQ ID NO.46, SEQ ID NO.47 and SEQ ID NO.57.

8. A recombinant vector, wherein the recombinant vector contains the polynucleotide according to claim 7.

9. The recombinant vector according to claim 8, wherein the recombinant vector is at least one of an insect baculovirus expression vector, a mammalian cell expression vector, an escherichia coli expression vector and a yeast expression vector; preferably, the insect baculovirus expression vector is pFastBac1; preferably, the escherichia coli expression vector is pET32a; the yeast expression vector is pPICZaA; the mammalian cell expression vector is a CHO cell expression vector; and further preferably, the CHO cell expression vector is pTT5 or FTP-002.

10. A host cell, wherein the host cell contains the recombinant vector according to claim 8 or 9.

11. The host cell according to claim 10, wherein the host cell is at least one of insect cells, mammalian cells, Escherichia coli, and yeasts; preferably, the insect cells are selected from at least one of sf9 cells, sf21 cells, and Hi5 cells; and the mammalian cells are CHO cells.

12. A preparation method for the protein according to claim 1 or 2 or the precursor according to any one of claims 3-5, wherein the preparation method contains the following steps: culturing the host cell according to claim 10 or 11, such that the host cell expresses the protein or the precursor, then recovering the protein, to obtain the protein.

13. A recombinant protein vaccine for preventing and/or treating infection by SARS-CoV-2 Omicron subvariants, wherein the recombinant protein vaccine contains the protein according to claim 1 or 2 or the precursor according to any one of claims 3-5, and a pharmacologically acceptable adjuvant component.

14. The recombinant protein vaccine according to claim 13, wherein the adjuvant component is an immunologic adjuvant; and preferably, the immunologic adjuvant is selected from at least one of the following: squalene-based oil-in-water emulsion, aluminum salt, calcium salt, phytosaponin, phytopolysaccharide, monophosphate lipid A, muramyl dipeptide, muramyl tripeptide, bacterial toxin, GM-CSF cytokine, lipid and cationic liposomal material.

15. The recombinant protein vaccine according to claim 14, wherein at least one of the following is met:
the squalene-based oil-in-water emulsion is MF59;
the aluminum salt is selected from at least one of aluminum hydroxide and alum;
the calcium salt is tricalcium phosphate;
the phytosaponin is either QS-21 or ISCOM;
the plant polysaccharide is astragalus polysaccharide;
the bacterial toxin is selected from at least one of recombinant cholera toxin and diphtheria toxin;
the lipid is selected from at least one of the following: phosphatidylethanolamine, phosphatidylcholine, cholesterol, and dioleoylphosphatidylethanolamine;
the cationic liposome material is selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyl dodecyl ammonium bromide, trimethyl tetradecyl ammonium bromide, trimethyl cetyl mmonium bromide, dimethyl dioctadecyl ammonium bromide, and CpG ODN.

16. A protein composition for preventing and/or treating infection by SARS-CoV-2 Omicron subvariants, wherein the protein composition contains any two or more proteins according to claim 1 or 2 or any two or more protein precursors according to any one of claims 3-5; the protein is constructed with any amino acid sequence selected from SEQ ID NO.1 to SEQ ID NO.18, and SEQ ID NO.55; and the precursor is constructed with any amino acid sequence selected from SEQ ID NO.19 to SEQ ID NO.36, and SEQ ID NO.56.

17. A recombinant protein vaccine composition for preventing and/or treating infection by SARS-CoV-2 Omicron subvariants, wherein the recombinant protein vaccine composition is the combination of any two or more different recombinant protein vaccines; and the recombinant protein vaccines are according to claim 13, 14 or 15.

18. An adenoviral vector vaccine for preventing and/or treating infection by SARS-CoV-2 Omicron subvariants, wherein the adenoviral vector vaccine contains a polynucleotide encoding SARS-CoV-2 or a variant spike protein thereof; and the polynucleotide sequence is obtained through codon optimization or cell optimization.

19. The adenoviral vector vaccine according to claim 18, wherein a nucleotide sequence of the polynucleotide is selected from at least one of SEQ ID NO.37 to SEQ ID NO.54; and preferably, the polynucleotide sequence is selected from a least one of SEQ ID NO.48 to SEQ ID NO.54; and more preferably, the polynucleotide sequence is selected from at least one of SEQ ID NO.48, SEQ ID NO.49, SEQ ID NO.50 and SEQ ID NO.51.

20. The adenoviral vector vaccine according to claim 19, wherein the adenovirus vector is selected from at least one of the following: adenovirus, Ankara vaccinia virus, and adeno-associated virus; preferably, the adenovirus vector is a replication-defective human adenovirus 5, 35, or 26 or/and replication-deficient chimpanzee adenovirus AdC68 or AdC7; and more preferably, the adenovirus vector is the replication-defective human adenovirus 5 with combined deletion of E1 and E3.

21. A method for preparing an adenovirus of the adenoviral vector vaccine according to any one of claims 18 to 20, wherein the method comprises the following steps: constructing a shuttle plasmid vector of polynucleotide encoding SARS-CoV-2 or a variant spike protein thereof; transfecting the constructed shuttle plasmid vector and a backbone plasmid into the host cell to culture the host cell; obtaining a replication-deficient recombinant adenovirus; and performing large-scale cultivation and purification.

22. A method for preparing the adenovirus according to claim 21, wherein the polynucleotide sequence encoding SARS-CoV-2 or a variant spike protein thereof is selected from at least one of SEQ ID NO.37 to SEQ ID NO.54; preferably, the polynucleotide sequence is selected from at least one of SEQ ID NO.48 to SEQ ID NO.54; and more preferably, the polynucleotide sequence is selected from at least one of SEQ ID NO.48, SEQ ID NO.49, SEQ ID NO.50 and SEQ ID NO.51.

23. The method for preparing the adenovirus vector according to claim 21, wherein at least one of the following is met:
the shuttle plasmid vector is at least one of pDC316-S, pDC315-S, pDC516-S or pDC515-S;
the backbone plasmid is at least one of pBHGlox_E1,3Cre and pBHGlox_E1,3FLP;
the host cell is HEK293.

24. An adenoviral vector vaccine composition for preventing and/or treating infection by SARS-CoV-2 Omicron subvariants, wherein the adenoviral vector vaccine composition is the combination of any two or more different adenoviral vector vaccines; and the adenoviral vector vaccines are according to claim 18, 19 or 20.

25. The protein according to claim 1 or 2, the protein precursor according to any one of claims 3-5, the protein composition according to claim 16, the recombinant protein vaccine according to any one of claims 13-15, the recombinant protein vaccine composition according to claim 17, and the adenoviral vector vaccine according to any one of claims 18-20 or the adenoviral vector vaccine composition according to claim 24, wherein these matters are formulated as an intradermal injection preparation or a subcutaneous injection preparation, an intramuscular injection preparation, an intravenous injection preparation, an oral preparation or a nasal spray preparation; and preferably, they are formulated as the intramuscular injection preparation and the nasal spray preparation.

26. A pharmaceutical composition for treating and/or preventing infection by SARS-CoV-2 Omicron subvariants, wherein the pharmaceutical composition is the combination of at least one of recombinant protein, protein composition, recombinant protein vaccine, and recombinant protein vaccine composition, as well as at least one of adenoviral vector vaccine and adenoviral vector vaccine composition;
preferably, the pharmaceutical composition is a polyvalent vaccine combination of at least one of the recombinant protein vaccine and the recombinant protein vaccine composition, as well as at least one of the adenoviral vector vaccine and the adenoviral vector vaccine composition; preferably, the pharmaceutical composition is a polyvalent vaccine combination of at least one of the recombinant protein and the protein composition, as well as at least one of the adenoviral vector vaccine and the adenoviral vector vaccine composition;
wherein the recombinant protein, the protein composition, the recombinant protein vaccine, the recombinant protein vaccine composition, the adenoviral vector vaccine, and the adenoviral vector vaccine composition are respectively the protein according to claim 1 or 2, the protein composition according to claim 16, the recombinant protein vaccine according to any one of claims 13-15, the recombinant protein vaccine composition according to claim 17, the adenoviral vector vaccine according to any one of claims 18-20, and the adenoviral vector vaccine composition according to claim 24.

27. A combined drug for treating and/or preventing infection by SARS-CoV-2 Omicron subvariants, wherein the combined drug is the combination of at least one of the recombinant protein, the protein composition, the recombinant protein vaccine, the recombinant protein vaccine composition and at least one of the adenoviral vector vaccine and the adenoviral vector vaccine composition, which are separately or simultaneously administered;
preferably, the combined drug is the combination of at least one of the recombinant protein vaccine and the recombinant protein vaccine composition, as well as at least one of the adenoviral vector vaccine and the adenoviral vector vaccine composition;
preferably, the combined drug is the combination of at least one of the recombinant protein and the protein composition, as well as at least one of the adenoviral vector vaccine and the adenoviral vector vaccine composition;
wherein the recombinant protein, the protein composition, the recombinant protein vaccine, the recombinant protein vaccine composition, the adenoviral vector vaccine, and the adenoviral vector vaccine composition are respectively the protein according to claim 1 or 2, the protein composition according to claim 16, the recombinant protein vaccine according to any one of claims 13-15, the recombinant protein vaccine composition according to claim 17, the adenoviral vector vaccine according to any one of claims 18-20, and the adenoviral vector vaccine composition according to claim 24.

28. The composition according to claim 26 or the combined drug according to claim 27, wherein the recombinant protein vaccine contains the protein or precursor for preventing and/or treating infection by SARS-CoV-2 Omicron XBB subvariants; an amino acid sequence of the protein or the precursor is selected from at least one of SEQ ID NO.1 to SEQ ID NO.11, SEQ ID NO.55, SEQ ID NO.19 to SEQ ID NO.29, and SEQ ID NO.56; preferably, the amino acid sequence of the protein or the precursor is selected from at least one of SEQ ID NO.1, SEQ ID NO.5, SEQ ID NO.8, SEQ ID NO.9, SEQ ID NO.10, SEQ ID NO.11, SEQ ID NO.55, SEQ ID NO.19, SEQ ID NO.23, SEQ ID NO.26, SEQ ID NO.27, SEQ ID NO.28, SEQ ID NO.29 and SEQ ID NO.56;
the recombinant protein vaccine composition is the combination of any two or more recombinant protein vaccines with different proteins;
the adenoviral vector vaccine contains at least one polynucleotide sequence as shown from SEQ ID NO.48 to SEQ ID NO.54; preferably, the adenoviral vector vaccine contains at least one polynucleotide sequence as shown from SEQ ID NO.48, SEQ ID NO.49, SEQ ID NO.50 and SEQ ID NO.51;
the adenoviral vector vaccine composition refers to the combination of any two or more adenoviral vector vaccines prepared with different polynucleotide sequences.

29. The composition or the combined drug according to claim 28, wherein the amino acid sequence of the recombinant protein vaccine is selected from at least one of SEQ ID NO.1, SEQ ID NO.5, SEQ ID NO.8, SEQ ID NO.9, SEQ ID NO.10, SEQ ID NO.11, and SEQ ID NO.55; and the adenoviral vector vaccine contains at least one polynucleotide sequence as shown from SEQ ID NO.48, SEQ ID NO.49, SEQ ID NO.50 and SEQ ID NO.51.

30. The pharmaceutical composition according to any one of claims 26, 28 and 29 or the combined drug according to any one of claims 27, 28 and 29, wherein it is formulated as an intradermal injection preparation or a subcutaneous injection preparation, an intramuscular injection preparation, an intravenous injection preparation, an oral preparation or a nasal spray preparation; and preferably, it is formulated as the intramuscular injection preparation and the nasal spray preparation.

31. A use of the protein according to claim 1 or 2, the protein precursor according to any one of claims 3-5, the protein composition according to claim 16, the recombinant protein vaccine according to any one of claims 13-15, the recombinant protein vaccine composition according to claim 17, the adenoviral vector vaccine according to any one of claims 18-20, the adenoviral vector vaccine composition according to claim 24, the pharmaceutical composition according any one of claims 26, 28 and 29, or the combined drug according to any one of claims 27, 28 and 29, for preparing a drug for treating and/or preventing infections or pathogenicity caused by SARS-CoV-2 or variants thereof.

32. The use according to claim 31, wherein the SARS-CoV-2 variant comprises at least one SARS-CoV-2 variant, specifically Alpha, Beta, Gamma, Delta, Omicron, Omicron subvariants BA.1, BA.2, BA.2.12.1, BA.4/5, BQ.1.1, XBB.1.16, XBB.2, XBB.1.5, XBB.2.3, XBB.1.6, XBB.1.9.1, XBB.1.16.6, FL.1.5.1, HV.1, EG.5, EG.5.1, BA.2.86, JN.1, JN.1.13, KP.2, and KP.3.
